Europäisches Patentamt
European Patent Office
Office européen des brevets

(11) Veröffentlichungsnummer: **0 412 350 A2**

# EUROPÄISCHE PATENTANMELDUNG

(21) Anmeldenummer: **90114115.0**

(22) Anmeldetag: **24.07.90**

(51) Int. Cl.5: **C07K 5/02**, A61K 31/415, C07D 233/64, A61K 37/64, C07D 263/26, C07D 209/48, C07D 401/12, C07D 403/12, C07C 271/22, C07C 229/36

(30) Priorität: **05.08.89 DE 3926021**
**16.02.90 DE 4004820**

(43) Veröffentlichungstag der Anmeldung:
**13.02.91 Patentblatt 91/07**

(84) Benannte Vertragsstaaten:
**AT BE CH DE DK ES FR GB GR IT LI LU NL SE**

(71) Anmelder: **BAYER AG**

**D-5090 Leverkusen 1 Bayerwerk(DE)**

(72) Erfinder: **Bender, Wolfgang, Dr.**
**Claudiusweg 5**
**D-5600 Wuppertal 1(DE)**
Erfinder: **Kinast, Günther, Dr.**
**Am Eckbusch 15a**
**D-5600 Wuppertal 1(DE)**
Erfinder: **Knorr, Andreas, Dr.**
**Trillser Graben 10**
**D-4006 Erkrath 2(DE)**
Erfinder: **Stasch, Johannes-Peter, Dr.**
**Schneewittchenweg 37**
**D-5600 Wuppertal 1(DE)**

(54) **Renininhibitoren, Verfahren zur Herstellung und ihre Verwendung in Arzneimitteln.**

(57) Die Erfindung betrifft neue renininhibitorische Peptide der allgemeinen Formel (I)

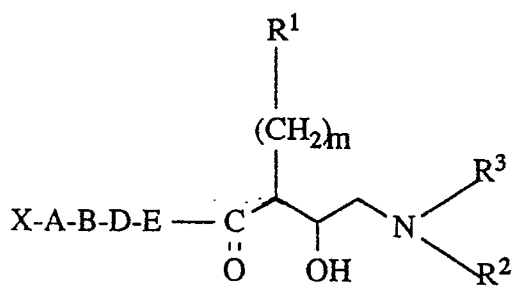

(I)

in welcher, X, A, B, D, E, m, $R^1$, $R^2$ und $R^3$ die in der Beschreibung angegebene Bedeutung haben, Verfahren zu ihrer Herstellung und ihre Verwendung in Arzneimitteln, insbesondere in kreislaufbeeinflussenden Arzneimitteln.

EP 0 412 350 A2

# RENININHIBITOREN, VERFAHREN ZUR HERSTELLUNG UND IHRE VERWENDUNG IN ARZNEIMITTELN

Die Erfindung betrifft neue renininhibitorische Peptide, Verfahren zu ihrer Herstellung und ihre Verwendung in Arzneimitteln, insbesondere in kreislaufbeeinflussenden Arzneimitteln.

Renin ist ein proteolytisches Enzym, das überwiegend von den Nieren produziert und ins Plasma sezerniert wird. Es ist bekannt, daß Renin in vivo vom Angiotensinogen das Dekapeptid Angiotensin I abspaltet. Angiotensin I wiederum wird in der Lunge, den Nieren oder anderen Geweben zu dem blutdruckwirksamen Oktapeptid Angiotensin II abgebaut. Die verschiedenen Effekte des Angiotensin II wie Vasokonstriktion, $Na^+$-Retention in der Niere, Aldosteronfreisetzung in der Nebenniere und Tonuserhöhung des sympathischen Nervensystems wirken synergistisch im Sinne einer Blutdruckerhöhung.

Die Aktivität des Renin-Angiotensin-Systems kann durch die Hemmung der Aktivität von Renin oder dem Angiotensin-Konversionsenzym (ACE) sowie durch Blockade von Angiotensin II-Rezeptoren pharmakologisch manipuliert werden. Die Entwicklung von oral einsetzbaren ACE-Hemmern hat somit zu neuen Antihypertensiva geführt (vgl. DOS 3 628 650, Am. J. Med 77 , 690, 1984).

Ein neuerer Ansatz ist, in die Renin-Angiotensin-Kaskade zu einem früheren Zeitpunkt einzugreifen, nämlich durch Inhibition der hochspezifischen Peptidase Renin.

Bisher wurden verschiedene Arten von Renininhibitoren entwickelt: Reninspezifische Antikörper, Phospholipide, Peptide mit der N-terminalen Sequenz des Prorenins, synthetische Peptide als Substratanaloga und modifizierte Peptide.

Außerdem ist die Aminosäure (3S, 4S)-4-Amino-3-hydroxy-6-methyl-heptancarbonsäure (Statin) bekannt [vgl. D.H. Rich, J. Med. Chem 28 , 263-73 (1985), Boger, J. ; Lohr, N.S.; Ulm, E.W., Pe, M.; Blaine, E.H.; Fanelli, G.M.; Lin, T.-Y.; Payne, L.S.; Schorn, T.W.; LaMont, B.I.; Vassil, T.C.; Stabilito, I.I.; Veber, D.F.; Rick, D.H.; Boparai, A.S. Nature (London) 1983, 303, 81].

Es wurden nun neue Renininhibitoren mit einem retrostatinen oder retrostatinanalogen Mittelteil gefunden, wobei sich der Säure-C-Terminus in N-terminaler Position und der Säure-N-Terminus in C-terminaler Position befindet und die flankierenden Aminosäurensequenzen ebenfalls umgedreht sind. Diese Renininhibitoren zeigen überraschenderweise eine hohe Selektivität gegenüber humanen Renin.

Die Erfindung betrifft Renininhibitoren der allgemeinen Formel (I)

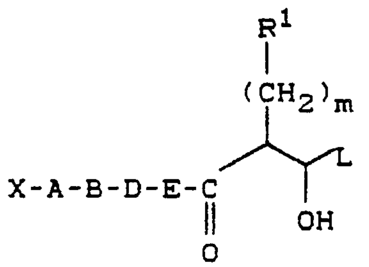

(I)

in welcher

X- für eine Gruppe der Formel

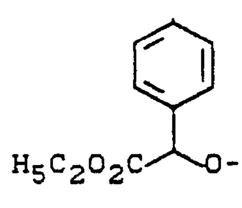

oder

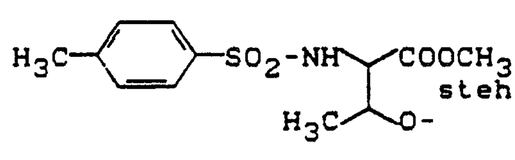

steht oder

für Hydroxy, Alkoxy mit bis zu 8 Kohlenstoffatomen, Benzyloxy oder für eine Gruppe der Formel $-NR^4R^5$ steht, worin $R^4$ und $R^5$ gleich oder verschieden sind und jeweils Wasserstoff, geradkettiges oder verzweigtes Alkyl mit bis zu 8 Kohlenstoffatomen bedeuten, das gegebenenfalls durch Phenyl substituiert ist oder Cycloalkyl mit 3 bis 8 Kohlenstoffatomen oder Aryl mit 6 bis 10 Kohlenstoffatomen, das seinerseits durch Halogen substituiert sein kann bedeuten

oder

$R^4$ und $R^5$ gemeinsam mit dem Stickstoffatom einen 5 bis 7-gliedrigen Heterocyclus mit bis zu 3

Heteroatomen aus der Reihe Stickstoff, Schwefel oder Sauerstoff bilden, A, B, D und E gleich oder verschieden sind und jeweils
- für eine direkte Bindung stehen,
- für einen Rest der Formel

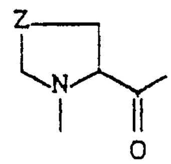

stehen,

worin
Z - Sauerstoff, Schwefel oder die Methylengruppe bedeutet oder
- für eine Gruppierung der Formel

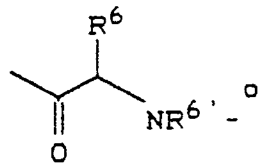

oder

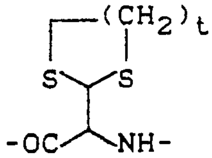

steht,

worin
t - die Zahl 1 oder 2 bedeutet,
$R^6$ - Wasserstoff oder Phenyl bedeutet oder
- geradkettiges oder verzweigtes Alkyl mit bis zu 8 Kohlenstoffatomen bedeutet, das gegebenenfalls durch Carboxy, Hydroxy, Halogen oder durch Alkoxy oder Alkoxycarbonyl mit jeweils bis zu 6 Kohlenstoffatomen substituiert ist, die ihrerseits durch Phenyl substituiert sein können,
oder durch eine Gruppe der Formel $-NR^7R^8$ oder $-CONR^7R^8$ substituiert ist
worin
$R^7$ und $R^8$ gleich oder verschieden sind und jeweils eine Aminoschutzgruppe, Wasserstoff, Acetoxy, Aryl mit 6 bis 10 Kohlenstoffatomen oder geradkettiges oder verzweigtes Alkyl mit bis zu 6 Kohlenstoffatomen bedeuten, das seinerseits durch Amino substituiert sein kann,
oder durch Aryl mit 6 bis 10 Kohlenstoffatomen substituiert ist, das gegebenenfalls bis zu 3-fach gleich oder verschieden durch Halogen, Nitro, Hydroxy oder Alkoxy mit bis zu 6 Kohlenstoffatomen substituiert ist,
oder durch Indolyl, Imidazolyl, Pyridyl, Triazolyl oder Pyrazolyl substituiert ist, die ihrerseits durch $R^8$ substituiert sein können, worin $R^8$ die oben angegebene Bedeutung hat,
$R^{6'}$ - Wasserstoff, Benzyl oder geradkettiges oder verzweigtes Alkyl mit bis zu 6 Kohlenstoffatomen bedeutet,
in ihrer D-Form, L-Form oder als D, L-Isomerengemisch,
m - für eine Zahl 0, 1 oder 2 steht,
$R^1$ - für geradkettiges oder verzweigtes Alkyl mit bis zu 8 Kohlenstoffatomen steht,
- für Cycloalkyl mit 3 bis 8 Kohlenstoffatomen steht,
- für Aryl mit 6 bis 10 Kohlenstoffatomen steht, das gegebenenfalls durch Nitro, Cyano, Halogen oder durch geradkettiges oder verzweigtes Alkyl mit bis zu 6 Kohlenstoffatomen substituiert sein kann,
L - für eine Gruppe der Formel $-CH_2-NR^2R^3$ oder

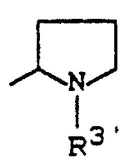

steht,

worin
$R^2$ und $R^3$ gleich oder verschieden sind und jeweils
- für Wasserstoff, Phenyl oder

- für geradkettiges oder verzweigtes Alkyl mit bis zu 8 Kohlenstoffatomen stehen, das gegebenenfalls durch Aryl mit 6 bis 10 Kohlenstoffatomen oder Pyridyl substituiert ist,
-für eine Gruppe der Formel -$COR^9$ stehen,
worin
$R^9$ - geradkettiges oder verzweigtes Alkyl oder Alkoxy mit bis zu 8 Kohlenstoffatomen oder Aryl mit 6 bis 10 Kohlenstoffatomen bedeutet, das seinerseits durch Carboxy, Alkoxycarbonyl mit bis zu 8 Kohlenstoffatomen oder durch eine Gruppe der Formel -$CONR^4R^5$ oder -CO-$NR^{10}R^{11}$ substituiert sein kann,
worin
$R^4$ und $R^5$ die oben angegebene Bedeutung haben und
$R^{10}$ und $R^{11}$ gleich oder verschieden sind und jeweils
-Wasserstoff oder geradkettiges oder verzweigtes Alkyl mit bis zu 8 Kohlenstoffatomen bedeuten, das seinerseits durch Phenyl oder Pyridyl substituiert sein kann,
oder
$R^2$ oder $R^3$ für eine Aminoschutzgruppe steht oder
$R^2$ und $R^3$ gemeinsam für Phthalimido oder Morpholino stehen oder für eine Gruppe der Formel

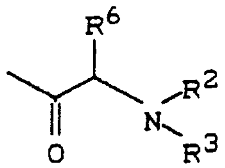 oder 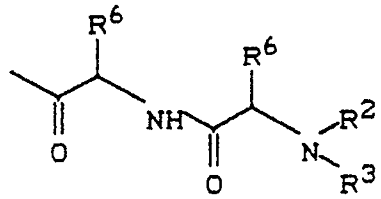 stehen

worin
$R^2$, $R^3$ und $R^6$ die oben angegebene Bedeutung haben
$R^{3'}$ die oben angegebene Bedeutung von $R^2$ hat und mit dieser gleich oder verschieden ist
und deren physiologisch unbedenklichen Salze.

Aminoschutzgruppe steht im Rahmen der Erfindung für die üblichen in der Peptid-Chemie verwendeten Aminoschutzgruppen.

Hierzu gehören bevorzugt: Benzyloxycarbonyl, 4-Brombenzyloxycarbonyl, 2-Chlorbenzyloxycarbonyl, 3-Chlorbenzyloxycarbonyl, Dichlorbenzyloxycarbonyl,3,4-Dimethoxybenzyloxycarbonyl, 3,5-Dimethoxybenzyloxycarbonyl, 2,4-Dimethoxybenzyloxycarbonyl, 4-Methoxybenzyloxycarbonyl, 4-Nitrobenzyloxycarbonyl, 2-Nitrobenzyloxycarbonyl, 2-Nitro-4,5-dimethoxybenzyloxycarbonyl, 3,4,5-Trimethoxybenzyloxycarbonyl, Methoxycarbonyl, Ethoxycarbonyl, Propoxycarbonyl, Isopropoxycarbonyl, Butoxycarbonyl, Isobutoxycarbonyl, tert-Butoxycarbonyl, Pent oxycarbonyl, Isopentoxycarbonyl, Hexoxycarbonyl, Cyclohexoxycarbonyl, Octoxycarbonyl, 2-Ethylhexoxycarbonyl, 2-Iodhexoxycarbonyl, 2-Bromethoxycarbonyl, 2-Chlorethoxycarbonyl, 2,2,2-Trichlorethoxycarbonyl, 2,2,2-Trichlor-tert-butoxycarbonyl, Benzhydryloxycarbonyl, Bis(4-methoxyphenyl)methoxycarbonyl, Phenacyloxycarbonyl, 2-Trimethylsilylethoxycarbonyl, 2-(Di-n-butyl-methylsilyl)-ethoxycarbonyl, 2-Triphenylsilylethoxycarbonyl, 2-(Dimethyl-tert-butylsilyl)ethoxycarbonyl, Menthyloxycarbonyl, Vinyloxycarbonyl, Allyloxycarbonyl, Phenoxycarbonyl, Tolyloxycarbonyl, 2,4-Dinitrophenoxycarbonyl, 4-Nitrophenoxycarbonyl, 2,4,5-Trichlorphenoxycarbonyl, Naphthyloxycarbonyl, Fluorenyl-9-methoxycarbonyl, Ethylthiocarbonyl, Methylthiocarbonyl, Butylthiocarbonyl, Tert.-Butylthiocarbonyl, Phenylthiocarbonyl, Benzylthiocarbonyl, Methylaminocarbonyl, Ethylaminocarbonyl, Methylthiocarbonyl, Butylthiocarbonyl, tert-Butylthiocarbonyl, Phenylthiocarbonyl, Benzylthiocarbonyl, Methylaminocarbonyl, Ethylaminocarbonyl, Propylaminocarbonyl, iso-Propylaminocarbonyl, Formyl, Acetyl, Propionyl, Pivaloyl, 2-Chloracetyl, 2-Bromacetyl, 2-Iodacetyl, 2,2,2-Trifluoracetyl, 2,2,2-Trichloracetyl, Benzoyl, 4-Chlorbenzoyl, 4-Methoxybenzoyl, 4-Nitrobenzyl, 4-Nitrobenzoyl, Naphthylcarbonyl, Phenoxyacetyl, Adamantylcarbonyl, Dicyclohexylphosphoryl, Diphenylphosphoryl, Dibenzylphosphoryl, Di-(4-nitrobenzyl)phosphoryl, Phenoxyphenylphosphoryl, Diethylphosphinyl, Diphenylphosphinyl, Phthaloyl oder Phthalimido.

Besonders bevorzugte Aminoschutzgruppen sind Benzyloxycarbonyl, 3,4-Dimethoxybenzyloxycarbonyl, 3,5-Dimethoxybenzyloxycarbonyl, 4-Methoxybenzyloxycarbonyl, 4-Nitro benzyloxycarbonyl, 2-Nitrobenzyloxycarbonyl, 3,4,5-Trimethoxybenzyloxycarbonyl, Methoxycarbonyl, Ethoxycarbonyl, Propoxycarbonyl, Isopropoxycarbonyl, Butoxycarbonyl, Isobutoxycarbonyl, tert-Butoxycarbonyl, Cyclohexoxycarbonyl, Hexoxycarbonyl, Octoxycarbonyl, 2-Bromethoxycarbonyl, 2-Chlorethoxycarbonyl, Phenoxyacetyl, Naphthylcarbonyl, Adamatylcarbonyl, Phthaloyl, 2,2,2-Trichlorethoxycarbonyl, 2,2,2-Trichlor-tert-butoxycarbonyl, Menthyloxycarbonyl, Vinyloxycarbonyl, Allyloxycarbonyl, Phenoxycarbonyl, 4-Nitrophenoxycarbonyl, Fluorenyl-9-methoxycarbonyl, Formyl, Acetyl, Propionyl, Pivaloyl, 2-Chloracetyl, 2-Bromacetyl, 2,2,2-Trifluoracetyl, 2,2,2-Trichloracetyl, Benzoyl, 4-Chlorbenzoyl, 4-Brombenzoyl, 4-Nitrobenzoyl, Phthalimido oder Isovaleroyl.

Die erfindungsgemäßen Verbindungen der allgemeinen Formel (I), haben mehrere asymmetrische Kohlenstoffatome. Sie können unabhängig voneinander in der D- oder der L-Form vorliegen. Die Erfindung umfaßt die optischen Antipoden ebenso wie die Isomerengemische oder Racemate.
Bevorzugt liegen die Grupppen A, B, D, E und $R^3$ unabhängig voneinander in der optisch reinen, bevorzugt in der D-Form vor.

Die Gruppe der Formel

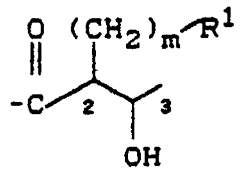

besitzt 2 asymetrische Kohlenstoffatome, die unabhängig voneinander in der R- oder S-Konfiguration vorliegen können, beispielsweise in der 2S3S, 2R3R, 2S3R oder 2R3S-Konfiguration vor.

Ebenso wird die Gruppierung als Isomerengemisch eingesetzt.

Die erfindungsgemäßen Verbindungen existieren in stereoisomeren Formen, die sich entweder wie Bild und Spiegelbild (Enantiomere) oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen (vgl. E.L. Eliel, Stereochemistry of Carbon Compounds, McGraw Hill, 1962).

Die erfindungsgemäßen Verbindungen der allgemeinen Formel (I) können in Form ihrer Salze vorliegen. Dies können Salze der erfindungsgemäßen Verbindung mit anorganischen oder organischen Säuren oder Basen sein. Zu den Säureadditionsprodukten gehören bevorzugt Salze mit Salzsäure, Bromwasserstoffsäure, Iodwasserstoffsäure, Schwefelsäure, Phosphorsäure oder mit Carbonsäuren wie Essigsäure, Propionsäure, oxalsäure, Glykolsäure, Bernsteinsäure, Maleinsäure, Hydroxymaleinsäure, Methylmaleinsäure, Fumarsäure, Adipinsäure, Äpfelsäure, Weinsäure, Zitronensäure, Benzoesäure, Zimtsäure, Milchsäure, Ascorbinsäure, Salicylsäure, 2-Acetoxybenzoesäure, Nicotinsäure, Isonicotinsäure, oder Sulfonsäuren wie Methansulfonsäure, Ethansulfonsäure, Benzolsulfonsäure, Toluolsulfonsäure, Naphthalin-2-sulfonsäure oder Naphthalindisulfonsäure.

Bevorzugt sind Verbindungen der allgemeinen Formel (I), in welcher
X- für eine Gruppe der Formel

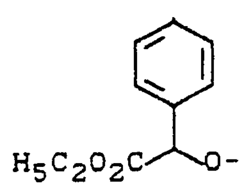

oder

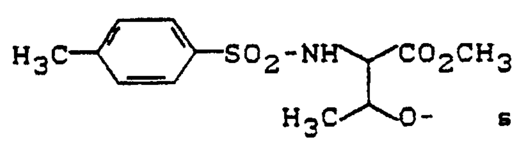

steht,

für Hydroxy, Alkoxy mit bis zu 6 Kohlenstoffatomen, Benzyloxy oder für eine Gruppe der Formel $-NR^4R^5$ steht, worin
$R^4$ und $R^5$ gleich oder verschieden sind und jeweils Wasserstoff, geradkettiges oder verzweigtes Alkyl mit bis zu 6 Kohlenstoffatomen bedeuten, das gegebenenfalls durch Phenyl substituiert ist, Cyclopropyl, Cyclopentyl, Cyclohexyl oder Phenyl bedeuten, das seinerseits durch Fluor oder Chlor substituiert ist, oder
$R^4$ und $R^5$ gemeinsam mit dem Stickstoffatom einen Morpholinring ausbilden,
A, B, D und E gleich oder verschieden sind und jeweils
- für eine direkte Bindung stehen oder
- für einen Rest der Formel

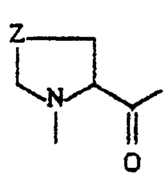

stehen,

worin
Z - Sauerstoff, Schwefel oder die Methylengruppe bedeutet,
oder
- für einen Rest der Formel

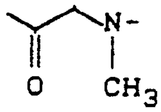 , 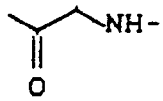 , 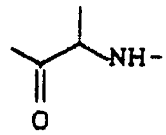 , 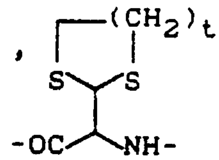 ,

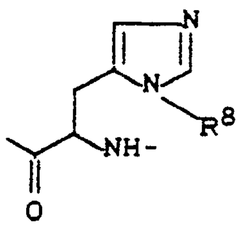 , 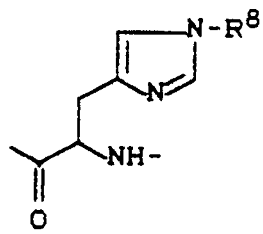 , 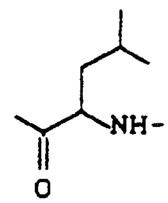 ,

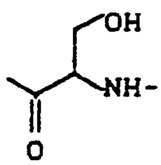 , 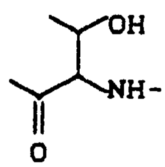 , 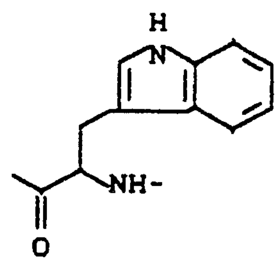 ,

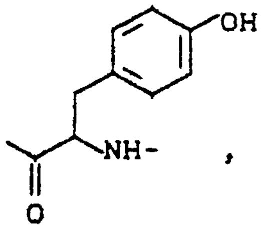 , 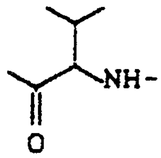 , 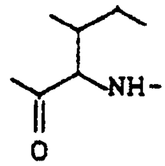 ,

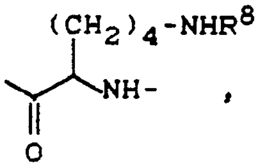 , 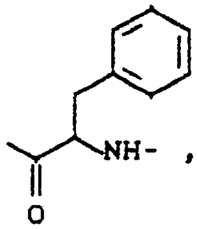 , 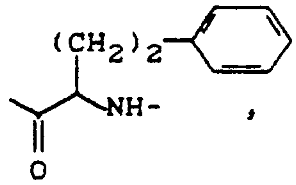 ,

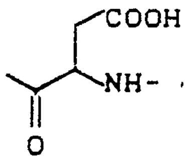 , 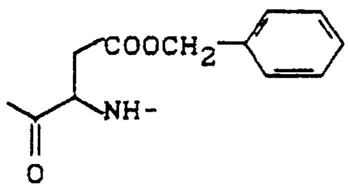 , 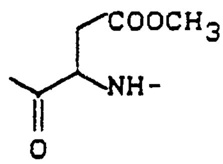

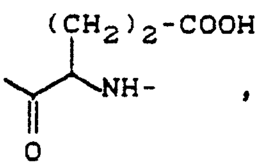 , 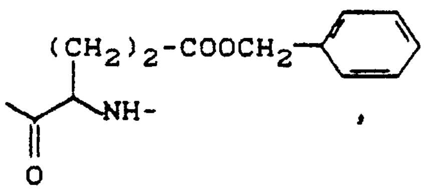 , 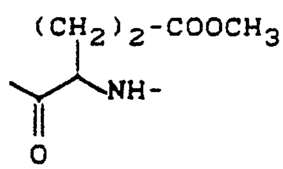

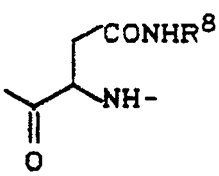 , 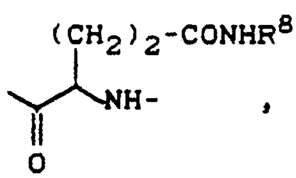 , 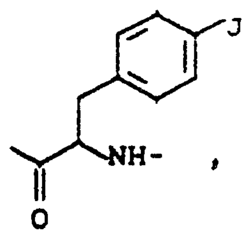 ,

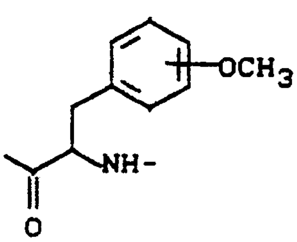 , 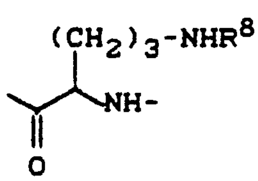 , 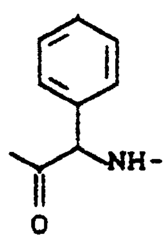 ,

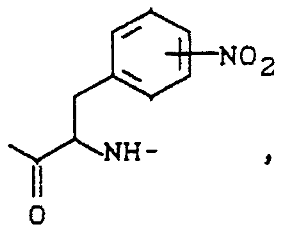 , 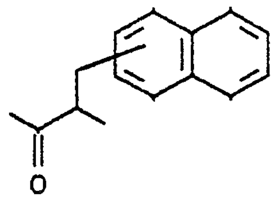 ,

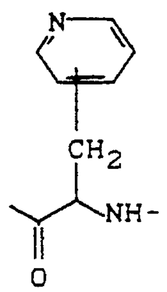 , 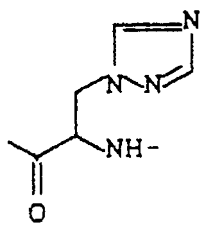 oder 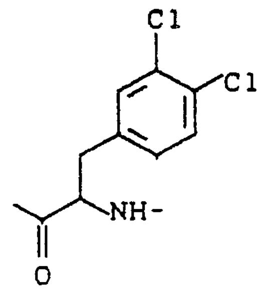

steht,
in welcher
t - die Zahl 1 oder 2 bedeutet,
$R^8$ - Wasserstoff, geradkettiges oder verzweigtes Alkyl mit bis zu 4 Kohlenstoffatomen oder eine Aminoschutzgruppe bedeutet,
in ihrer D-Form, L-Form oder als D, L-Isomerengemisch,
m - für eine Zahl 0, 1 oder 2 steht,
$R^1$ - für geradkettiges oder verzweigtes Alkyl mit bis zu 6 Kohlenstoffatomen steht,
- für Cyclopropyl, Cyclopentyl oder Cyclohexyl steht,
- für Phenyl steht, das gegebenenfalls durch Nitro, Cyano, Fluor oder Chlor substituiert ist.
L - für eine Gruppe der Formel $-CH_2-NR_2R^3$ oder

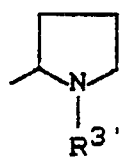 **steht,**

worin
$R^2$ und $R^3$ gleich oder verschieden sind und jeweils
- für Wasserstoff, Phenyl oder
- für geradkettiges oder verzweigtes Alkyl mit bis zu 6 Kohlenstoffatomen stehen, das gegebenenfalls durch Phenyl oder Pyridyl substituiert ist,
- für eine Gruppe der Formel $-COR^9$ stehen,
worin
$R^9$ - geradkettiges oder verzweigtes Alkyl oder Alkoxy mit bis zu 6 Kohlenstoffatom oder Phenyl bedeutet, das seinerseits durch Carboxy, Alkoxycarbonyl mit bis zu 6 Kohlenstoffatomen oder durch eine Gruppe der Formel $-CONR^4R^5$ oder $-CO-NR^{10}R^{11}$ substituiert sein kann,
worin
$R^4$ und $R^5$ die oben angegebene Bedeutung haben,
$R^{10}$ und $R^{11}$ gleich oder verschieden sind und jeweils
-Wasserstoff oder geradkettiges oder verzweigtes Alkyl mit bis zu 6 Kohlenstoffatomen bedeuten, das seinerseits durch Phenyl oder Pyridyl substituiert sein kann,
oder
$R^2$ oder $R^3$ - für eine Aminoschutzgruppe steht oder
$R^2$ und $R^3$ - gemeinsam für Phthalimido oder Morpholino stehen oder
$R^2$ oder $R^3$ - für die Gruppe der Formel

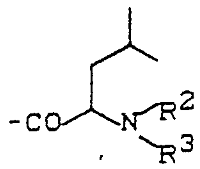 oder 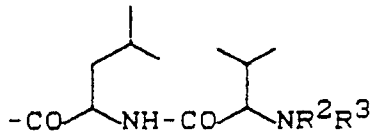 steht,

worin
$R^2$ und $R^3$ die oben angegebene Bedeutung haben, und
$R^{3'}$ die oben angegebene Bedeutung von $R^3$ hat und mit dieser gleich oder verschieden ist und deren physiologisch unbedenklichen Salze.

Besonders bevorzugt sind Verbindungen der allgemeinen Formel (I)
in welcher
X - für eine Gruppe der Formel

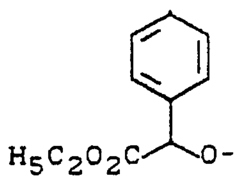 oder 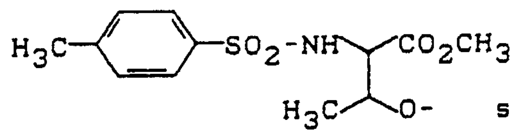 steht oder

für Hydroxy, Alkoxy mit bis zu 4 Kohlenstoffatomen, Benzyloxy oder für eine Gruppe der Formel $-NR^4R^5$ steht, worin
$R^4$ und $R^5$ gleich oder verschieden sind und jeweils Wasserstoff, geradkettiges oder verzweigtes Alkyl mit bis zu 4 Kohlenstoffatomen bedeuten, das gegebenenfalls durch Phenyl substituiert ist, Cyclopentyl oder Phenyl, das seinerseits durch Chlor substituiert sein kann bedeuten, oder $R^4$ und $R^5$ gemeinsam mit dem Stickstoffatom einen Morpholinring bilden,
A, B, D und E gleich oder verschieden sind und jeweils
- für eine direkte Bindung stehen oder
- für Glycyl (Gly), Alanyl (Ala), Arginyl (Arg), Histidyl (His), Leucyl (Leu), Isoleucyl (Ile), Seryl (Ser), Threonyl (Thr), Tryptophyl (Trp), Tyrosyl (Tyr), Valyl (Val), Lysyl (Lys), Aspartyl (Asp), Asparaginyl (Asn), Glutamyl (Glu) oder Phenylalanyl (Phe) oder Prolyl (Pro) oder für eine Gruppe der Formel

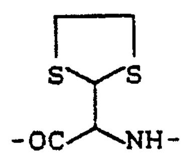 stehen,

gegebenenfalls mit einer Aminoschutzgruppe, in ihrer L-Form oder D-Form,
m - für die Zahl 1 steht,
$R^1$ - für geradkettiges oder verzweigtes Alkyl mit bis zu 4 Kohlenstoffatomen, Cyclohexyl oder Phenyl steht,
L - für eine Gruppe der Formel $-CH_2-NR^2R^3$ oder

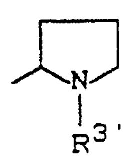 steht,

worin
$R^2$ und $R^3$ gleich oder verschieden sind und jeweils
- für Wasserstoff, Phenyl oder
- für geradkettiges oder verzweigtes Alkyl mit bis zu 4 Kohlenstoffatomen stehen, das gegebenenfalls durch Phenyl oder Pyridyl substituiert ist,

- für eine Gruppe der Formel $-COR^9$ stehen, worin

$R^9$ - geradkettiges oder verzweigtes Alkyl oder Alkoxy mit bis zu 4 Kohlenstoffatomen oder Phenyl bedeutet, das seinerseits durch Carboxy, Alkoxycarbonyl mit bis zu 4 Kohlenstoffatomen oder durch eine Gruppe der Formel $-CONR^4R^5$ oder $-CONR^{10}R^{11}$ substituiert sein kann,

$R^4$ und $R^5$ die oben angegebene Bedeutung haben,

$R^{10}$ und $R^{11}$ gleich oder verschieden sind und jeweils

- Wasserstoff oder geradkettiges oder verzweigtes Alkyl mit bis zu 4 Kohlenstoffatomen bedeuten, das seinerseits durch Phenyl oder Pyridyl substituiert sein kann,

oder

$R^2$ oder $R^3$ - für eine Aminoschutzgruppe steht

oder

$R^2$ und $R^3$ gemeinsam für Phthalimido oder Morpholino stehen oder

$R^2$ oder $R^3$ - für die Gruppe der Formel

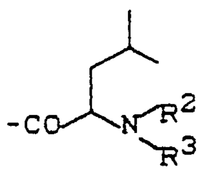 oder 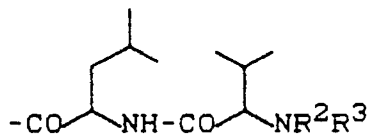 steht,

worin

$R^2$ und $R^3$ die oben angegebene Bedeutung haben,

und

$R^{3'}$ die oben angegebene Bedeutung von $R^3$ hat und mit dieser gleich oder verschieden ist

und

deren physiologisch unbedenklichen Salze.

Die erfindungsgemäßen Verbindungen der allgemeinen Formel (I)

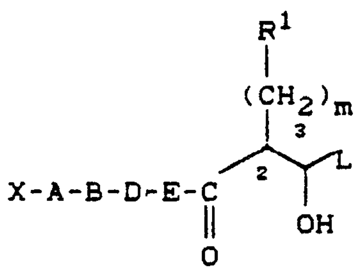

(I)

in welcher

X, A, B, D, E, $R^1$, m, und L die oben angegebene Bedeutung haben,

erhält man, indem man

[A] entweder Verbindungen der allgemeinen Formel (II)

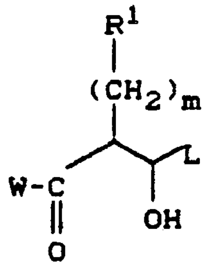

(II)

in welcher

$R^1$, L, und m die oben angegebene Bedeutung haben,
und
W - für eine typische Carboxylschutzgruppe, wie beispielsweise Benzyloxy oder Alkoxy mit bis zu 6 Kohlenstoffatomen, die gegebenenfalls durch Alkoxycarbonyl mit bis zu 6 Kohlenstoffatomen substituiert sind, steht,
oder stereoselektiv Verbindungen der allgemeinen Formel (III)

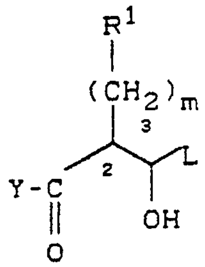

(III)

in welcher
$R^1$, L, und m die oben angegebene Bedeutung haben,
und
Y - für einen N-gebundenen Oxazolidin-2-on-Ring steht, der gegebenenfalls durch Alkyl mit bis zu 4 Kohlenstoffatomen, Benzyl oder Phenyl zweifach substituiert ist,
zunächst durch Abspaltung der Schutzgruppen nach üblicher Methode in die entsprechenden Säuren überführt und in einem zweiten Schritt mit Verbindungen der allgemeinen Formel (IV)
$X'$-A-B-D-E-$NH_2$ (IV)
in welcher
A, B, D und E die oben angegebene Bedeutung haben
und
$X'$ - für eine Carboxylschutzgruppe steht
in inerten Lösungsmitteln, gegebenenfalls in Anwesenheit eines Hilfsstoffes umsetzt und die Schutzgruppe $X'$ nach üblicher Methode abspaltet und die Reste X und L zur Erfassung des oben aufgeführten Bedeutungumfangs nach bekannter Methode, beispielsweise durch Aminierung, Veresterung oder Hydrolyse einführt,
oder indem man
[B] Verbindungen der allgemeinen Formeln (II) und (III) durch Umsetzung von einem entsprechenden Bruchstück, bestehend aus einer oder mehreren Aminosäuregruppierungen, mit einer freien, gegebenenfalls in aktivierter Form vorliegenden Carboxylgruppe mit einem komplementierenden Bruchstück, bestehend aus einer oder mehreren Aminosäuregruppierungen, mit einer Aminogruppe, gegebenenfalls in aktivierter Form, herstellt, und diesen Vorgang gegebenenfalls so oft mit entsprechenden Bruchstükken wiederholt, bis man die gewünschten Peptide der allgemeinen Formel (I) hergestellt hat, anschließend gegebenenfalls Schutzgruppen abspaltet, gegen andere Schutzgruppen austauscht und gegebenenfalls die Reste L und X nach den unter Verfahren [A] aufgeführten Methoden derivatisiert, wobei zusätzliche reaktive Gruppen, wie z.B. Amino- oder Hydroxygruppen, in den Seitenketten der Bruchstükke gegebenenfalls durch übliche Schutzgruppen geschützt werden können.

Die Verfahren können durch folgendes Formelschema erläutert werden:

[A]

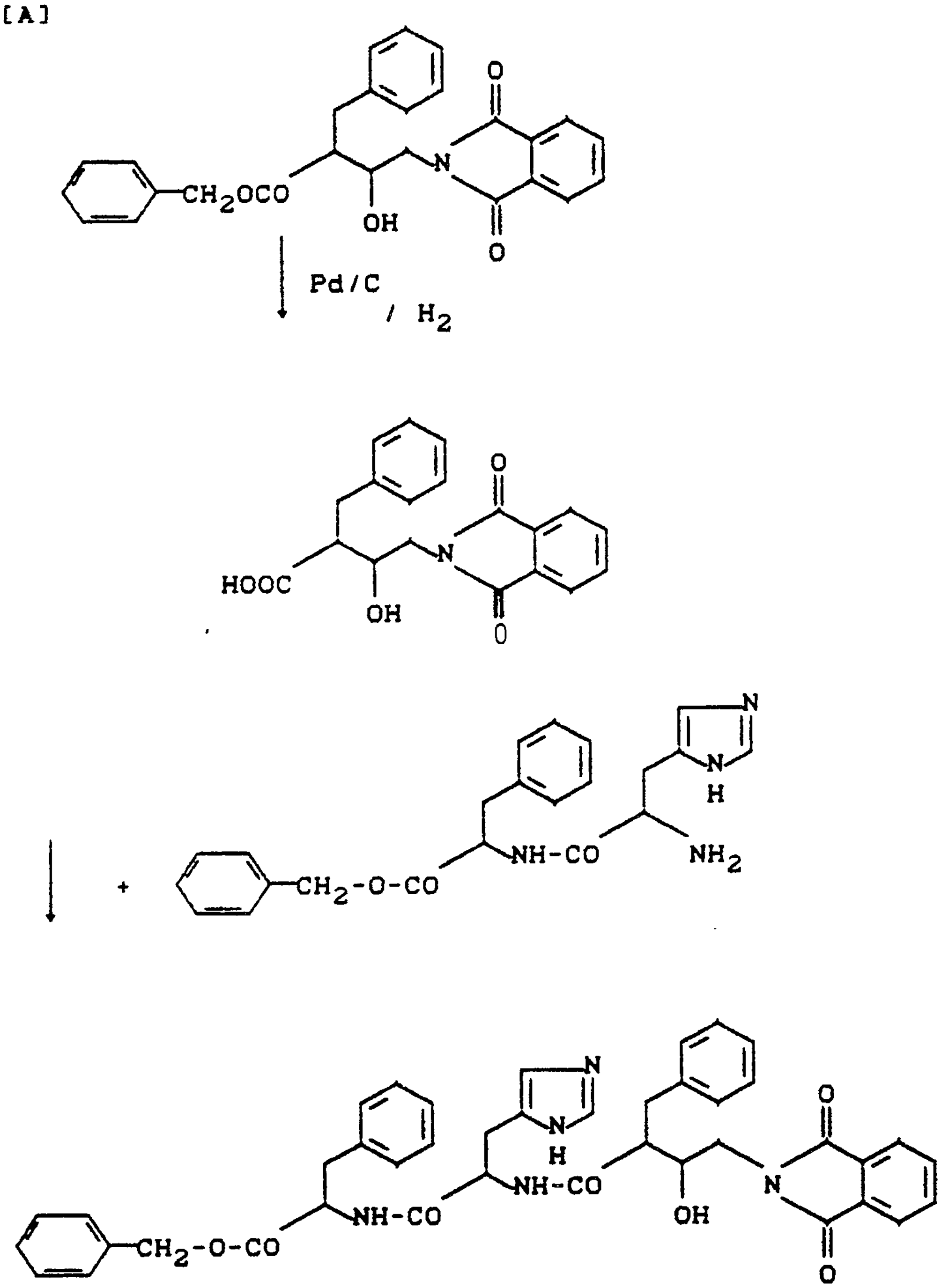

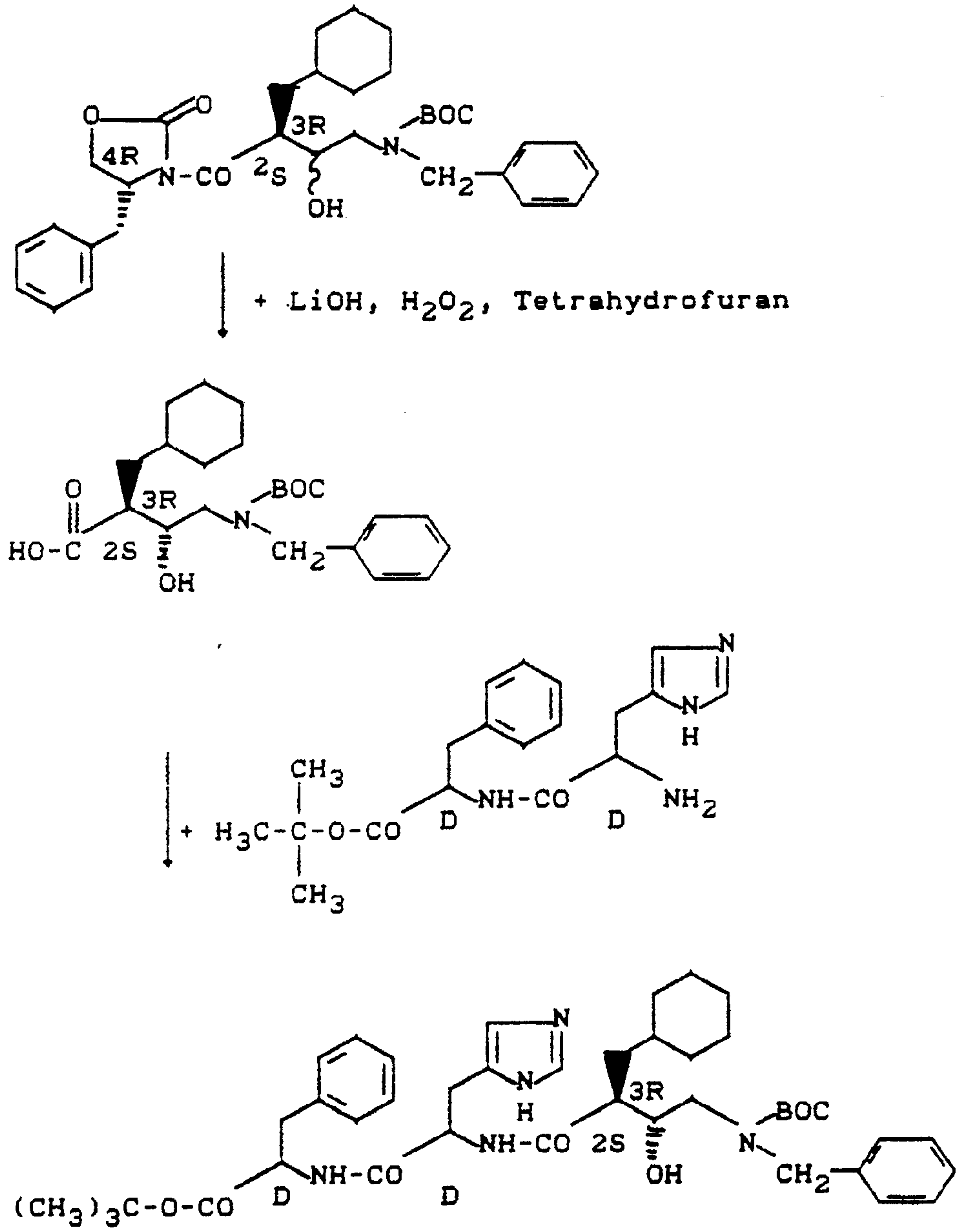
+ LiOH, H2O2, Tetrahydrofuran

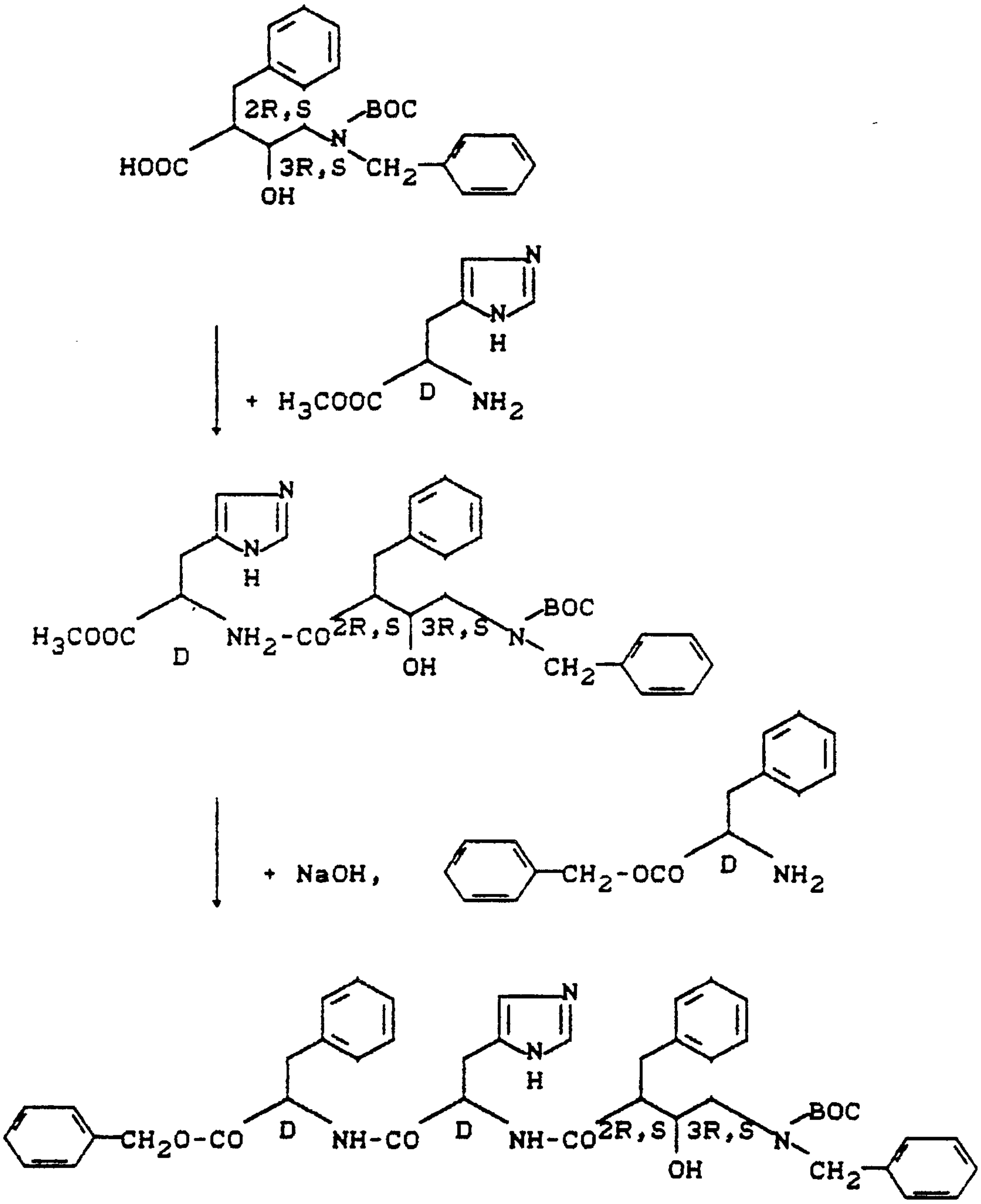


Als Hilfsstoffe werden bevorzugt Kondensationsmittel eingesetzt, die auch Basen sein können, insbesondere wenn die Carboxylgrupppe als Anhydrid aktiviert vorliegt. Bevorzugt werden hier die üblichen Kondensationsmittel wie Carbodiimide z.B. N,N′-Diethyl-, N,N′-Dipropyl-, N,N′-Diisopropyl-, N,N′-Dicyclohexylcarbodiimid, N-(3-Dimethylaminoisopropyl)-N′-ethylcarbodiimid Hydrochlorid, oder Carbonylverbindungen wie Carbonyldiimidazol, oder 1,2-Oxazoliumverbindungen wie 2-Ethyl-5-phenyl-1,2-oxazolium-3-sulfonat oder 2-tert-Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Benzotriazolyloxy-tris(dimethylamino)phosphonium-hexafluorophosphat, oder als Basen Alkalicarbonate z.B. Natrium-oder Kaliumcarbonat oder -hydrogencarbonat, oder organische Basen wie Trialkylamine z.B. Triethylamin, N-Ethylmorpholin oder N-Methylpiperidin eingesetzt.

Als Lösemittel eignen sich die üblichen inerten Lösemittel, die sich unter den jeweils gewählten Reaktionsbedingungen, vor allem der jeweils gewählten Aktivierungmethode nicht verändern. Hierzu gehören bevorzugt Wasser oder organische Lösemittel wie Methanol, Ethanol, Propanol, Isopropanol, oder Ether wie Diethylether, Glykolmono- oder dimethylether, Dioxan oder Tetrahydrofuran, oder Kohlenwasserstoffe wie Benzol, Toluol, Xylol, Cyclohexan oder Erdölfraktionen, oder Halogenkohlenwasserstoffe wie Methylenchlorid, Chloroform, Tetrachlorkohlenstoff, oder Aceton, Dimethylsulfoxid, Dimethylformamid, Hexamethylphosphorsäuretriamid, Essigester, Pyridin, Triethylamin oder Picolin. Ebenso ist es möglich, Gemische der genannten Lösemittel zu verwenden.

Als aktivierte Carboxylgruppen eignen sich beispielsweise:
Carbonsäureazide (erhältlich z.B. durch Umsetzung von geschützten oder ungeschützten Carbonsäurehydraziden mit salpetiger Säure, deren Salzen oder Alkylnitriten (z.B. Isoamylnitrit),
oder ungesättigte Ester, insbesondere Vinylester, (erhältlich z.B. durch Umsetzung eines entsprechenden Esters mit Vinylacetat), Carbamoylvinylester (erhältlich z.B. durch Umsetzung einer entsprechenden Säure mit einem Isoxazoliumreagenz), Alkoxyvinylester (erhältlich z.B. durch Umsetzung der entsprechenden Säuren mit Alkoxyacetylenen, bevorzugt Ethoxyacetylen),
oder Amidinoester z.B. N,N′- bzw. N,N-disubstituierte Amidinoester (erhältlich z.B. durch Umsetzung der entsprechenden Säure mit einem N,N′-disubsituierten Carbodiimid (bevorzugt Dicyclohexylcarbodiimid, Diisopropylcarbodiimid oder N-(3-Dimethylaminopropyl)-N′-ethylcarbodiimidhydrochlorid) oder mit einem N,N-disubstituierten Cyanamid, oder Arylester, insbesondere durch elektronenziehende Substituenten substituierte Phenylester, z.B. 4-Nitrophenyl-, 4-Methylsulfonylphenyl-, 2,4,5-Trichlorphenyl-, 2,3,4,5,6-Pentachlorphenyl-, 4-Phenyldiazophenylester (erhältlich z.B. durch Umsetzung der entsprechenden Säure mit einem entsprechend substituierten Phenol, gegebenenfalls in Anwesenheit eines Kondensationsmittels wie z.B. N,N′-Dicyclohexylcarbodiimid, Diisopropylcarbodiimid, N-(3-Dimethylaminopropyl)-N′-ethylcarbodiimid-Hydrochlorid, Isobutylchloroformat, Propanphosphonsäureanhydrid), Benzotriazolyloxytris-(dimethylamino)phosphoniumhexafluorphosphat, oder Cyanmethylester (erhältlich z.B. durch Umsetzung der entsprechenden Säure mit Chloracetonitril in Gegenwart einer Base),
oder Thioester, insbesondere Nitrophenylthioester (erhältlich z.B. durch Umsetzung der entsprechenden Säure mit Nitrothiophenolen, gegebenenfalls in Gegenwart von Kondensationsmitteln wie N,N′-Dicyclohexylcarbodiimid, Diisopropylcarbodiimid, N-(3-Dimethylaminopropyl)-N′-ethylcarbodiimid-Hydrochlorid, Isobutylchloroformat, Propanphosphonsäureanhydrid, Benzotriazolyloxytris (dimethylamino)-phosphoniumhexafluorphosphat), oder Amino- bzw. Amidoester (erhältlich z.B. durch Umsetzung der entsprechenden Säure mit einer N-Hydroxyamino- bzw. N-Hydroxyamido-Verbindung, insbesondere N-Hydroxy-succinimid, N-Hydroxypiperidin, N-Hydroxy-phthalimid, N-Hydroxy-5-norbornen-2,3-dicarbonsäureimid oder 1-Hydroxybenzotriazol, gegebenenfalls in Anwesenheit von Kondensationsmitteln wie N,N′-Dicyclohexylcarbodiimid, Diisopropylcarbodiimid oder N-(3-Dimethylaminopropyl)- N′-ethylcarbodiimid-Hydrochlorid, Isobutylchloroformat oder Propanphosphonsäureanhydrid),
oder Anhydride von Säuren, bevorzugt symmetrische oder unsymmetrische Anhydride der entsprechenden Säuren, insbesondere Anhydride mit anorganischen Säuren (erhältlich z.B. durch Umsetzung der entsprechenden Säure mit Thionylchlorid, Phosphorpentoxid oder Oxalylchlorid), oder Anhydride mit Kohlensäurehalbderivaten z.B. Kohlensäureniederalkylhalbester (erhältlich z.B. durch Umsetzung der entsprechenden Säure mit Halogenameisensäureniedrigalkylestern, z.B.Chlorameisensäuremethylester, -ethylester, -propylester, -isopropylester, -butylester oder -isobutylester oder mit 1-Niedrigalkoxycarbonyl-2-niedrigalkoxy-1,2-dihydro-chinolin, z.B. 1-Methoxycarbonyl-2-ethoxy-1,2-dihydrochinolin),
oder Anhydride mit Dihalogenphosphorsäuren (erhältlich z.B. durch Umsetzung der entsprechenden Säure mit Phosphoroxychlorid),
oder Anhydride mit Phosphorsäurederivaten oder Phosphorigsäurederivaten, (z.B. Propanphosphonsäureanhydrid, H. Wissmann und H.J. Kleiner, Angew. Chem. Int Ed. 19 , 133 (1980))
oder Anhydride mit organischen Carbonsäuren (erhältlich z.B. durch Umsetzung der entsprechenden Säuren mit einem gegebenenfalls substituierten Niederalkan- oder Phenylalkancarbonsäurehalogenid, insbesondere Phenylessigsäure, Pivalinsäure- oder Trifluoressigsäurechlorid),
oder Anhydride mit organischen Sulfonsäuren (erhältlich z.B. durch Umsetzung eines Alkalisalzes einer entsprechenden Säure mit einem Sulfonsäurehalogenid, insbesondere Methan-, Ethan-, Benzol- oder Toluolsulfonsäurechlorid),
oder symmetrische Anhydride (erhältlich z.B. durch Kondensation entsprechender Säuren, gegebenenfalls in Gegenwart von Kondensationsmitteln wie N,N′-Dicyclohexylcarbodiimid, Diisopropylcarbodiimid, N-(3-Dimethylaminopropyl)-N-ethylcarbodiimid-Hydrochlorid Isobutylchloroformat, Propanphosphonsäureanhydrid oder Benzotriazolyloxy-tris(dimethylamino)phosphoniumhexafluorphosphat.

Reaktionsfähige cyclische Amide sind insbesondere Amide mit fünfgliedrigen Heterocyclen mit 2 Stickstoffatomen und gegebenenfalls aromatischem Charakter, bevorzugt Amide mit Imidazolen oder Pyrazolen (erhältlich z.B. durch Umsetzung der entsprechenden Säuren mit N,N′-Carbonyldiimidazol oder -gegebenenfalls in Gegenwart von Kondensationsmitteln wie z.B. N,N′-Dicyclohexylcarbodiimid, N,N-Diisopropylcarbodiimid, N-(3-Dimethylaminopropyl)-N′-ethylcarbodiimid-Hydrochlorid, Isobutylchloroformat, Propanphosphonsäureanhydrid, Benzotriazolyloxy-tris(dimethylamino)phosphonium-hexafluorphosphat- mit z.B. 3,5-Dimethyl-pyrazol, 1,2,4-Triazol oder Tetrazol.

Ein komplementierendes Bruchstück mit freier Aminogruppe ist Ammoniak, ein primäres oder sekundäres Amin, oder eine Aminosäure oder ein Peptidrest, der nach den beschriebenen Methode A,B getrennt hergestellt wurde.

Die an der Reaktion teilnehmende Aminogruppe in einem komplementierenden Bruchstück einer erfindungsgemäßen Verbindung liegt bevorzugt in freier Form vor, insbesondere dann, wenn die damit zur Umsetzung gebrachte Carboxylgruppe in aktivierter Form eingesetzt wird. Sie kann jedoch ebenso selbst aktiviert sein. Eine solche reaktive Form ist beispielsweise ein Isocyanat oder ein Carbaminsäurehalogenid.

Ist das komplementierende Bruchstück mit einer freien Aminogruppe Ammoniak, ein mono- oder ein disubstituiertes Amin so kann auch ein entsprechender Harnstoff eine reaktive Form dieses Amins sein.

Die Durchführung des erfindungsgemäßen Verfahrens erfolgt in an sich bekannter Weise, wobei die Reaktionsbedingungen entsprechend der Art der Carboxylgruppenaktivierung gewählt werden. Üblicherweise wird das Verfahren in Gegenwart geeigneter Löse- bzw Verdünnungsmittel, gegebenenfalls in Anwesenheit eines Hilfsstoffes in einem Temperaturbereich von -80°C bis 300°C, bevorzugt von -30°C bis 200°C bei normalem Druck durchgeführt. Ebenso ist es möglich, bei erhöhtem oder erniedrigtem Druck zu arbeiten.

Die Herstellung der erfindungsgemäßen Verbindung ist auch nach weiteren üblichen Varianten des beschriebenen Verfahrens durchgeführt worden (vgl. z.B. Houben-Weyls "Methoden der organischen Chemie" XV/1 und 2; M. Bodanszky, A. Bodanszky in "The Practice of Peptide Synthesis", Springer Verlag, Berlin, 1984; George R. Pettit in "Synthetic Peptides", Volume 4, Elsevier Scientific Publishing Company, Amsterdam-Oxford-New York, 1976; E. Gross und J. Meienhofer (Editors) in "The Peptides", Vol. 1-3, Academic Press, New York-London-Toronto-Sydney-San Francisco, 1981; M. Bodanszky in "Principles of Peptide Synthesis", Springer Verlag, Berlin-Heidelberg-New York-Tokyo, 1984; R. Uhmann und K. Radscheit, Offenlegungsschrift, DE 3 411 244 A1) oder auch nach der "Solid-Phase-Methode", wie sie beispielsweise von M. Bodanszky, A. Bodanszky in "The Practice of Peptide Synthesis", Springer-Verlag, Berlin, 1984, oder G. Barany, R.B. Merrifield in "Solid-Phase Peptide Synthesis" aus "The Peptides", Vol 2, S. 3-254, edited by E. Gross, J. Meienhofer, Academic Press, New York-London-Toronto-Sydney-San Francisco (1980) beschrieben wird.

Die Verseifung der Carbonsäureester erfolgt nach üblichen Methoden, indem man die Ester in inerten Lösemitteln mit üblichen Basen behandelt, wobei die zunächst entstehenden Salze durch Behandeln mit Säure in die freien Carbonsäuren überführt werden können.

Als Basen eignen sich für die Verseifung die üblichen anorganischen Basen. Hierzu gehören bevorzugt Alkalihydroxide oder Erdalkalihydroxide wie beispielsweise Natriumhydroxid, Kaliumhydroxid oder Bariumhydroxid oder Alkalicarbonate wie Natrium- oder Kaliumcarbonat oder Natriumhydrogencarbonat, oder Alkalialkoholate wie Natriummethanolat, Natriumethanolat, Kaliummethanolat, Kaliumethanolat oder Kalium-tert.-butanolat. Besonders bevorzugt werden Natriumhydroxid oder Kaliumhydroxid eingesetzt.

Als Lösemittel eignen sich für die Verseifung Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Die Verseifung wird im allgemeinen in einem Temperaturbereich von 0°C bis +100°C, bevorzugt von +20°C bis +80°C durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Bei der Durchführung der Verseifung wird die Base im allgemeinen in einer Menge von 1 bis 3 mol, bevorzugt von 1 bis 1,5 mol bezogen auf 1 mol des Esters bzw. des Lactons eingesetzt. Besonders bevorzugt verwendet man molare Mengen der Reaktanten.

Die Abspaltung der Ester kann außerdem nach üblicher Methode mit Säuren, wie beispielsweise Chlorwasserstoffsäure oder Trifluoressigsäure im Fall der tert.-Butylester oder durch Hydrogenolyse in Anwesenheit von Katalysatoren wie beispielsweise Pd/Kohlenstoff oder Pt/Kohlenstoff im Fall der Benzylester erfolgen.

Die Abspaltung des substituierten Oxazolidin-2-on-Rings (Gruppe Y) aus den Verbindungen der allgemeinen Formel (III) erfolgt in einem der oben aufgeführten inerten Lösungsmitteln oder Wasser, bevorzugt in Gemischen von Ethern mit Wasser, beispielsweise Tetrahydrofuran/Wasser mit Wasserstoffperoxid und Alkalihydroxiden, vorzugsweise mit Lithiumhydroxid nach bekannter Methode, (vgl. D.A. [Evans, T.C. Britton, J.R. Ellman, THL., Vol. 28 , No. 49, pp, 6141-6144, 1987).

Die Verbindungen der allgemeinen Formel (II) sind neu und können hergestellt werden, indem man Verbindungen der allgemeinen Formel (V)

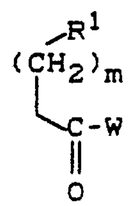

(V)

in welcher
$R^1$, W und m die oben angegebene Bedeutung haben mit Verbindungen der allgemeinen Formel (VI)

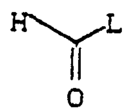

(VI)

in welcher
L die oben angegebene Bedeutung hat,
in einem der oben aufgeführten inerten Lösungsmitteln, gegebenenfalls in Anwesenheit einer Base kondensiert.

Die Reaktion kann durch folgendes Formelschema erläutert werden.

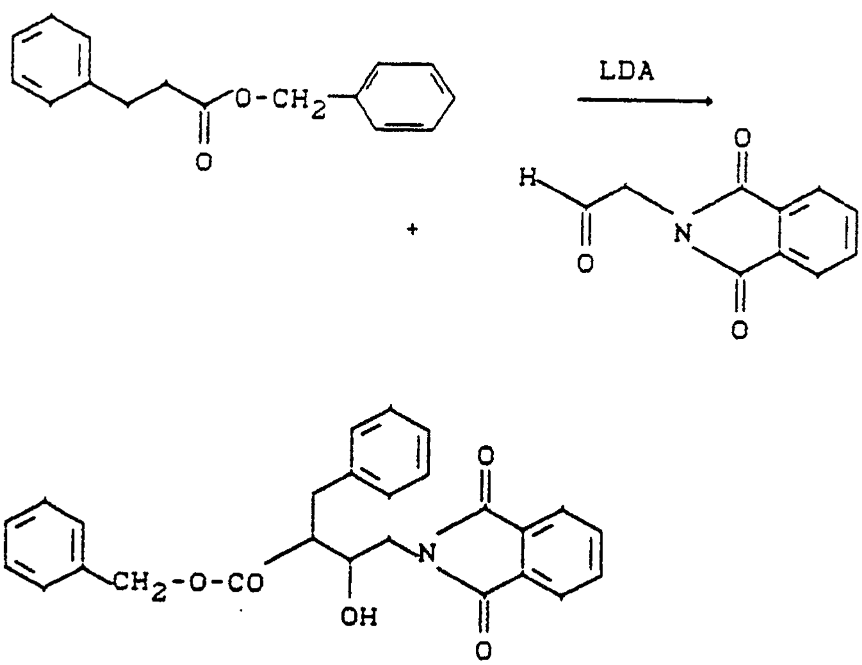


Als Lösungsmittel bei der Kondensation eignen sich die oben aufgeführten inerten Lösungsmittel, bevorzugt sind Ether wie Diethylether, Dioxan oder Tetrahydrofuran.

Als Basen für die Kondensation eignen sich Alkalihydroxide, Boralkyle, Alkaliamide oder metallorganische Verbindungen, wie beispielsweise Lithiumdiisopropylamid, Dibutylborantriflat, Natrium-bis(-trimethylsilyl)amid oder Natriumhydrid.

Die Kondensation kann gegebenenfalls über chirale Enolate nach bekannter Methode verlaufen (vgl. D.A. Evans, T.C. Britten, J. Am. Chem. Soc. 1987 , 109, 6881-6883, J. Am. Chem. Soc. 1981 , 103, 2172-2129).

Die Reaktion verläuft in einem Temperaturbereich von -80° C bis +30° C, vorzugsweise bei -70° C bis 0° C bei Normaldruck.

Die Verbindungen der allgemeinen Formel (V) sind an sich bekannt oder können nach üblicher Methode hergestellt werden (vgl. Beilstein 21, 481, Beilstein 9, 511 und J. March, Advanced Organic Chemistry, Second Editon 1977)).

Die Verbindungen der allgemeinen Formel (VI) sind teilweise bekannt oder neu.
Sie sind in den Fällen neu, wenn $R^2$ für die Benzylgruppe oder für die tert.-Butylgruppe steht und $R^3$ für den oben aufgeführten Rest -$COR^9$ steht, worin $R^9$ die dort angegebene Bedeutung hat.

Sie können hergestellt werden, indem man entweder die entsprechenden Alkohole mit den üblichen Oxidationsmitteln, vorzugsweise Pyridiniumchlorochromat, umsetzt, oder die entsprechenden Ester mit den üblichen Reduktionsmitteln, beispielsweise Diisobutylaluminiumhydrid, nach bekannter Methode in einem der oben aufgeführten Lösemitteln bei Raumtemperatur reduziert

Die Verbindungen der allgemeinen Formel (III) sind ebenfalls neu und können hergestellt werden, indem man

Verbindungen der allgemeinen Formel (VII)

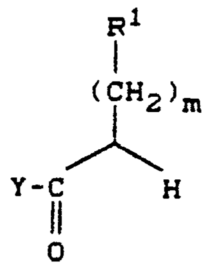

(VII)

in welcher
Y, $R^1$ und m die oben angegebene Bedeutung haben,
nach der oben aufgeführten Methode mit Verbindungen der allgemeinen Formel (VI) umsetzt.

Die Gruppe Y in den Verbindungen der allgemeinen Formel (IV) fungiert als chirales Hilfsreagenz, d.h. die vorgegebene Konfiguration im jeweiligen Oxazolidin-2-on-Ring induziert stereoselektiv die entsprechende Konfiguration an den oben aufgeführten Stereozentren 2 und 3 in den Verbindungen der allgemeinen Formel (III) und somit auch in den erfindungsgemäßen Verbindungen der allgemeinen Formel (I).

Außerdem ist es möglich, Stereoisomerengemische, insbesondere Diastereomerengemische, in an sich bekannter Weise, z.B. durch fraktionierte Kristallisation, Chromatographie oder Craigverteilung in die einzelnen Isomere aufzutrennen (vgl. "Verteilungsverfahren im Laboratorium", E. Hecker, Verlag Chemie GmbH, Weinheim, Bergstr. (1955)).

Racemate können in an sich bekannter Weise, z.B. durch Überführung der optischen Antipoden in Diastereomere gespalten werden.

Die Verbindungen der allgemeinen Formel (VII) sind an sich bekannt oder können nach üblicher Methode hergestellt werden (vgl. J. J. Plattner et al; J. Med. Chem 1988, 31 , (12), 2277-2288).

Die als Ausgangsstoffe eingesetzten Aminosäuren in der Definition A, B, D und E und somit auch die Verbindungen der allgemeinen Formel (IV) sind an sich bekannt oder können nach bekannter Methode hergestellt werden bzw. sind natürliche Aminosäuren [Houben-Weyl; "Methoden der organischen Chemie", Band XVI 1 und 2].

Salze der erfindungsgemäßen Verbindungen mit salzbildenden Gruppen können in an sich bekannter Weise hergestellt werden, zum Beispiel durch Umsetzung der erfindungsgemäßen Verbindungen die saure Gruppen enthalten, mit entsprechenden Säuren, jeweils bevorzugt mit den vorne genannten Basen bzw. Säuren.

Die erfindungsgemäßen Verbindungen, insbesondere diejenigen mit D-konfigurierten Aminosäuren, besitzen überraschenderweise eine kreislaufbeeinflussende Wirkung. Sie können deshalb in Arzneimitteln zur Behandlung des Bluthochdrucks und Herzinsuffizienz eingesetzt werden.

In vitro Test

Die inhibitorische Stärke der erfindungsgemäßen Peptide gegen endogenes Renin vom Humanplasma wird in vitro bestimmt. Gepooltes Humanplasma wird unter Zusatz von Ethylendiamintetraessigsäure (EDTA) als Antikoagulanz erhalten und bei -20 °C gelagert. Die Plasmareninaktivität (PRA) wird als Bildungsrate von Angiotensin I aus endogenem Angiotensinogen und Renin nach Inkubation bei 37 °C bestimmt. Die Reaktionslösung enthält 150 µl Plasma, 3 µl 6,6%ige 8-Hydroxychinolinsulfatlösung, 3 µl 10%ige Dimercaprollösung und 144 µl Natriumphosphatpuffer (0,2 M; 0,1% EDTA; pH 5,6) mit oder ohne den erfindungsgemäßen Stoffen in verschiedenen Konzentrationen. Das pro Zeiteinheit gebildete Angiotensin I wird mit einem Radioimmunoassay (Sorin Biomedica, Italien) bestimmt.

Die prozentuale Inhibition der Plasmareninaktivität wird berechnet durch Vergleich der hier beanspruchten Substanzen. Der Konzentrationsbereich, in dem die hier beanspruchten Substanzen eine 50% Inhibition der Plasmareninaktivität zeigen, liegen zwischen $10^{-4}$ bis $10^{-9}$ M.

| Beispiel Nr. | Inhibition bei 0,05 mg/ml [%] |
|---|---|
| 70 | 100 |
| 71 | 92 |
| 72 | 100 |
| 74 | 93 |
| 75 | 65 |
| 76 | 78 |
| 77 | 100 |
| 78 | 100 |
| 82 | 100 |
| 83 | 100 |
| 85 | 60 |
| 86 | 80 |
| 88 | 100 |
| 89 | 100 |
| 90 | 63 |
| 92 | 100 |
| 93 | 100 |
| 95 | 100 |
| 103 | 88 |
| 105 | 100 |
| 108 | 100 |
| 107* | 100 |

Der $IC_{50}$-Wert des Beispiels 107 wurde mit $4,3 \cdot 10^{-8}$ M bestimmt.

Der $IC_{50}$-Wert des Beispiels 169 wurde mit $9,6 \cdot 10^{-9}$ M bestimmt.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt:

Wasser, nicht-toxische organische Lösungsmittel, wie Paraffine (z.B. Erdölfraktionen), pflanzliche Öle (z.B. Erdnuß/Sesamöl), Alkohole (z.B: Ethylalkohol, Glycerin), Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel (z.B. Polyoxyethylen-Fettsäure-Ester), Polyoxyethylen-Fettalkohol-Ether (z.B. Lignin-Sulfitablaugen, Methylcellulose, Stärke und Polyvinylpyrrolidon) und Gleitmittel (z.B. Magnesiumstearat, Talkum, Stearinsäure und Natriumsulfat).

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen können die Wirkstoffe außer den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden. Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht des Versuchstieres bzw. der Art der Applikation, aber auch aufgrund der Tierart und deren individuellem Verhalten gegenüber dem Medikament bzw. deren Art von dessen Formulierung und dem Zeitpunkt bzw. Intervall, zu welchem die Verabreichung erfolgt.

So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehrere Einzelgaben über den Tag zu verteilen. Für die Applikation in der Humanmedizin ist der gleiche Dosierungsspielraum vorgesehen. Sinngemäß gelten hierbei auch die obigen Ausführungen.

Erklärungen zum experimentellen Teil:

DC-Systeme

Stationäre Phase

Merck DC-Fertigplatten Kieselgel 60 F-254, 5 x 10 cm, Schichtdicke 0,25 mm, Art.-Nr. 5719.

Mobile Phasen (im Test als "DC-System")

I: $CH_2Cl_2$/MeOH 9:1
II: $CH_2Cl_2$/MeOH 95:5
III: $NH_3$/$CH_2Cl_2$/MeOH 0,2:9:1
IV: Essigsäure/$CH_2Cl_2$/MeOH 0,2:9:1
V: Eisessig/n-Butanol/$H_2O$ 1:3:1
VI: EtOAc/n-Hexan 2:1
VII: EtOAc/n-Hexan 1:3
VIII: EtOAc/n-Hexan 1:1
IX: $CH_2Cl_2$/MeOH 98:2
X: $CH_2Cl_2$/MeOH 7:3
XI: $CH_2Cl_2$
XII: Toluol/EtOAc 4:1
XIII: $CH_2Cl_2$/n-Hexan 4:1
XIV: $CH_2CO_2$/n-Hexan 9:1
XV: EtOAc/n-Hexan 1:5
XVI: EtOH/$H_2O$ 3:1

HPLC-Systeme:

HPLC-System I:

Säule Merck, Lichrosorb [R] RP-8, 250-4, 10 µm, Kat. Nr. 50318

HPLC-System II:

Säure Merck, Lichrosorb [R] RP-18, 250-4, 10 µm, Kat.Nr. 50334

Eluens bei System I und II

A: pH 7,00 Phosphatpuffer, Merck, Art.-Nr. 9439/$H_2O$ 1:50 B: Acetonitril A/B wie 1/1, Fluß: 2 ml/min, isokratisch, Detektion: 254 nm

HPLC-System III:

Säule Nucleosil 120-5 C 18, 5 µm, 125 x 4 mm Eluens;
A = 0,01 M $H_3PO_4$, B = Acetonitril
Eluentenprogramm:
0-1 min: 10 % B
1-9 min: Gradient mit 10 % B/min
9-13 min: 90 % B
Fluß: 2 ml/min, Raumtemperatur
5 µl, Probenmenge ca 1 mg/ml
Detektion: UV-Diodenarray bei 210 nm

HPLC-System IV:

Säule Chiral-AG P
Hersteller: Chrom-Tech-AB/Stockholm mobile Phase: 0,02 M Phospat pH = 6,5 und 8 % Acetonitril, Raumtemperatur,
Druck 42 bar, Fließrate 0,5 ml/min,
DET 225 nm.

HPLC-System V:

Säule Nucleosil 120-5 $C_{18}$ mobile Phase, Phosphatpuffer, Acetonitril 45:55, isokratisch gemischt, Temp 35° C, Flußrate 2 ml/min

HPLC-System VI:

Säule Lichrosorb RP 8, 5 µm; Phosphat pH 7, 1:10 verd; Acetonitril 45:55

HPLC-System VII (chiral):

Säule: wie unter HPLC-System IV;
Mobile Phase: Eluens A = 1,2 g $NaH_2PO_4$ und 0,2 g $Na_2HPO_4$/1 l $H_2O$; Eluens B = Acetonitril; A : B = 96 : 4;
alle weiteren Parameter wie unter IV.

GC-System I (chiral):

S. 264 = 25 M Cyclohexyl-Glycin-t-butylamid, Ofen: 80 - 200° C; 4° C/min.
0,4 bar Wasserstoff, Split
Inj : 230° C, FID-Det: 280° C, GC: 4*10
Injektion: Mikroliter Lösung

<u>Verzeichnis der benutzten Abkürzunaen</u>

| 1. allgemeine analytische Methoden | |
|---|---|
| DC | Dünnschichtchromatographie |
| PDC | präparative Dickschichtchromatographie |
| GC | Gaschromatographie |
| HPLC | Hochdruckflüssigkeitschromtographie |
| SC | Säulenchromatographie |
| NMR | Kernspinresonanzspektroskopie (Protonen) |
| MS | Massenspektrometrie (Elektronenstoßionisation) |
| (+)FAB-MS | Fast-atomatic-bombardement-Massenspektrometrie, positive Ionen, Matrixsubstanz; m-Nitrobenzylalkohol |
| MS-DCI | Massenspektrometrie, chemische Ionisation |

2. Aminosäuren

Im allgemeinen erfolgt die Bezeichnung der Konfiguration durch das Vorausstellen eines L bzw. D vor der Aminosäureabkürzung, im Fall des Racemats durch ein D,L-wobei zur Vereinfachung bei L-Aminosäuren die Konfigurationsbezeichnung unterbleiben kann und dann nur im Fall der D-Form bzw. des D,L-Gemisches explizierte Bezeichnung erfolgt.

| a) natürliche Aminosäuren | |
|---|---|
| Ala | L-Alanin |
| Arg | L-Arginin |
| Asn | L-Asparagin |
| Asp | L-Asparaginsäure |
| Cys | L-Cystein |
| Gln | L-Glutamin |
| Glu | L-Glutaminsäure |
| Gly | L-Glycin |
| His | L-Histidin |
| Ile | L-Isoleucin |
| Leu | L-Leucin |
| Lys | L-Lysin |
| Met | L-Methionin |
| Orn | L-Ornithin |
| Phe | L-Phenylalanin |
| Ser | L-Serin |
| Sar | L-Sarcosin (N-Methylglycin) |
| Thr | L-Threonin |
| Trp | L-Tryptophan |
| Tyr | L-Tyrosin |
| Val | L-Valin |

| b) unnatürliche Aminosäuren | |
|---|---|
| D- oder L-Nal(1) | β-(1-Naphthyl)-D- oder -L-alanin |
| D- oder L-Nal(2) | β-(2-Naphthyl)-D- oder -L-alanin |
| D- oder L-Phg | D- oder L-Phenylglycin |
| D- oder L-Pyr(2) | β-(2-Pyridyl)-D- oder -L-alanin |
| D- oder L-Pyr(3) | β-(3-Pyridyl)-D- oder -L-alanin |
| D- oder L-Pyr(4) | β-(4-Pyridyl)-D- oder -L-alanin |
| D- oder L-Trz(1) | β-(1-Triazolyl)-D- oder -L-alanin |
| D- oder L-Phe(4I) | β-(4-Iodophenyl)-D- oder -L-alanin |
| D- oder L-Phe(4OCH$_3$) | β-(4-Methoxyphenyl)-D- oder -L-alanin |

| 3. Aktivierungsgruppen | |
|---|---|
| HOBT | 1-Hydroxybenzotriazol |
| HOSU | N-Hydroxysuccinimid |

| 4. Kupplungsreagenzien | |
|---|---|
| DCC | Dicyclohexylcarbodiimid |
| DPPA | Diphenylphosphorylazid |
| PPA | n-Propanphosphonsäureanhydrid |
| BOP | Benzotriazolyloxy-tris(dimethylamino)phosphoniumhexafluorphosphat |
| WSC | N-(3-Dimethylaminopropyl)-N′-ethylcarbodiimidhydrochlorid |

| 5. Reagentien | |
|---|---|
| NEM | N-Ethylmorpholin |
| NMM | N-Methylmorpholin |
| DIPEA | Diisopropylethylamin |
| TEA | Triethylamin |
| TFA | Trifluoressigsäure |

| 6. Lösemittel | |
|---|---|
| HOAc | Essigsäure |
| DMF | Dimethylformamid |
| EtOAc | Essigsäureethylester |
| MeOH | Methanol |
| EtOH | Ethanol |
| THF | Tetrahydrofuran |
| DMSO | Dimethylsulfoxid |
| HMPT | Hexamethylphosphorsäuretriamid |

| 7. Schutzgruppen | |
|---|---|
| Boc | Tert-Butoxycarbonyl |
| Z | Benzyloxycarbonyl |
| DNP | Dinitrophenyl |
| Fmoc | 9-Fluorenylmethoxycarbonyl |
| OEt | Ethylester |
| OMe | Methylester |

| 8. Sonstige | |
|---|---|
| DCU | N,N′-Dicyclohexylharnstoff |

Beispiel I

3-Phenylpropionsäurebenzylester

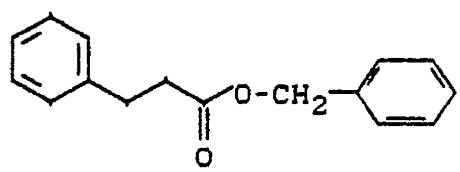

485 g (3,23 mol) 3-Phenylpropionsäure und 440 ml (4,25 mol) Benzylalkohol werden in 4 l Dichlormethan gelöst. Nach Zugabe von 4 g (0,0323 mol) Dimethylaminopyridin wird wird die Mischung auf 5°C gekühlt und bei dieser Temperatur tropfenweise unter Rühren mit einer Lösung von 732 g (3,55 mol) Dicyclohexylcarbodiimid versetzt.

Die Suspension wird über Nacht auf Raumtemperatur kommen gelassen. Der ausgefallene Dicyclohexylharnstoff wird abgesaugt und das Filtrat mit 75 g (1 mol) Glycin versetzt. Nach dreistündigem Rühren bei Raumtemperatur wird vom Rückstand filtriert und die organischen Lösungsmittel im Vakuum abgezogen. Der Rückstand wird im Hochvakuum destilliert.
Ausbeute: 476 g gelbes Öl (61 % der Theorie)
Kp: 145 - 148°C bei 1 mbar
MS-EI: 240 ($M^+$)

Beispiel II

N-tert.-Butoxycarbonyl-N-benzyl-glycinethylester

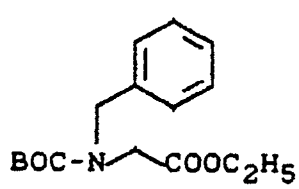

300 g (1,552 mol) N-Benzylglycinethylester (Aldrich) und 236 ml (1,707 mol) Triethylamin werden in 1200 ml Tetrahydrofuran gelöst. Zu der gerührten Mischung gibt man unter Eiskühlung tropfenweise eine Lösung von 338,3 g (1,552 mol) Di-tert.-butyl-dicarbonat (Fluka) in 300 ml Dichlormethan. Man rührt über Nacht nach und läßt den Ansatz hierbei auf Raumtemperatur kommen.

Die Mischung wird mit halbkonzentrierter Salzsäure auf pH 3 gestellt und am Rotationsverdampfer bei einer Badtemperatur von 30 °C vom organischen Lösungsmittel befreit. Der Rückstand wird mit Wasser aufgefüllt und dann dreimal mit je 1 l Essigester extrahiert. Die gereinigten organischen Phasen werden mit gesättigter Kochsalzlösung gewaschen, über Natriumsulfat getrocknet, filtriert und einrotiert.

Der Rückstand wird zur Entfernung von Restmengen Essigester mehrmals mit Dichlormethan gelöst, einrotiert und am Hochvakuum getrocknet.
Ausbeute: 464 g (100 % der Theorie)
DC-System II: Rf = 0,94
HPLC-System II: Rt = 10,54 min

Beispiel III

N-tert.-Butoxycarbonyl-N-benzyl-ethanolamin

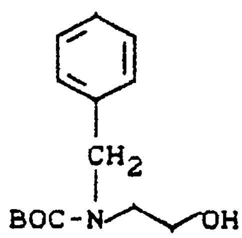

189 g (0,644 mol) der Verbindung aus Beispiel II werden in 1,8 l trockenem Tetrahydrofuran vorgelegt. Unter Rühren gibt man portionsweise 97,4 g (2,58 mol) Natriumborhydrid hinzu.

Bei leichtem Rückfluß (Badtemperatur 50 °C) tropft man vorsichtig innerhalb einer Stunde 900 ml Methanol p.A. zu der Mischung. Der Ansatz wird über Nacht am Rückfluß gekocht, abgekühlt und mit halbkonzentrierter Salzsäure auf pH 6 gestellt. Die organischen Lösungsmittel werden abrotiert. Der Rückstand wird mit Wasser aufgefüllt und dreimal mit je 1 l Diethylether extrahiert. Die vereinigten organischen Phasen werden mit Salzsäure pH 3, gesättigter Bicarbonatlösung und ges. Kochsalzlösung gewaschen, über Natriumsulfat getrocknet, filtriert und eingeengt.
Ausbeute: 144,4 g (89,2 % der Theorie)
DC-System II: Rf = 0,49
DC-System VIII: Rf = 0,53
(+)FAB-MS: m/z 252 (M + H)

Beispiel IV

N-tert.-Butoxycarbonyl-N-benzyl-glycinaldehyd

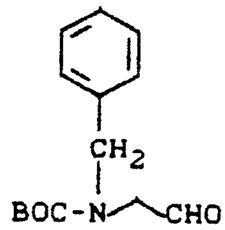

Variante A

14,65 g (58,3 mmol) der Verbindung aus Beispiel III und 37,3 g (175 mmol) Pyridiniumchlorochromat (Aldrich) werden in 500 ml Dichlormethan gegeben und 2 Stunden bei Raumtemperatur gerührt.

Der Ansatz wird einrotiert und über eine Fritte mit 600 g Kieselgel 60 (Merck) und einem Stufengradienten von 1 l Dichlormethan, 1 l Dichlormethan/Methanol 98/2 und 1,5 l Dichlormethan/Methanol 95 : 5 kurzfiltriert. Es wurden 500 ml Fraktionen genommen, die produktenthaltenden Eluate vereinigt, über Natriumsulfat getrocknet und am Rotationsverdampfer eingeengt.
Ausbeute: 4 g (27,5 % der Theorie)

Variante B

270 g (0,92 mmol) der Verbindung aus Beispiel II werden in 2 l trockenem Tetrahydrofuran gelöst und unter Stickstoff und Rühren bei - 60°C tropfenweise mit 1,84 l 1N-Diisobutylaluminiumhydridlösung in Hexan (Aldrich) versetzt. Es wird 35 Minuten bei dieser Temperatur nachgerührt und dann mit 5 l 1 M wäßriger Kalium-Natriumtartrat-Lösung hydrolysiert, wobei anfänglich starkes Schäumen auftritt. Man rührt eine Stunde bei Raumtemperatur nach, gibt 1 l Diethylether hinzu und trennt die organische Phase ab. Die wäßrige Phase wird zweimal mit je 1 l Diethylether extrahiert. Die vereinigten organischen Phasen werden mit gesättigter Kochsalzlösung gewaschen, über Natriumsulfat getrocknet, filtriert und zur Trockene eingeengt.

Das Rohprodukt (234 g) wird über 3,8 kg Kieselgel 60 (Merck, Art.-Nr. 9385), 0,040 - 0,063 mm (230 -400 mesh) und einem Stufengradienten aus 20 l Dichlormethan/Methanol 99 : 1, 2 l Dichlormethan/Methanol 98,5 : 1,5, 2 l Dichlormethan/Methanol 97/3, 5 l Dichlormethan/Methanol 9 : 1 und 4 l Dichlormethan/Methanol 8 : 2 chromatographiert. Nach Vereinigen und Einengen der produktenthaltenden Fraktion wird der Aldehyd am Hochvakuum getrocknet.
Ausbeute: 88,7 g (38,7 % der Theorie)
DC-System IX: Rf = 0,55
DC-System XI: Rf = 0,50
DC-System XIII: Rf = 0,19
DC-System XIV: Rf = 0,40
(+)FAB-MS: m/z 250 (M + H)

Beispiel V

N-Methyloxycarbonyl-D-valinol

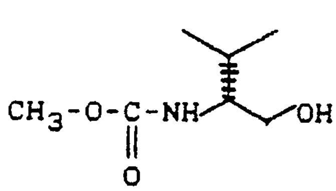

Unter Stickstoff werden 39,7 g (0,385 mol) D-Valinol (Aldrich) in 500 ml trockenem THF gelöst. Man

gibt 58,7 ml (0,424 mol) Triethylamin hinzu, kühlt auf - 20° C ab und tropft unter Rühren 32,8 ml (0,424 mol) Chlorameisensäuremethylester (Aldrich) zu der Mischung. Bei dieser Temperatur wird 30 Minuten nachgerührt und dann der Ansatz auf Raumtemperatur gebracht. Es wird 6,0 g (0,078 mol) Glycin hinzugegeben und noch einmal 15 Minuten nachgerührt. Der Niederschlag wird abgesaugt, das Filtrat eingeengt und in Diethylether aufgenommen. Die organische Phase wird mit 1 N HCl, ges. $NaHCO_3$-Lösung und ges. Kochsalzlösung gewaschen, über Natriumsulfat getrocknet, filtriert, eingeengt und im Hochvakuum getrocknet.
Ausbeute: 27,7 g (45 % der Theorie)
DC-System I: Rf = 0,59
MS-EI: m/z 162 ($M^+$)

Beispiel VI

(4R)-4-(2-Propyl)oxazolidin-2-on

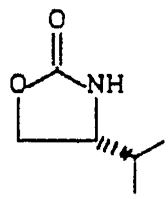

27,2 g (168,7 mmol) der Verbindung aus Beispiel V werden in 400 ml Toluol gelöst und unter Stickstoffeinleitung im Ölbad bei 150° C am Rückfluß gerührt.

Mit einem Wasserabscheider werden 100 ml feuchtes Toluol abgetrennt. Der Ansatz wird auf 100° C gekühlt und mit 1,5 g feingemahlenem und vakuumgetrocknetem Kaliumcarbonat versetzt. Die Temperatur wird auf 120° C erhöht. Nach etwa 15 Minuten schäumt die Mischung spontan auf, wobei Methanol (Sp 64° C) abdestilliert (7,5 ml, ber. Menge 6,8 ml) wird. Anschließend werden bei 140° C Badtemperatur weitere 70 ml Toluol abdestilliert. Der Ansatz wird heiß vom $K_2CO_3$ abfiltriert und am Vakuum eingeengt. Der kristalline Rückstand wird mit 50 ml n-Hexan verrieben, abgesaugt und am Hochvakuum getrocknet.
Ausbeute: 19,8 g (91 % der Theorie)
Fp: 71° C (Lit. 71 - 72° C)
DC-System IX: Rf > 0,40
MS-EI: m/z 129 ($M^+$)
GC-System I: Rt = 28,389 min, isomerenrein
Vergleich S-Enantiomer (Fluka): Rt = 28,884 min

Beispiel VII

(4R)-3-(3-Phenylpropionyl)-4-(2-propyl)oxazolidin-2-on

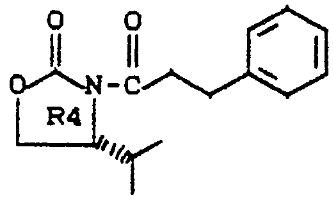

Die Titelverbindung wird nach dem für das S-Enantiomere beschriebene Verfahren (J. J. Plattner et. al., J. Med. Chem. 1988, 31 (12), 2277 - 2288), ausgehend von 19,4 g der Verbindung aus Beispiel VI und 25,3

ml (170 mmol) 3-Phenylpropionsäurechlorid dargestellt.
Ausbeute: 34 g (87,4 % der Theorie)
Fp: 62° C (Lit. 86,5 - 87,5° C; das S-Enantiomer wurde analog dargestellt und hatte ebenfalls einen Schmelzpunkt von 62° C)
DC-System XI: Rf = 0,63
HPLC-System II: Rt = 7,16 min
MS-EI: m/z 261 ($M^+$)

Beispiel VIII

(4R,5S)-3-(3-Phenylpropionyl)-4-Methyl-5-Phenyl-oxazolidin-2-on

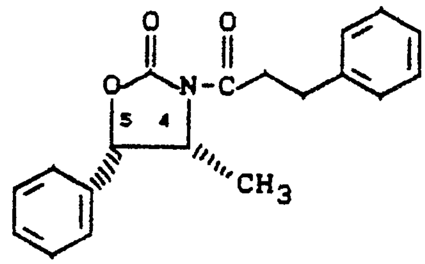

Die Titelverbindung wird analog Beispiel VII aus 25,1 g (141,6 mmol) 4R,5S-4-Methyl-5-phenyl-oxazolidin-2-on (Fluka) und 23,8 ml (142,4 mmol) Phenylpropionsäurechlorid erhalten.
Ausbeute: 40,3 g (92 % der Theorie)
Fp: 96° C (aus Ether/n-Hexan)
DC-System XI: Rf = 0,70
HPLC-System II: Rt = 12,72 min
MS-DCI: m/z 310 (M + H)

Beispiel IX

(4S)-3-(3-Phenylpropionyl)-4-phenyloxazolidin-2-on

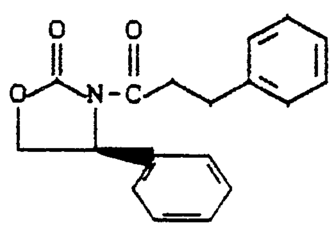

Die Titelverbindung wird analog Beispiel VII aus 19,75 g (121 mmol) (4S)-4-Phenyl-oxazolidin-2-on (Fluka) und 19,8 ml (133 mmol) Phenylpropionsäurechlorid erhalten.
Ausbeute: 34,6 g (97 % der Theorie)
Fp: 130 - 131° C (aus Ether/n-Hexan)
DC-System XI: Rf = 0,57
HPLC-System II: Rt = 7,26 min
MS-EI: m/z 295 ($M^+$)

Beispiel X

(4R)-3-(3-Phenylpropionyl)-4-Benzyloxazolidin-2-on

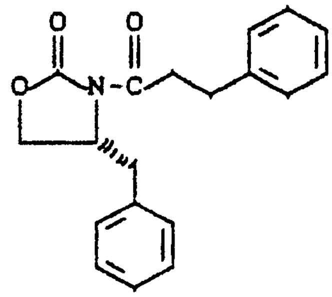

Die Titelverbindung wird analog Beispiel VII aus 25,45 g (143,6 mmol) (4R)-Benzyl-oxazolidin-2-on (Fluka) und 23,5 ml (158 mmol) Phenylpropionsäurechlorid erhalten.
Ausbeute: 40,2 g (95 % der Theorie)
Fp: 107 - 108° C (aus Ether/n-Hexan)
DC-System XI: Rf = 0,81
HPLC-System II: Rt = 9,55 min
MS-EI: m/z 309 ($M^+$)

Beispiel XI und Beispiel XII

(4S)-3-[(2R)-2-Benzyl-(3S)-3-hydroxy-3-phenylpropionyl]-4-(2-propyl)-oxazolidin-2-on (Beispiel XI)

(4S)-3-[(2R)-2-Benzyl-(3R)-3-hydroxy-3-phenylpropionyl]-4-(2-propyl)-oxazolidin-2-on (Beispiel XII)

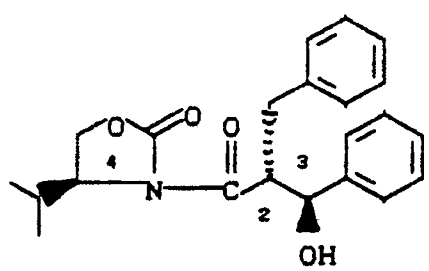

Beispiel XI

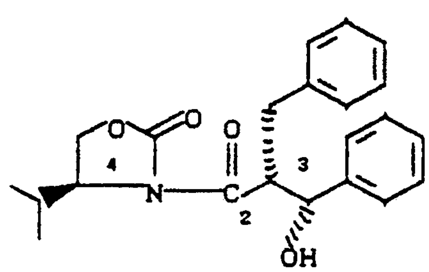

Beispiel XII

Unter Argon wird 1,55 ml (11 mmol) Diisopropylamin in 20 ml absolutem THF gelöst und bei -20° C unter Rühren mit 7,75 ml (12 mmol) n-Butyllithiumlösung (1,55 M in n-Hexan, Aldrich) versetzt. Man rührt

10 Minuten bei dieser Temperatur nach und gibt dann die so frisch hergestellte Lösung von Lithiumdiisopropylamid tropfenweise zu einer auf - 70 °C gekühlten Lösung von 2,61 g (10 mmol) (4S)-3-(3-Phenylpropionyl)-4-(2-propyl)-oxazolidin-2-on (J. J. Plattner et. al., J. Med. Chem. 1988, 31 (12), 2277 - 2288) in 30 ml absolutem THF. Man rührt 30 Minuten bei - 70 °C nach und spritzt über ein Septum 1,32 ml (13 mmol) Benzaldehyd zu der Mischung. Nach einer Stunde bei dieser Temperatur wird der Ansatz durch Zugabe von 100 ml ges. Ammoniumchloridlösung hydrolysiert, auf Raumtemperatur kommen lassen und mit 100 ml Diethylether versetzt. Die organische Phase wird abgetrennt und die wäßrige Phase mit 100 ml Diethylether extrahiert.

Die vereinigte organische Phase wird mit 1 N HCl, ges. Natriumhydrogencarbonat- und ges. Kochsalzlösung gewaschen, über $Na_2SO_4$ getrocknet und eingeengt.

Das Rohprodukt (34 g) wird an 150 g Kieselgel 60 (Merck) 40 bis 63 nm mit Dichlormethan als Laufmittel chromatographiert. Die produktenthaltnden Eluate werden zusammengefaßt und eingeengt. Die kristallinen Fraktionen werden mit n-Hexan verrührt, abgesaugt und getrocknet.

Ausbeute: 1,46 g Beispiel XI
0,13g Mischfraktion
0,53 g Beispiel XII
Gesamtausbeute: 2,12 g (57 % der Theorie)

Strukturzuordnung Beispiel XI

Vom Beispiel XI wurden aus Ether/n-Hexan Einkristalle erhalten und einer Röntgenstrukturanalyse unterzogen. Demnach kommt dem Beispiel XI die angegebene 4S, 2R, 3S-Konfiguration zu:

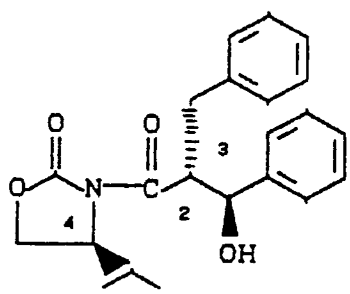

Strukturzuordnung Beispiel XII

Der Verbindung wurde nach Röntgenstrukturanalyse des Spiegelbildisomeren (Beispiel XIV) die 4S, 2R, 3R-Konfiguration zugeordnet.

Analytische Daten (Beispiel XI, 4S, 2R, 3S-Isomer)

Fp: 82 °C
DC-System XI: Rf = 0,28
HPLC-System II: Rt = 8,97 min
HPLC-System III: Rt = 7,287 min; Retentionsindex 895
(+)FAB-MS: m/z 368 (M + H); m/z 390 (M + Na)

Analytische Daten (Beispiel XII, 4S, 2R, 3R-Isomer)

Fp: 160 °C
DC-System XI: Rf = 0,16
HPLC-System II: Rt = 6,61 min
HPLC-System III: Rt 6,948 min; Retentionsindex 847
(+)FAB-MS: m/z 368 (M + H); m/z 390 (M + Na)

Beispiel XIII und Beispiel XIV

(4R)-3-[(2S)-2-Benzyl-(3R)-3-hydroxy-3-phenylpropionyl]-4-(2-propyl)-oxazolidin-2-on (Beispiel XIII)

(4R)-3-[(2S)-2-Benzyl-(3S)-3-hydroxy-3-phenylpropionyl]-4-(2-propyl)-oxazolidin-2-on (Beispiel XIV)

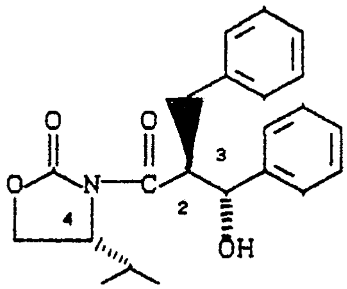

Beispiel XIII

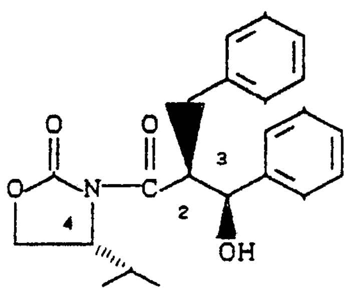

Beispiel XIV

Die Titelverbindungen werden analog Beispiel XI und XII durch Umsetzen des Lithiumanions von (4R)-3-(3-Phenylpropionyl)-4-(2-propyl)oxazolidin-2-on (Beispiel (VII)) mit Benzaldehyd und Chromatographie des Rohproduktes isomeren rein erhalten.

Strukturzuordnung

a) Beispiel XIII

Die Verbindung wurde nach Röntgenstrukturanalyse des Spiegelbildisomeren des Beispiels XI die 4R, 2S, 3R-Konfiguration zugeordnet.

b) Beispiel XIV

Vom Beispiel XIV wurden aus Ether/n-Hexan Einkristalle erhalten und einer Röntgenstrukturanalyse unterzogen. Demnach kommt der Verbindung die angegebene 4R, 2S, 3S-Konfiguration zu.

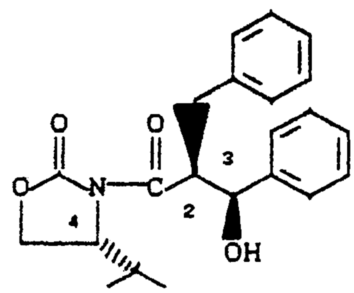

Die analysierten Daten (DC, HPLC und FAB) des Beispiels (XIII) sind identisch mit denen des Beispiels XI. Die analysierten Daten (DC, HPLC und FAB) des Beispiels XIV sind identisch mit denen des Beispiels XII.

Beispiel XV

(4R)-3-(3-Cyclohexylpropionyl)-4-Benzyloxazolidin-2-on

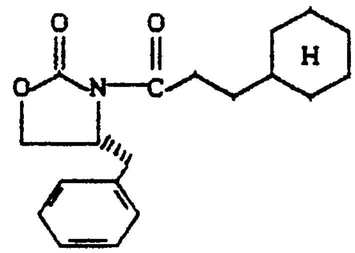

Die Titelverbindung wird analog Beispiel X aus 21,09 g (119 mmol) (4R)-Benzyloxazolidin-2-on (Fluka) und 22,8 g (22 ml, 130,6 mmol) Cyclohexylpropionsäurechlorid (3-Cyclohexylpropionsäurechlorid (3-Cyclohexylpropionsäure, $SOCl_2$, Δ; Destillation; Kp 0,3 mbar: 44 °C), erhalten.
Ausbeute: 33,2 g (88,4 % der Theorie)
Fp: 88 °C
DC-System XI: Rf = 0,63
HPLC-System II: Rt = 28,96 min
MS-DCI: m/z 316 (M + H)

Beispiel XVI

3-Phenylpropionsäure-(1-L-Carbethoxy)ethylester

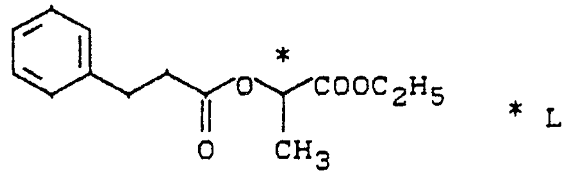

Die Titelverbindung wird analog Beispiel 1 durch Umsetzung von 37,24 g (0,25 mol) 3-Phenylpropionsäure und 26,4 g (0,22 mol) L-(-)-Milchsäureethylester in 600 ml $CH_2Cl_2$ mit 41,26 g DCC und 0,25 g DMAP erhalten. Der ausgefallene Dicyclohexylharnstoff (DCU) wird abgesaugt, die organische Phase wird mit

gesättigter Natriumbicarbonat-, verdünnter Salzsäure und gesättigter Natriumchloridlösung ausgeschüttelt, über Natriumsulfat getrocknet und eingeengt.
Ausbeute: 47,5 g (77 % der Theorie)
DC-System XI: Rf = 0,71
MS-EI: m/z 250 ($M^+$)
$^1$H-NMR (300 MHz, $CDCl_3$): δ = 7,22 (m, 5H); 5,06 (q, 1H); 4,18 (q, 2H); 2,98 (m, 2H); 2,71 (m, 2H); 1,45 (d, 3H); 1,25 (t, 3H)

Beispiel XVII

3-Phenylpropionsäure-(1-S-Carbethoxy)benzylester

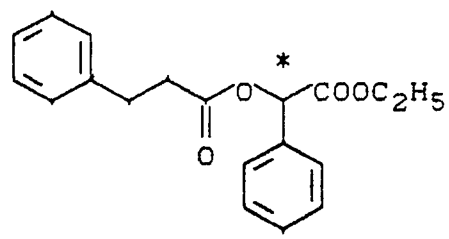

*(S)

Die Titelverbindung wird analog Beispiel XVI aus 30 g (0,2 mol) Phenylpropionsäure und 32,4 g (0,18 mol) (S)-(+)-Mandelsäureethylester durch DCC/DMAP-Kopplung und Standardaufarbeitung erhalten.
Ausbeute: 53 g (95 % der Theorie)
DC-System XI: Rf = 0,70
MS-DCI: m/z 313 (M + H); m/z 330 ($M + NH_4$)
$^1$H-NMR (250 MHz, DMSO): δ = 7,41 (m, 5H); 7,18 (m, 5H); 5,95 (s, 1H); 4,09 (m, 2H); 2,79 (m, 2H); 2,75 (m, 2H); 1,09 (t, 3H)

Beispiel XVIII

(4S)-3-(3-Cyclohexylpropionyl)-4-Benzyloxazolidin-2-on

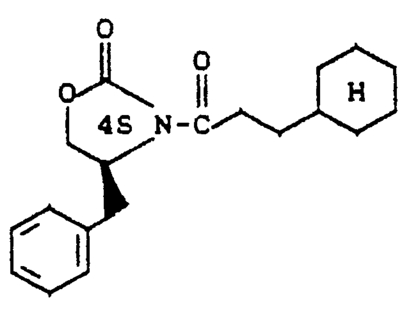

Die Titelverbindung wird analog Beispiel XV erhalten. In Abänderung wird (4S)-Benzyloxazolidin-2-on (Fluka) eingesetzt.

Analytische Daten: wie unter Beispiel XV.

Beispiel XIX

N-tert.-Butoxycarbonyl-L-Prolinal

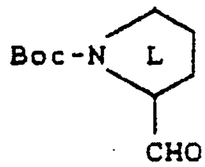

Die Titelverbindung wird ausgehend von N-tert.-Butoxycarbonyl-L-Prolinmethylester analog Beispiel IV, Variante B (Reduktion mit DIBAL) erhalten. Auf eine Chromatographie wird verzichtet.
Ausbeute: 52 g (81 % der Theorie)
(+)FAB-MS: m/z 200 (M + H).

Herstellungsbeispiele (allgemeine Formel I, II und III)

Beispiel 1

4-(N-Phthalyl)amino-2-R,S-benzyl-3-R,S-hydroxy-buttersäure-benzylester

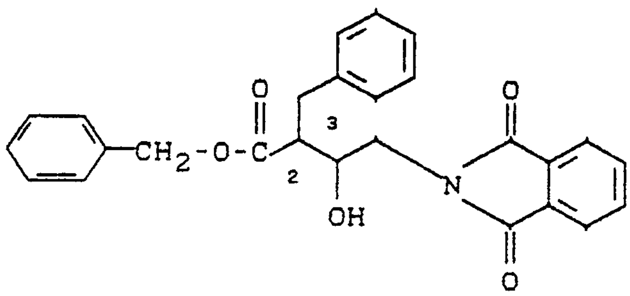

2R,S, 3R,S

66,7 ml (0,477 mol) Diisopropylamin werden in 300 ml absolutem Tetrahydrofuran unter Argon vorgelegt. Die Lösung wird auf - 20° C gekühlt, gerührt und tropfenweise mit 330 ml (0,528 mol) einer 1,6-molaren Lösung von n-Butyllithium in n-Hexan (Aldrich) versetzt, bei - 20° C wird 30 Minuten nachgerührt und sodann auf - 70° C gekühlt. Bei dieser Temperatur gibt man tropfenweise eine Lösung von 114,6 g (0,477 mol) der Verbindung aus Beispiel I hinzu und rührt 30 Minuten nach. Die so erhaltene klare gelbe Lösung wird innerhalb von 60 Stunden bei - 70° C und unter Argon in einer Lösung von 82,2 g (0,434 mol) N-Phthalylglycinaldehyd (CA 2913-97-5) in 300 ml absolutem Tetrahydrofuran gegeben. Man rührt 60 Minuten nach, läßt die Temperatur auf 0° C kommen und hydrolysiert den Ansatz mit 0,62 l 1 N HCl.

Die Mischung wird in einen Scheidetrichter gefüllt und über Nacht auf Raumtemperatur kommen lassen. Die organische Phase wird abgetrennt. Die wäßrige Phase wird auf pH 3 gestellt und zweimal mit je 7 l Diethylether extrahiert. Die vereinigten organischen Phasen werden je einmal mit gesättigter Natriumhydrogencarbonat-Lösung, 0,1 N Salzsäure und gesättigter Natriumchlorid-Lösung gewaschen, über Natriumsulfat getrocknet, filtriert und am Rotationsverdampfer eingeengt.

Das Rohprodukt (113 g gelbes Öl) wird über Kieselgel 60 (Merck, Art.-Nr. 9385), 0,040 - 0,063 mm (230 -400 mesh) mit einem Stufengradienten von je 2,5 l Dichlormethan, Dichlormethan/Methanol 98 : 2 und Dichlormehan/Methanol 96 : 4 filtriert. Die produktenthaltenden Fraktionen werden vereinigt und eingeengt.
Ausbeute: 44 g (23,6 % der Theorie);
Isomerengemisch, laut $^1$H-NMR = 4 : 1
DC-System I: Rf = 0,89
DC-System XI: Rf = 0,14

DC-System XII: Rf = 0,44
HPLC-System II: Rt = 10,36 min
(+)FAB-MS: m/z 430 (M + H); m/z 452 (M + Na)

Beispiel 2 und Beispiel 3

4-(N-Phthalyl)amino-2S- bzw. 2R-benzyl-3S bzw. 3R-hydroxy-buttersäure-benzylester (Beispiel 2, Enantiomerenpaar des Diastereomers A) und 4-(N-Phthalyl)amino-2S- bzw. 2R-benzyl-3R bzw. 3S-hydroxy-buttersäure-benzylester (Beispiel 3, Enantiomerenpaar des Diastereomers B)

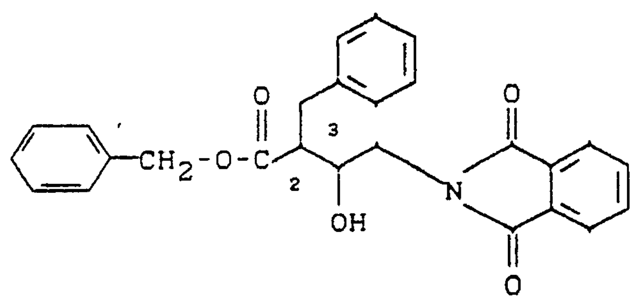

Beispiel 2: 2S, 3S und 2R, 3R;
Enantiomerenpaar des Diastereomers A
Beispiel 3: 2S, 3R und 2R, 3S;
Enantiomerenpaar des Diastereomers B

Das Isomerengemisch aus Beispiel 1 wird durch Säulenchromatographie an 600 g Kieselgel 60 (Merck, Art.-Nr. 9385), 0,040 - 0,063 mm (230 - 400 mesh) mit einem Stufengradienten von 8 l Dichlormethan/n-Hexan 4 : 1, 5 l Dichlormethan/Methanol 98 : 2, 5 l Dichlormethan/Methanol 95 : 5 und 2,5 l Dichlormethan/Methanol 9 : 1 getrennt. Es wurden 1 l Fraktionen genommen und die Isomerenreinheit über die $^{1}$H-NMR-Spektren bestimmt.

Die zuerst eluierten Fraktionen enthalten nur das Beispiel 2 (Diastereomer A, $^{1}$H-Signal bei $\delta$ = 5,45 ppm in DMSO, Dublett), gefolgt von Mischfraktionen aus Beispiel 2 und Beispiel 3 (Diastereomer B, $^{1}$H-Signal bei $\delta$ = 5,39 ppm in DMSO, Dublett). Die zuletzt eluierenden Mischfraktionen, die überwiegend das Beispiel 3 (Diastereomer B) mit Anteilen des Beispiels 2 (Diastereomer A) bis maximal 30 % enthielten, wurden vereinigt und an Kieselgel 60 rechromatographiert. Nach Durchführung der oben beschriebenen Prozedur wird so auch das Diastereomer B (Beispiel 3) erhalten.

Analytische Daten: wie unter Beispiel 1 beschrieben.

Die Zuordnung der 2S,3S/2R,3R-Konfiguration des Beispiels 2 bzw. der 2S,3R/2R,3S-Konfiguration des Beispiels 3 erfolgt über die chemische Verschiebung des Hydroxyldubletts am C-3 im $^{1}$H-NMR-Spekrum (DMSO) im Vergleich zu den Modellverbindungen der Beispiele XI/XIII (2R,3S/2S,3R; Dublett jeweils bei $\delta$ = 5,48 ppm) bzw. der Beispiele XII/XIV (2R,3R/2S,3S; Dublett jeweils bei $\delta$ = 5,64 ppm).

Beispiel 4

4-(N-tert.-Butoxycarbonyl-N-benzyl)amino-2R,S-benzyl-3R,S-hydroxy-buttersäure-benzylester

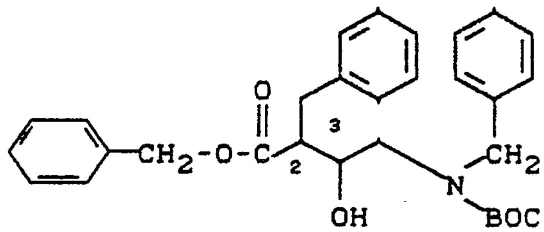

2 R,S
3 R,S

10,8 ml (77 mmol) Diisopropylamin werden in 100 ml absolutem THF unter Argon vorgelegt, auf -20 °C gekühlt und unter Rühren tropfenweise mit 53 ml (85 mmol) einer 15%igen Lösung von n-Butyllithium in Hexan versetzt. Es wird bei dieser Temperatur 30 Minuten nachgerührt. Dann kühlt man auf - 70 °C und tropft eine Lösung von 18,5 g (77 mmol) 3-Phenyl-propionsäurebenzylester (Beispiel I) in 50 ml absoluten THF hinzu. Nach 30-minütigem Nachrühren bei dieser Temperatur wird zu der Mischung eine Lösung von 17,2 g (69 mmol) der Verbindung aus Beispiel IV in 50 ml absolutem THF innerhalb von 20 Minuten zugetropft. Bei ansteigender Temperatur (Wegnahme des Kältebades) wird noch eine Stunde nachgerührt und dann der Ansatz unter Eisbadkühlung durch Zugabe von 170 ml 1 N HCl hydrolysiert.

Bei Raumtemperatur wird mit 150 ml Essigester verdünnt und von der organischen Phase abgetrennt. Die wäßrige Phase wird zweimal mit 100 ml Essigester extrahiert. Die vereinigten organischen Phasen werden mit gesättigter Natriumbicarbonat- und -chlorid-Lösung gewaschen, über Natriumsulfat getrocknet, filtriert und zur Trockene eingeengt.

Das Rohprodukt (35 g) wird an 1,7 kg Kieselgel 60 (Merck, Art.-Nr. 9385) 0,040 - 0,063 mm (230 - 400 mesh) mit einem Stufengradienten aus 1 l Petrolether (PE), 2 l PE/Essigester (EtOAc) 95 : 5, 2 l PE/EtOAc 9 : 1, 2 l PE/EtOAc 85 : 15 und 7 l PE/EtOAc 8 : 2 chromatographiert. Die produktenthaltenden Fraktionen werden vereinigt, vom Elutionsmittel abrotiert und zur Entfernung von Restspuren des Essigesters mehrmals mit Dichlormethan gelöst, eingeengt und schließlich am Hochvakuum getrocknet.

Ausbeute: 11,53 g (34 % der Theorie)
DC-System VII: Rf = 0,42
DC-System IX: Rf = 0,79
DC-System XV: Rf = 0,26
Stereoisomere nicht eindeutig unterscheidbar.
HPLC-System II: Rt = 50,44 min
(+)FAB-MS: m/z 490 (M + H)
MS-DCI: m/z 490 (M + H)

Eine Unterscheidung bzw. Zuordnung der Diastereomeren kann nicht eindeutig durchgeführt werden, da die $^{1}$H-NMR-Spektren durch Rotationsisomeren kompliziert werden (siehe $^{1}$H-NMR-Spektren der Beispiele II, III und IV).

Beispiel 5

4-(N-Phthalyl)amino-2R,S-benzyl-3R,S-hydroxybuttersäure

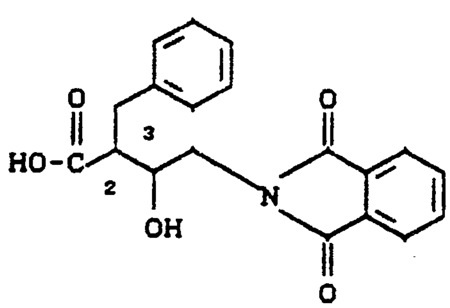

2 R,S
3 R,S

5 g (11,6 mmol) 4-(N-Phthalyl)amino-2R,S-benzyl-3R,S-hydroxybuttersäurebenzylester

(Diastereomerengemisch aus Beispiel 2 und 3 im Verhältnis 1 : 1) werden in 100 ml MeOH gelöst und nach Zugabe von 0,5 g Pd/C 10 % (Aldrich) bei 2 bar bis zur vollständigen Umsetzung hydriert. Man filtriert vom Katalysator ab, rotiert bei einer Badtemperatur von 30° C das Methanol ab und nimmt den Rückstand in verdünnter Natriumbicarbonatlösung (pH 8,5) auf. Es wird dreimal mit Ether gewaschen. Die basischwäßrige Phase wird mit 1 N HCl auf pH 2 angesäuert und das Produkt in Diethylether extrahiert. Man trocknet über Natriumsulfat, filtriert und engt am Rotationsverdampfer ein.
Ausbeute: 2,75 g (70 % der Theorie)
DC-System I: Rf = 0,50
DC-System III: Rf = 0,11
DC-System IV: Rf = 0,55
(Stereoisomere nicht eindeutig unterscheidbar)
HPLC-System VII: Rt = 3,88 min, 5,06 min, 5,61 min, 7,34 min (chiral)
MS-DCI: m/z 340 (M + H), m/z 357 (M + $NH_4$)

Beispiel 6

4-(N-Phthalyl)amino-2S- bzw. 2R-benzyl-3S- bzw. 3R-hydroxy-buttersäure (Enantiomerenpaar des Diastereomers A)

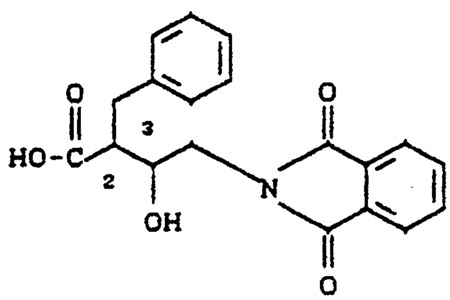

2S, 3S und
2R, 3R

Die Titelverbindung wird analog Beispiel 5 durch katalytische Hydrierung von 3,9 g (9 mmol) 4-(N-Phthalyl)-amino-2S-bzw. 2R-benzyl-3S- bzw. 3R-hydroxybuttersäure (Beispiel 2, Enantiomerenpaar des Diastereomers A) dargestellt.
Ausbeute: 2,6 g (85 % der Theorie)
DC-System III: Rf = 0,12
HPLC-System VII: Rt = 3,88 min und 5,06 min (chiral)
MS-DCI: m/z 340 (M + H) m/z 357 (M + $NH_4$)

Beispiel 7 und Beispiel 8

4-(N-Phthalyl)amino-2S-benzyl-3S-hydroxybuttersäure

4-(N-Phthalyl)amino-2R-benzyl-3R-hydroxybuttersäure

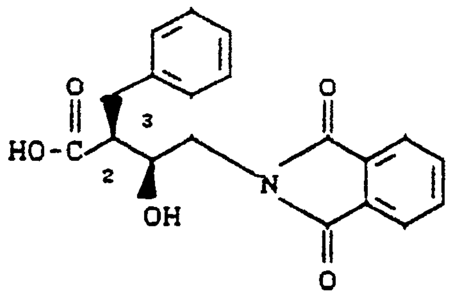

2S, 3S: Beispiel 7

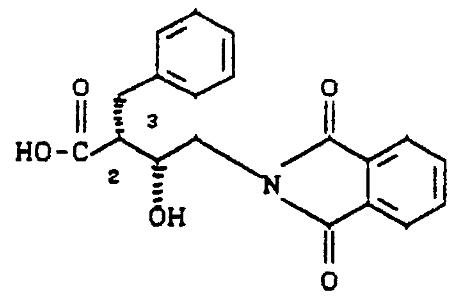

2R, 3R: Beispiel 8

Die Titelverbindungen werden durch Chromatographie des Enantiomerenpaares aus Beispiel 6 an einer chiralen Phase (Perlpolymerisat aus L-Menthylacrylamid, EP 218 089, Laufmittel Toluol/THF) in optisch reiner Form erhalten. Die Zuordnungen der Konfiguration erfolgt anhand von Vergleichsverbindungen.

Analytische Daten

Beispiel 7: $[\alpha]_D$ = + 13,2 (c = 0,7, $CHCl_3$)
Beispiel 8: $[\alpha]$D = - 11,0 (c = 0,6, $CHCl_3$)
HPLC-System VII(chiral): Beispiel 7: Rt = 3,88 min
Beispiel 8: Rt = 5,06 min

Alle anderen Daten (DC-Rf-Werte, $^1$H-NMR, MS-DCI) entsprechen den bereits bei Beispiel 6 aufgeführten.

Beispiel 9

4-(N-tert.-Butoxycarbonyl-N-benzyl)amino-2R,S-benzyl-3R,S-hydroxy-buttersäure

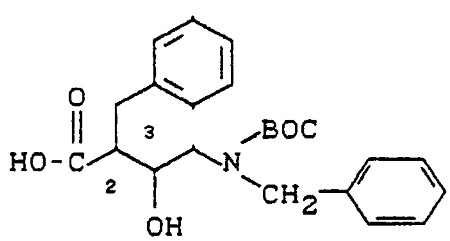

2 R,S
3 R,S

11,1 g (22,7 mmol) 4-(N-tert.-Butoxycarbonyl-N-benzyl)amino-2-R,S-benzyl-3-R,S-hydroxybuttersäurebenzylester (Beispiel 4) werden in 120 ml Methanol gelöst. Nach Zugabe von 1 ml 1 N HCl und 2 g Pd/C (10 %, Aldrich) wird die Mischung bei 3,5 bar Wasserstoffdruck bis zur vollständigen Umsetzung hydriert. Man filtriert den Katalysator ab, wäscht mit Methanol nach, zieht das Lösungsmittel am Rotationsverdampfer ab (Badtemperatur 30° C) und trocknet im Hochvakuum.
Ausbeute: 7,7 g (85 % der Theorie)
DC-System I: Rf = 0,58 (Diastereomere A)
Rf = 0,49 (Diastereomere B)
HPLC-System III: Rt 6,866 min, Retentionsindex 836 (Diastereomere A)
Rt = 6,959 min, Retentionsindex 849 (Diastereomere B)
HPLC-System IV: Rt = 12,30 min
15,50 min (Diastereomere A)
Rt = 24,13 min
32,57 min (Diastereomere B)
MS-DCI: m/z 400 (M + H); m/z 344; m/z 300

Beispiel 10 und Beispiel 11

4-(N-tert.-Butoxycarbonyl-N-benzyl)amino-2R- bzw. 2S-benzyl-3R- bzw. 3S-hydroxybuttersäure (Diastereomere A)

4-(N-tert.-Butoxycarbonyl-N-benzyl)amino-2R- bzw. 2S-benzyl-3S- bzw. 3R-hydroxybuttersäure (Diastereomere B)

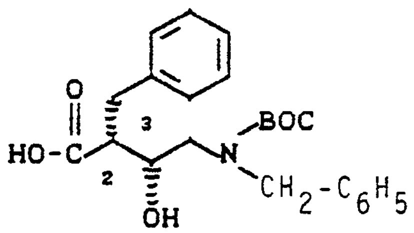

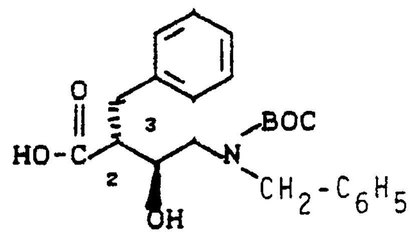

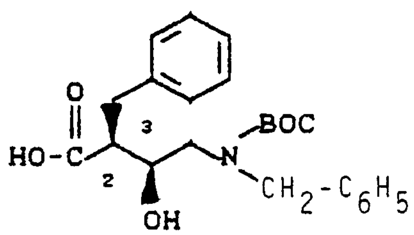

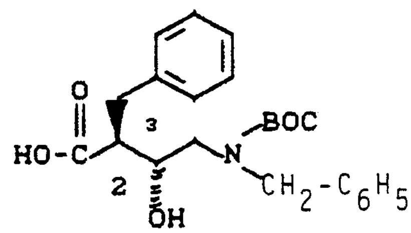

**Beispiel 10**
2R, 3R
2S, 3S
Diastereomere A

**Beispiel 11**
2R, 3S
2S, 3R
Diastereomere B

7,6 g 4-(N-tert.-Butoxycarbonyl-N-benzyl)amino-2-R,S-benzyl-3-R,S-hydroxybuttersäure (Beispiel 9) werden an 500 g Kieselgel 60 (Merck, Art.-Nr. 9385), 0,040 -0,063 mm mit einem Stufengradienten sus 1 l $CH_2Cl_2$, 1 l $CH_2Cl_2$/MeOH 99 : 1, 1 l $CH_2Cl_2$/MeOH 98 : 2, 1 l $CH_2Cl_2$/MeOH 97 : 3, 1 l $CH_2Cl_2$/MeOH 96 : 4 und 3 l $CH_2Cl_2$/MeOH 95 : 5 chromatographiert. Die produktenthaltenden Fraktionen werden vereinigt, eingeengt und am Hochvakuum getrocknet.
Ausbeute: 3,55 g Diastereomere A (Beispiel 10)
1,14 g Mischfraktion aus Beispiel 10 und 11
0,89 g Diastereomere B (Beispiel 11)

Die Zuordnung der absoluten Konfigurationen erfolgt durch Vergleich mit den enantiomeren reinen Verbindungen der Beispiele 34 bis 37 durch HPLC-Vergleich auf dem chiralen System IV.

Analytische Daten

Diastereomere A (Beispiel 10)

DC-System I: Rf = 0,58
HPLC-System III: Rt = 6,866 min, Retentionsindex 836
HPLC-System IV: Rt = 12,30 min und Rt = 15,50 min
MS-DCI: m/z 400 (M + H); m/z 356; m/z 300

Diastereomere B (Beispiel 11)

DC-System I: Rf = 0,49
HPLC-System III: Rt = 6,959 min, Retentionsindex 849
HPLC-System IV: Rt = 24,13 min und Rt = 32,57 min
MS-DCI: m/z 400 (M + H); m/z 356; m/z 300

Beispiel 12

(4R)-3-[4-(N-tert.-Butoxycarbonyl-N-benzyl)amino-(2S)-2-cyclohexylmethyl-(3R,S)-3-hydroxybutyryl]-4-benzyloxazolidin-2-on

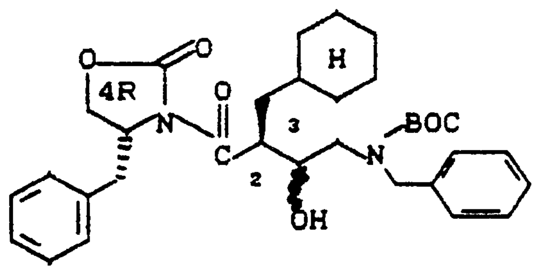

2S 3R,S

Die Titelverbindung wird analog Beispiel 22 ausgehend von der Verbindung aus Beispiel XV und dem Aldehyd aus Beispiel IV nach Chromatographie rein erhalten.
DC-System IX: Rf = 0,82 (4R,2S,3R-Isomer)
Rf = 0,78 (4R,2S,3S-Isomer)
HPLC-System II: Rt = 91,73 min (4R,2S,3S-Isomer)
Rt = 117,52 min (4R,2S,3R-Isomer)
(+)FAB-MS: m/z 565 (M + H); m/z 587 (M + Na)

Beispiel 13

4-(N-tert.-Butoxycarbonyl-N-benzyl)amino-(2S)-2-cyclohexylmethyl-(3R,S)-3-hydroxybuttersäure

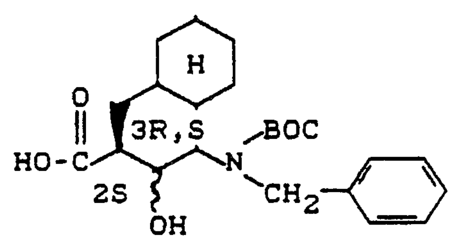

Die Titelverbindung wird durch Abspaltung des chiralen Hilfsreagenzes und nachfolgender Chromatographie (wie in Beispiel 34 beschrieben) aus der Verbindung des Beispiels 12 erhalten.
DC-System II: Rf = 0,42; 2S,3S-Isomer,
0,38; 2S,3R-Isomer,
HPLC-System III: Rt 7,605 min (Ret-index 956),
2S,3S-Isomer
Rt 7,754 min (Ret-index 979)

2S,3R-Isomer
(+)FAB-MS: m/z 444 (M+K), m/z 482 (M+2K-H); m/z 520 (M+3K-2H).

Beispiel 14

(4R)-3-[4-(N-tert.-Butoxycarbonyl-N-benzyl)amino-(2S)-2-benzyl-(3R,S)-3-hydroxybutyryl])-4-(2-propyl)-oxazolidin-2-on

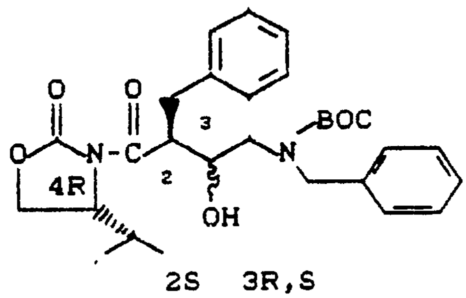

2S 3R,S

Unter Stickstoff werden 3,32 ml (23,6 mmol) Diisopropylamin in 40 ml absolutem THF gelöst, auf -20 °C gekühlt und unter Rühren mit 16,1 ml (25,8 mmol) einer 1,6 N-Lösung von n-Butyllithium (n-Hexan) versetzt. Man läßt 10 Minuten nachrühren und kühlt auf - 70 °C. Bei dieser Temperatur gibt man eine Lösung von 5,61 g (21,5 mmol) (4R)-3-(3-Phenylpropionyl)-4-(2-propyl)oxazolidin-2-on (Beispiel VII) in 60 ml trockenem HF hinzu und rührt 30 Minuten nach. Bei - 70 °C wird die Mischung tropfenweise mit einer Lösung von 5,9 g (23,65 mmol) N-tert.-Butoxycarbonyl-N-benzylglycinal (Beispiel IV) versetzt und 2 Stunden nachgerührt.

Der Ansatz wird mit 250 ml ges. $NH_4Cl$-Lösung hydrolysiert, auf Raumtemperatur gebracht und mit 300 ml Diethylether versetzt. Die organische Phase wird abge trennt und die wäßrige Phase einmal mit 300 ml Diethylether extrahiert. Die vereinigten Etherphasen werden mit gesättigter NaCl-Lösung gewaschen, über Natriumsulfat getrocknet und eingeengt.

Die Rohausbeute (11,75 g) wird an 580 g Kieselgel 60 (Merck, Art.-Nr. 9385) 40 - 63 µm (230 - 400 mesh) mit einem Gradienten aus je 1 l Petrolether/Essigester von 95 : 5 auf 84 : 16 (12 absteigende Stufen in 1 % Schritten) und schließlich 6 l Petrolether/Essigester 83 : 17 chromatographiert.

Die produktenthaltenden Fraktionen werden zusammengefaßt, wobei die Stereoisomeren mittels der HPLC detektiert und entsprechend vereinigt werden. Als Nebenkomponente wird auch das 4R, 2R, 3R-Isomere erhalten. Zuerst eluiert das 4R, 2S, 3R-, dann das 4R, 2R, 3R (Nebenkomponente) und schließlich das 4R, 2S, 3S-Isomere (umgekehrt zur Elutionsfolge in den reversed Phase HPLC-Systemen II und V).
Gesamtausbeute: 5,88 g (53 % der Theorie)

Analytische Daten aus dem Rohgemisch

DC-System XI: Rf = 0,36
HPLC-System II:
Rt = 22,80 min Isomer 4R, 2S, 3S
Rt = 26,87 min Isomer 4R, 2R, 3R (Nebenkomponente)
Rt = 27,01 min Isomer 4R, 2S, 3R
HPLC-System V:
Rt = 12,07 min Isomer 4R, 2S, 3S
Rt = 13,65 min Isomer 4R, 2R, 3R (Nebenkomponente)
Rt = 14,07 min Isomer 4R, 2S, 3R

Die Zuordnung der Konfigurationen erfolgt mit den entsprechenden Reinsubstanzen. Für die beiden Hauptprodukte werden hierzu $^1$H-NMR- bzw. HPLC-Datenvergleiche mit den Modellverbindungen der Beispiele XI - XIV durchgeführt. Die wesentlichsten Kriterien sind

1. die relativen Unterschiede der $^1H$-Absorptionslagen (in DMSO)
   a) der Hydroxylprotonen,
   b) der $H_4$-Oxazolidinonprotonen und
2. die Elutionsreihenfolgen auf den reversed phase HPLC-Systemen II und V.

Die Konfigurationszuordnung der Nebenkomponente erfolgt durch Überführen in die freie Säure (Abspaltung des optischen Hilfsreagenzes) und HPLC-Vergleich (chirales System IV) mit den Hauptprodukten der entsprechenden S-Reihe (siehe unter Beispiel 48).

Beispiele 15, 16 und 17

(4R)-3-[4-(N-tert.-Butoxycarbonyl-N-benzyl)amino-(2S)-2-benzyl-(3R)-3-hydroxy-butyryl]-4-(2-propyl)-oxazolidin-2-on (Beispiel 15)

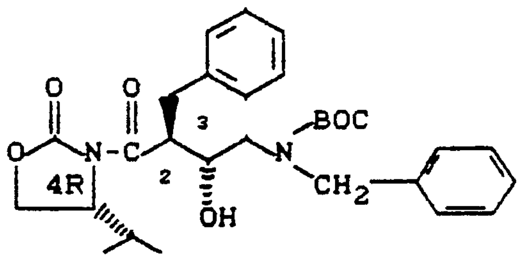

2S, 3R

(15)

(4R)-3-[4-(N-tert.-Butoxycarbonyl-N-benzyl)amino-(2R)-2-benzyl-(3R)-3-hydroxy-butyryl]-4-(2-propyl)-oxazolidin-2-on (Beispiel 16)

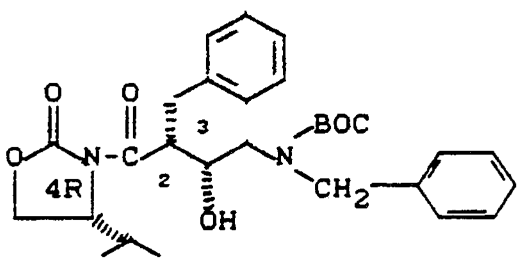

2R, 3R

(16)

Nebenkomponente

(4R)-3-[4-(N-tert.-Butoxycarbonyl-N-benzyl)amino-(2S)-2-benzyl-(3S)-3-hydroxy-butyryl]-4-(2-propyl)-oxazolidin-2-on (Beispiel 17)

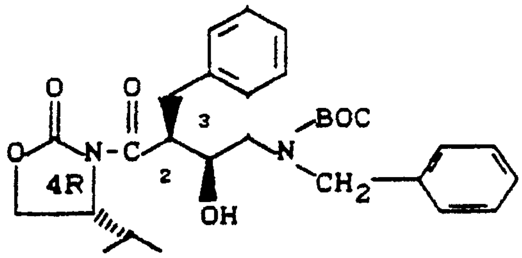

(17)

4R 2S, 3S

Die Titelverbindungen werden durch Kieselgelchromatographie des Beispiels 14 rein erhalten. Mischfraktionen können gegebenenfalls rechromatographiert werden.

Analytische Daten des Beispiels 15 (4R, 2S, 3R-Isomer)

HPLC-System II: Rt = 27,01 min
HPLC-System III: Rt = 8,381 min, Retentionsindex 1070
HPLC-System V: Rt = 14,07 min
$[\alpha]_D$ = - 90,75 (c = 1, Methanol)
(+)FAB-MS = m/z 511 (M + H); m/z = 533 (M + Na)

Analytische Daten des Beispiels 16 (4R, 2R, 3R-Isomer)

HPLC-System II: Rt = 26,87 min
HPLC-System V: Rt = 13,65 min
$[\alpha]_D$ = - 41,20 (c = 1, Methanol)
(+)FAB-MS = m/z 511 (M + H); m/z = 533 (M + Na)

Analytische Daten des Beispiels 17 (4R, 2S, 3S-Isomer)

HPLC-System II: Rt = 22,80 min
HPLC-System III: Rt = 8,299 min, Retentionsindex 1045
HPLC-System V: Rt = 12,07 min
$[\alpha]_D$ = - 58,88 (c = 1, Methanol)
(+)FAB-MS = m/z 511 (M + H); m/z = 533 (M + Na)

Vom Beispiel 17 wurden aus Ether/n-Hexan Einkristalle erhalten und einer Röntgenstrukturanalyse unterzogen. Hierdurch wurde die unter Beispiel 14 beschriebene Strukturzuordnung bestätigt. Dem Beispiel 17 kommt die abgebildete 4R,2S,3S-Konfiguration zu.

Beispiel 18

(4S)-3-[4-(N-tert.-butoxycarbonyl-N-benzyl)amino-(2R)-2-benzyl-(3R,S)-3-hydroxy-butyryl]-4-(2-propyl)-oxazolidin-2-on

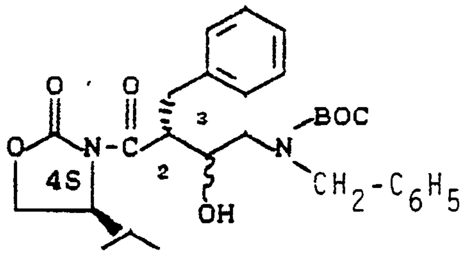

2R, 3R,S

Die Titelverbindung wird analog Beispiel 14 durch Umsetzung des Lithiumanions von 21,2 g (81 mmol) (4S)-3-(3-Phenyl-propionyl)-4-(2-propyl)oxazolidin-2-on (J.J. Plattner et al., J. Med. Chem. 1988, 31 (12), 2277 -2288) mit 22,2 g (89 mmol) des Aldehydes aus Beispiel IV erhalten. Als Nebenprodukt entsteht das 4S, 2S, 3S-Isomere (analog Beispiel 14), die Zuordnung erfolgt wie dort bereits beschrieben.
DC-System IX: Rf = 0,76
Rf = 0,16
HPLC-System II:
Rt = 22,80 min Isomer 4S, 2R, 3R
Rt = 26,86 min Isomer 4S, 2S, 3S (Nebenprodukt)
Rt = 27,00 min Isomer 4S, 2R, 3S
(+)FAB-MS: m/z 511 (m + H); m/z 533 (M + Na)
Gesamtausbeute: 25,68 g (62,1 % der Theorie) (nach Trennung in die Isomeren Beispiele 19, 20 und 21)

Beispiele 19, 20 und 21

(4S)-3-[4-(N-tert.-butoxycarbonyl-N-benzyl)amino-(2R)-2-benzyl-(3S)-3-hydroxy-butyryl]-4-(2-propyl)-oxazolidin-2-on (Beispiel 19)

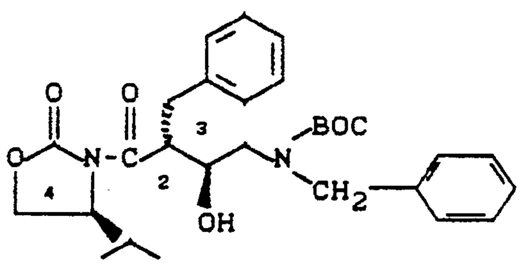

(19)

4S 2R, 3S

(4S)-3-[4-(N-tert.-butoxycarbonyl-N-benzyl)amino-(2S)-2-benzyl-(3S)-3-hydroxy-butyryl]-4-(2-propyl)-oxazolidin-2-on (Beispiel 20)

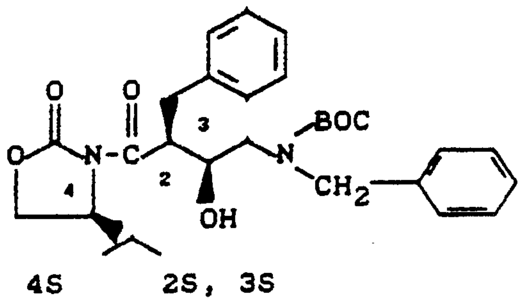

(20)

**Nebenkomponente**

(4S)-3-[4-(N-tert.-butoxycarbonyl-N-benzyl)amino-(2R)-2-benzyl-(3R)-3-hydroxy-butyryl]-4-(2-propyl)-oxazolidin-2-on (Beispiel 21)

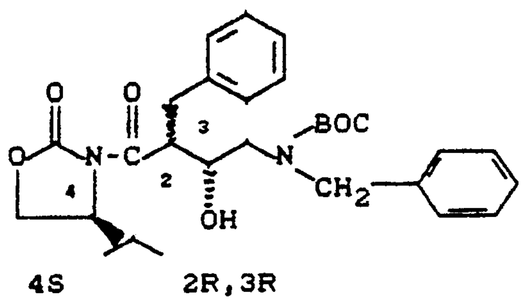

(21)

Die Titelverbindungen werden durch Isomerentrennung des Gemisches aus Beispiel 18 (analog der Beispiele 15, 16 und 17) rein erhalten.

Analytische Daten des Beispiels 19 (4S, 2R, 3S-Isomer)

HPLC-System VI: Rt = 12,53 min
$[\alpha]_D$ = + 89,42 (c = 1, Methanol)
(+)FAB-MS = m/z 511 (M + H); m/z = 533 (M + Na)

Analytische Daten des Beispiels 20 (4S, 2S, 3S-Isomer)

HPLC-System VI: Rt = 12,01 min
$[\alpha]_D$ = + 41,67 (c = 1, Methanol)

Analytische Daten des Beispiels 21 (4S, 2R, 3R-Isomer)

HPLC-System VI: Rt = 11,12 min
$[\alpha]_D$ = + 58,87 (c = 1, Methanol)

Alle weiteren Daten (HPLC-Systeme II, III, V, $^1$H-NMR in Dimethylsulfoxid, ((+)FAB-MS) entsprechen denen der spiegelbildisomeren Verbindungen aus den Beispielen 15, 16 und 17.

Beispiel 22

(4R)-3-[4-(N-tert.-Butoxycarbonyl-N-benzyl)amino-(2S)-2-benzyl-(3R,S)-3-hydroxybutyryl]-4-benzyl-oxazolidin-2-on

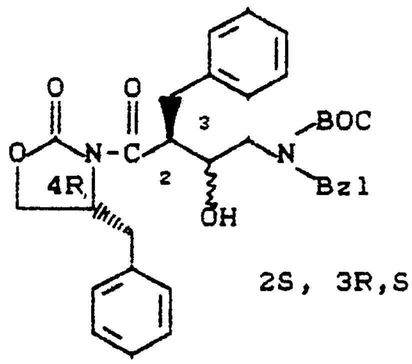

2S, 3R,S

Die Titelverbindung wird in Analogie zur Vorschrift des Beispiels 14 durch Umsetzung der Verbindung aus Beispiel X und der Verbindung aus Beispiel IV erhalten. Als Nebenprodukt tritt wieder das 4R,2R,3R-Isomer auf.

DC-System IX:
Rf = 0,90 4R, 2S, 3S-Isomer
Rf = 0,82 4R, 2S, 3S-Isomer
Rf = 0,66 4R, 2R, 3R-Isomer (Nebenprodukt)

HPLC-System II:
Rt = 33,56 min 4R, 2S, 3R-Isomer
Rt = 37,48 min 4R, 2R, 3R-Isomer (Nebenprodukt)
Rt = 43,29 min 4R, 2S, 3R-Isomer
(+)FAB-MS: m/z 529 (M + H); m/z 581 (M + Na)

Beispiele 23, 24 und 25

(4R)-3-[4-(N-tert.-butoxycarbonyl-N-benzyl)amino-(2S)-2-benzyl-(3R)-3-hydroxy-butyryl]-4-benzyl-oxazolidin-2-on (Beispiel 23)

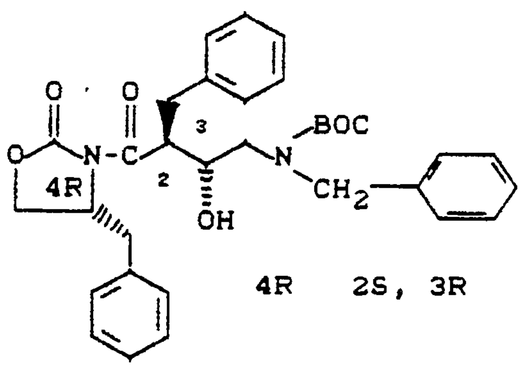

4R 2S, 3R

(23)

(4R)-3-[4-(N-tert.-butoxycarbonyl-N-benzyl)amino-(2R)-2-benzyl-(3R)-3-hydroxy-butyryl]-4-benzyl-oxazolidin-2-on (Beispiel 24)

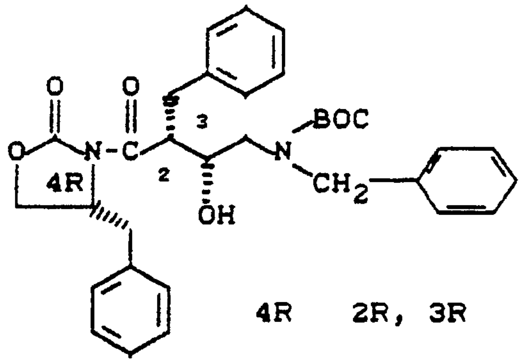

(24)

4R 2R, 3R

(4R)-3-[4-(N-tert.-butoxycarbonyl-N-benzyl)amino-(2S)-2-benzyl-(3S)-3-hydroxy-butyryl]-4-benzyl-oxazolidin-2-on (Beispiel 25)

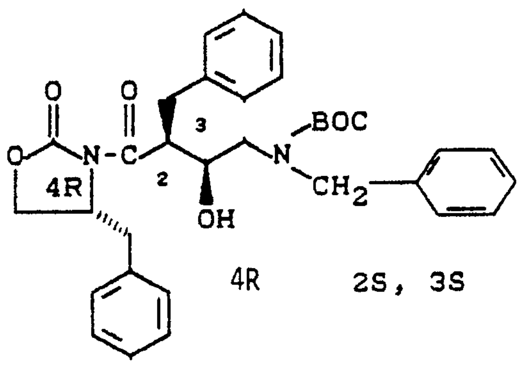

(25)

4R 2S, 3S

Die Titelverbindungen werden durch Isomerentrennung des Gemisches aus Beispiel 22 (analog der Beispiele 15, 16 und 17) rein erhalten.

Analytische Daten (Beispiele 23, 24, 25)

Die DC- und HPLC-Werte wurden bereits unter Beispiel 22 aufgeführt.
Beispiel 23: $[\alpha]_D$ = -110,84 (c = 1, Methanol)
Beispiel 25: $[\alpha]_D$ = - 53,26 (c = 1, Methanol)

Beispiel 26

(4R,5S)-3-[4-(N-tert.-Butoxycarbonyl-N-benzyl)-amino-(2S)-2-benzyl-(3R,S)-3-hydroxy-butyryl]-4-Methyl-5-phenyloxazolidin-2-on

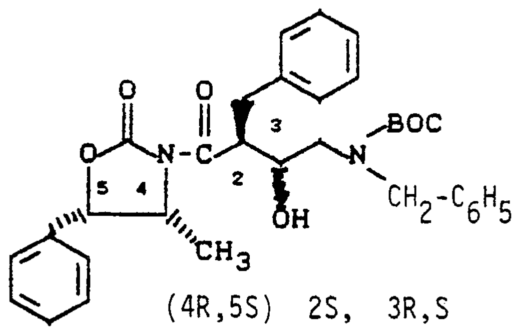

(4R,5S) 2S, 3R,S

Die Titelverbindung wird analog Beispiel 14, ausgehend von (4R,5S)-3-(3-Phenylpropionyl)-4-Methyl-5-phenyloxazolidin-2-on (Beispiel VIII) und dem Aldehyd aus Beispiel IV erhalten. Als Nebenprodukt tritt wieder das (4R,5S),2R,3R-Isomere auf.

DC-System IX: Rf = 0,83

DC-System XV: Rf = 0,20

HPLC-System II:

Rt = 46,94 min (4R,5S), 2S,3S-Isomer

Rt = 49,85 min (4R,2S), 2R,3R-Isomer (Nebenprodukt)

Rt = 56,74 min (4R,5S), 2S,3R-Isomer

(+)FAB-MS: m/z 559 (M + H); m/z 581 (M + Na)

Beispiele 27, 28 und 29

(4R,5S)-3-[4-(N-tert.-butoxycarbonyl-N-benzyl)amino-(2S)-2-benzyl-(3R)-3-hydroxy-butyryl]-4-methyl-5-phenyloxazolidin-2-on (Beispiel 27)

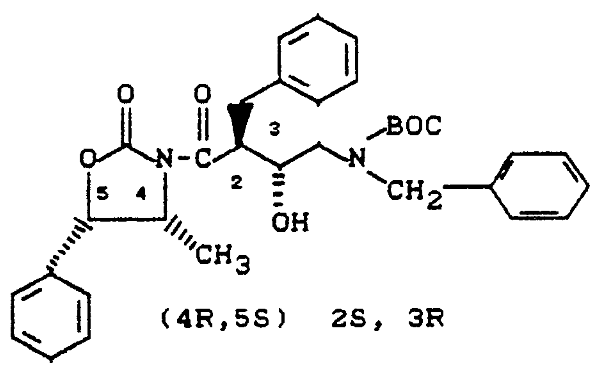

(27)

(4R,5S) 2S, 3R

(4R,5S)-3-[4-(N-tert.-butoxycarbonyl-N-benzyl)amino-(2R)-2-benzyl-(3R)-3-hydroxy-butyryl]-4-methyl-5-phenyloxazolidin-2-on (Beispiel 28)

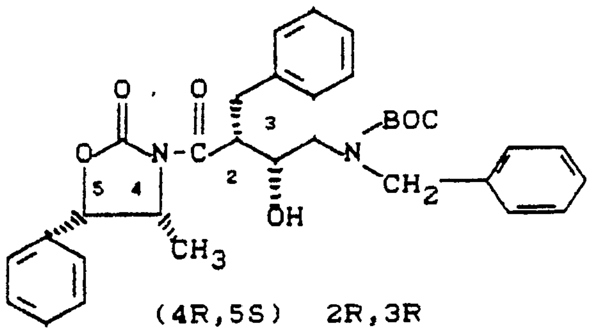

(28)

(4R,5S) 2R,3R

(4R,5S)-3-[4-(N-tert.-butoxycarbonyl-N-benzyl)amino-(2S)-2-benzyl-(3S)-3-hydroxy-butyryl]-4-methyl-5-phenyloxazolidin-2-on (Beispiel 28)

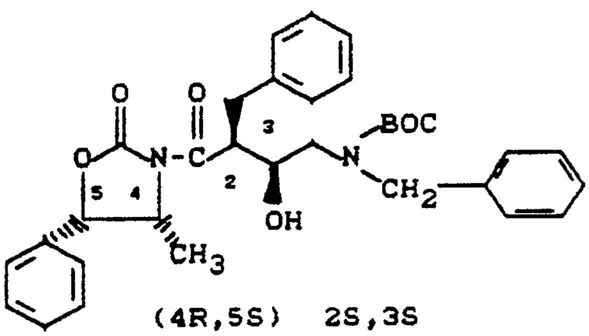

(29)

(4R,5S) 2S,3S

Die Titelverbindungen werden durch Isomerentrennung des Gemisches aus Beispiel (26 (analog Beispiel 15, 16 und 17) rein erhalten.

Analytische Daten des Beispiels 27

(4R, 5S, 2S, 3R-Isomer)
HPLC-System V: Rt = 25,59 min
(+)FAB-MS: m/z 559 (M + H); m/z 581 (M + Na)
$[\alpha]_D$ = -50,60 (c = 1, Methanol)

Analytische Daten des Beispiels 28

(4R, 5S, 2R, 3R-Isomer)
HPLC-System V: Rt = 23,11 min
(+)FAB-MS: m/z 559 (M + H); m/z 581 (M + Na)

Analytische Daten des Beispiels 29

(4R, 5S, 2S, 3R-Isomer)
HPLC-System V: Rt = 22,02 min
(+)FAB-MS: m/z 559 (M + H); m/z 581 (M + Na)

Beispiel 30

(4S)-3-[4-(N-tert.-butoxycarbonyl-N-benzyl)amino-(2R)-2-benzyl-(3R,S)-3-hydroxy-butyryl]-4-Phenyl-oxazolidin-2-on

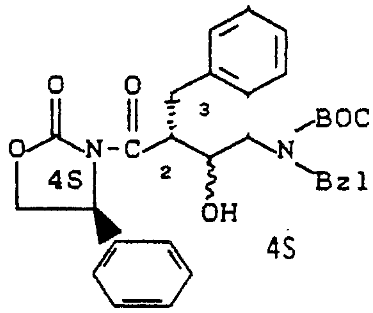

4S 2R,3R,S

Die Titelverbindung wird analog Beispiel 14, ausgehend von (4S)-3-(3-Phenylpropionyl)-4-Phenyloxazolidin-2-on (Beispiel IX) und dem Aldehyd aus Beispiel IV erhalten. Als Nebenprodukt tritt das 4S, 2S, 3S-Isomere auf.

DC-System IX: Rf = 0,77

DC-System XV: Rf = 0,21

HPLC-System II;

Rt = 25,59 min 4S, 2R, 3R-Isomer

Rt = 27,74 min 4S, 2S, 3S-Isomer

Rt = 31,61 min 4S, 2R, 3S-Isomer

(+)FAB-MS: m/z 545 (M + H); m/z 567 (M + Na)

Beispiele 31, 32 und 33

(4S)-3-[4-(N-tert.-butoxycarbonyl-N-benzyl)amino-(2R)-2-benzyl-(3S)-3-hydroxy-butyryl]-4-phenyl-oxazolidin-2-on (Beispiel 31)

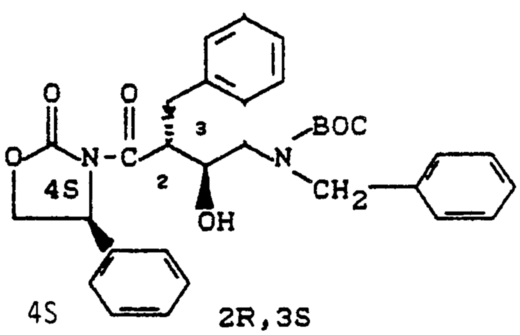

4S 2R,3S

(31)

(4S)-3-[4-(N-tert.-butoxycarbonyl-N-benzyl)amino-(2S)-2-benzyl-(3S)-3-hydroxy-butyryl]-4-phenyl-oxazolidin-2-on (Beispiel 32)

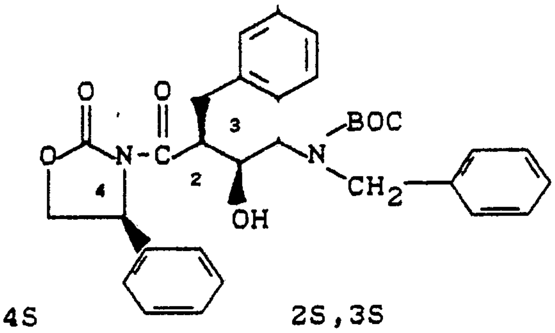

(32)

4S 2S,3S

(4S)-3-[4-(N-tert.-butoxycarbonyl-N-benzyl)amino-(2R)-2-benzyl-(3R)-3-hydroxy-butyryl]-4-phenyl-oxazolidin-2-on (Beispiel 33)

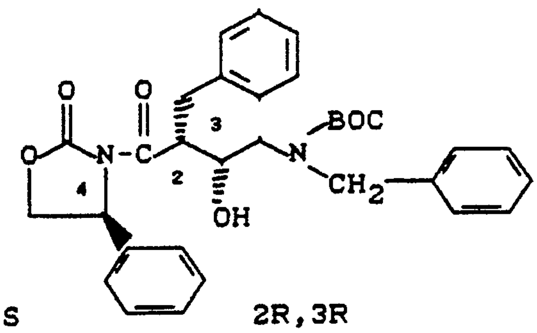

(33)

4S 2R,3R

Die Titelverbindungen wurden durch Isomerentrennung des Gemisches aus Beispiel 30 (analog der Beispiele 15, 16 und 17) rein erhalten.

Analytische Daten des Beispiels 31

(4S, 2R, 3S-Isomer)
HPLC-System II: Rt = 25,73 min
(+)FAB-MS: m/z 545 (M + H)

Analytische Daten des Beispiels 32

(4S, 2S, 3S-Isomer)
HPLC-System II: Rt = 28,74 min
(+)FAB-MS: m/z 545 (M + H)

Analytische Daten des Beispiels 33

(4S, 2R, 3R-Isomer)
HPLC-System II: Rt = 31,61 min
(+)FAB-MS: m/z 545 (M + H)

Beispiel 34

4-(N-tert.-butoxycarbonyl-N-benzyl)amino-(2S)-2-benzyl-(3R)-3-hydroxybuttersäure

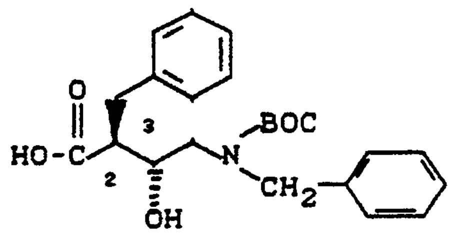

11,8 g (21,1 mol) (4R,5S)-3-[4-(N-tert.-Butoxycarbonyl-N-benzyl)amino-(2S)-2-benzyl-(3R)-3-hydroxybutyryl]-4-methyl-5-phenyloxazolidin-2-on (Beispiel 27) werden in 840 ml THF/$H_2O$ 3 : 1 gelöst. Dann gibt man 28,4 ml (278,5 mmol) $H_2O_2$ (30%ig, Aldrich) und 2,03 g (84,4 mmol) Lithiumhydroxid hinzu und rührt über Nacht bei 3 °C.

Der Ansatz wird mit 84 ml 1 N HCl versetzt und das THF am Rotationsverdampfer abgezogen. Die wäßrige Phase wird auf pH 3 gestellt und zweimal mit Essigester extrahiert. Die vereinigten organischen Phasen werden über Natriumsulfat getrocknet, filtriert und am Rotationsverdampfer eingeengt. Das Rohprodukt wird an 600 g Kieselgel 60 (Merck, Art.-Nr. 9385) 40 - 63 µm (230 - 400 mesh) mit einem Stufengradienten (wie in Beispiel 14 und 15 beschrieben) chromatographiert. Die produktenthaltenden Fraktionen werden vereinigt und eingeengt. Das Produkt wird aus Ether/n-Hexan kristallisiert, abgesaugt und getrocknet.

Die Titelverbindung kann analog durch Abspaltung des chiralen Hilfsreagenzes auch aus folgenden Beispielen enantiomerenrein erhalten werden: Beispiel 15, 23.

Ausbeute: 3,68 g (43,7 % der Theorie)
DC-System I: Rf = 0,49
HPLC-System IV: Rt = 32,57 min
(+)FAB-MS: m/z 438 (M + K); m/z 476 (M + 2K - H)
$[\alpha]_D$: - 21,75 (c = 1, MeOH)

## Beispiel 35

4-(N-tert.-butoxycarbonyl-N-benzyl)amino-(2R)-2-benzyl-(3S)-3-hydroxybuttersäure

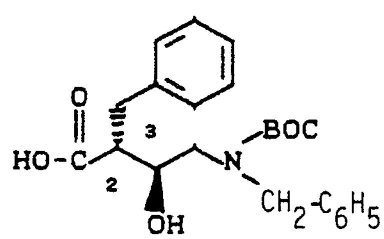

Die Titelverbindung wird durch Abspaltung des chiralen Hilfsreagenzes und nachfolgende Chromatographie (wie in Beispiel 34 beschrieben) aus folgenden Beispielen enantiomerenrein erhalten: Beispiel 19, 31.

## Analytische Daten

DC-System I: Rf = 0,49
HPLC-System IV: Rt = 24,13 min
(+)FAB-MS: m/z 438 (M + K); m/z 476 (M + 2K - H)
$[\alpha]_D$: + 20,36 (c = 1, MeOH)
$^1$H-NMR (250 MHz, DMSO) identisch mit den Daten des Beispiels 34.

Beispiel 36

4-(N-tert.-butoxycarbonyl-N-benzyl)amino-(2R)-2-benzyl-(3R)-3-hydroxybuttersäure

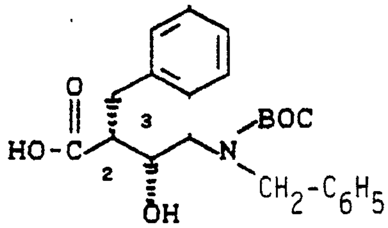

Die Titelverbindung wird durch Abspaltung des Hilfsreagenzes und nachfolgende Chromatographie (wie in Beispiel 34 beschrieben) aus folgenden Beispielen enantiomerenrein erhalten: Beispiel 16, 21, 24, 28, 33.

Analytische Daten

DC-System I: Rf = 0,58
HPLC-System IV: Rt = 15,10 min
(+)FAB-MS: m/z 438 (M + K); m/z 476 (M + 2K - H)
$[\alpha]_D$: - 17,58 (c = 1, MeOH)

Beispiel 37

4-(N-tert.-butoxycarbonyl-N-benzyl)amino-(2S)-2-benzyl-(3S)-3-hydroxybuttersäure

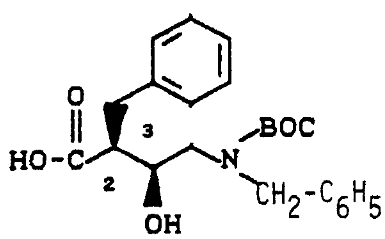

Die Titelverbindung wird durch Abspaltung des chiralen Hilfsreagenzes und nachfolgender Chromatographie (wie in Beispiel 34 beschrieben) aus folgenden Beispielen enantiomerenrein erhalten: Beispiel 17, 20, 25, 29, 32.

Analytische Daten

DC-System I: Rf = 0,58
HPLC-System IV: Rt = 12,30 min
(+)FAB-MS: m/z 438 (M + K); m/z 476 (M + 2K - H)
$[\alpha]_D$: + 17,96 (c = 1, MeOH)
$^1$H-NMR (250 MHz, DMSO): identisch mit Beispiel 36.

Beispiel 38

4-Amino-(2R,S)-benzyl-(3R,S)-hydroxybuttersäure

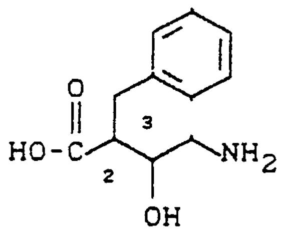

2R,S; 3R,S

5,11 g (15 mmol) 4-(N-Phthalyl)amino-2-R,S-benzyl-3R,S-hydroxybuttersäure (Beispiel 5) werden in 10 ml $MeOH/H_2O$ 1 : 1 gelöst und mit 913,86 µl (18,8 mmol) Hydrazinhydrat (Bayer, LLB Lev C 703, 01-825) versetzt. Die Mischung wird 3 Tage bei 5°C gehalten und erneut mit Hydrazinhydrat (182 µl, 3,75 mmol) versetzt. Nach einem Tag bei + 5°C wird der Ansatz mit 1 N HCl neutral gestellt und vorsichtig bis zur Halbtrockene einrotiert.

Der Rückstand wird in 1 N NaOH aufgenommen (bis pH 10), dreimal mit Ether und anschließend dreimal mit Essigester extrahiert. Die wäßrige Phase wird mit 1 N HCl auf pH 2 gestellt und vom Rückstand abfiltriert. Der Rückstand wird in 0,1 N HCl verrührt und abgesaugt. Die vereinigten wäßrig-sauren Filtrate werden zur Hälfte ihres Volumes eingeengt, eingefroren und lyophilisiert. Das Rohprodukt wird durch Adsorption durch Mitsubishi Diaion HP-20, Spülen mit Wasser und Elution mit einem Gradienten aus $MeOH/H_2O$ 10 : 90 - 50 : 50 entsalzt.

Die produktenthaltenden Filtrate werden vereinigt, vom MeOH abrotiert, eingefroren und lyophilisiert.
Ausbeute: 2,2 g (70,1 % der Theorie)
DC-System IV: Rf = 0,52
DC-System V: Rf = 0,73
DC-System XIV: Rf = 0,68
MS-DCI: m/z 210 (M + H), m/z 227 (M + $NH_4$)

Beispiel 39

4-(N-tert.-Butoxycarbonyl)-amino-(2R,S)-benzyl-(3R,S)-hydroxybuttersäure

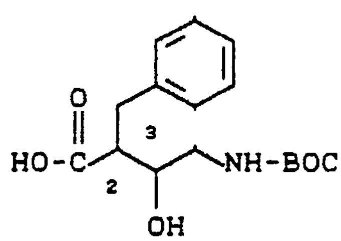

2,1 g (10 mmol) 4-Amino-2-R,S-benzyl-3-R,S-hydroxybuttersäure (Beispiel 38) werden in 40 ml Dioxan und 20 ml 1 N NaOH gelöst. Unter Eiskühlung gibt man 3,3 g (15 mmol) Di-tert.-butyldicarbonat (Fluka) zu der Mischung und rührt über Nacht.

Der Ansatz wird durch Zugabe von 1 N HCl neutral gestellt und das Dioxan am Rotationsverdampfer abgezogen. Die wäßrige Phase wird auf pH 9 gestellt und dreimal mit Ether gewaschen. Danach stellt man mit 1 N HCl auf pH 3 und extrahiert das Produkt in Essigester. Die vereinigten Essigesterphasen werden mit gesättigter NaCl-Lösung gewaschen, über Natriumsulfat getrocknet, filtriert und eingeengt.
Ausbeute: 2,6 g (84 % der Theorie)
DC-System I: Rf = 0,45
(+)FAB-MS: m/z 310 (M + H), m/z 322 (M + Na)

Beispiel 40

4-(N-Benzyl)amino-(2R) bzw. (2S)-benzyl-(3S) bzw. (3R)-3-hydroxybuttersäure-Hydrochlorid

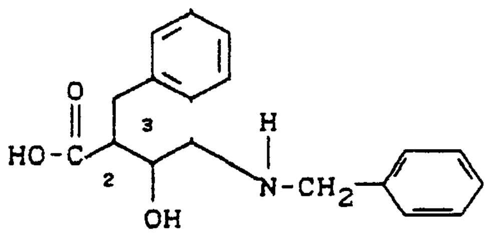

x HCl

2R, 3S und 2S, 3R

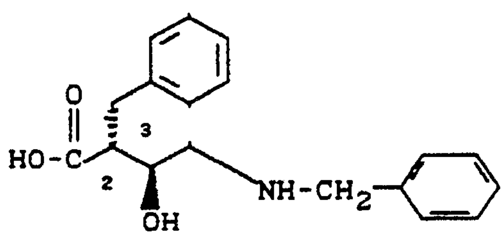

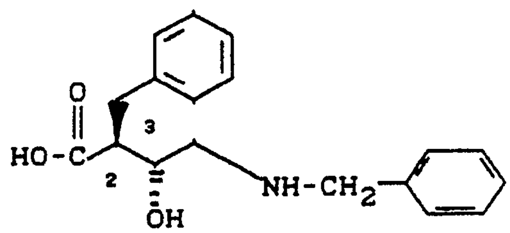

xHCl

400 mg (1 mmol) der Diastereomeren B aus Beispiel 11 werden zur Entfernung der N-tert.-Butoxycarbonylschutzgruppe in 10 ml 4 N HCl in Dioxan 1 Stunde bei Raumtemperatur gerührt. Der Ansatz wird zur Trockene eingeengt und mehrmals mit Ether koevaporiert. Der Rückstand wird über KOH getrocknet.
Ausbeute: 320 mg (96 % der Theorie)
DC-System III: Rf = 0,04
DC-System V: Rf = 0,51
DC-System XVI: Rf = 0,82
(+)FAB-MS: m/z 300 (M + H); m/z 322 (M + Na)

Beispiel 41

4-Amino-(2R) bzw. (2S)-benzyl-(3S) bzw. (3R)-3-hydroxybuttersäure

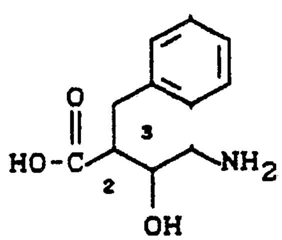

2R, 3S und 2S, 3R

280 mg (0,93 mmol) der N-Benzylverbindung aus Beispiel (40), die durch Neutralisation mit wäßrigem Ammoniak und mehrmalige Lyophilisierung in die freie Base übergeführt wird, werden durch 75-minütiges

Kochen in 15 ml Methanol, 1,1 g (18,6 mmol) Ammoniumformiat und 300 mg 10%iger Pd/C katalytisch N-debenzyliert. Das Gemisch wird vom Katalysator abfiltriert, im Wasser aufgenommen und mehrmals gefriergetrocknet.
Ausbeute; 49 mg (25 % der Theorie)
DC-System XVI: Rf = 0,67
(+)FAB-MS: m/z 210 (M + H), m/z 232 (M + Na)

Beispiel 42

4-(N-tert.-Butoxycarbonyl)-Amino-(2R) bzw. (2S)-benzyl-(3S)- bzw. (3R)-3-hydroxybuttersäure

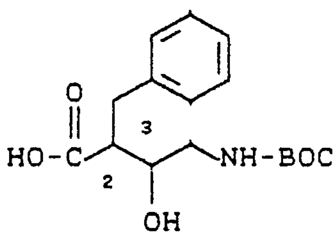

2R, 3S und 2S, 3R

Die Titelverbindung wird wie in Beispiel 39 beschrieben durch Umsetzen von 44 mg (0,22 mmol) des Beispieles 41 mit 96 mg Di-tert.-Butyldicarbonat erhalten. Das Rchprodukt wird an Kieselgel mit dem Laufmittelsystem $CH_2Cl_2$/MeOH 95 : 5 chromatographiert.
Ausbeute: 11,1 mg (17 % der Theorie)
DC-System IV: Rf = 0,49
MS-DCI: m/z 310 (M + H)

Beispiel 43

4-(N-Isopropyl)amino-(2R) bzw. (2S)-2-benzyl-(3S) bzw. (3R)-3-hydroxybuttersäure

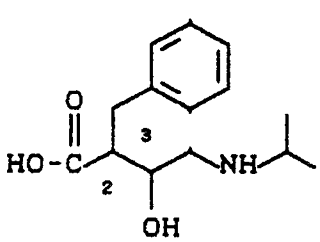

2R, 3S und 2S, 3R

400 mg (2 mmol) der Diastereomeren B aus Beispiel 11 werden wie in Beispiel 40 beschrieben mit 4 N HCl in Dioxan zur Entfernung der BOC-Schutzgruppe gerührt. Der Ansatz wird eingeengt, mit 150 ml Wasser verdünnt, mit 25%igem Ammoniak auf pH 8 gestellt und gefriergetrocknet. Der voluminös-amorphe Rückstand der intermediären N-Benzylverbindung (Beispiel 41) wird mit Aceton koevaporiert und an der Ölpumpe getrocknet. Der Ansatz wird in 30 ml Methanol gelöst und nach Zugabe von 400 mg 20-%igem $Pd(OH)_2$ als Katalysator bei 3,5 bar hydriert. Der Katalysator wird abfiltriert und das organische Lösungsmittel abrotiert. Der Rückstand wird in 1 ml Acetonitril gelöst. Nach Zugabe von 50 ml $H_2O$ wird der Ansatz

eingefroren und gefriergetrocknet.
Ausbeute: 220 mg (87 % der Theorie)
DC-System XVI: Rf = 0,67

Beispiel 44

4-(N-tert.-Butoxycarbonyl-N-isopropyl)amino-(2R) bzw. (2S)-2-benzyl-(3S) bzw. (3R)3-hydroxybuttersäure

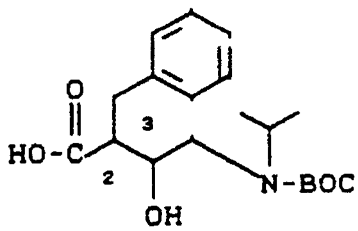

2R, 3S und 2S, 3R

220 mg (0,87 mmol) der Verbindung aus Beispiel 43 werden wie in Beispiel 39 beschrieben mit 480 mg (2,2 mmol) Ditert.-Butyldicarbonat umgesetzt und analog aufgearbeitet. Die saure organische Phase wird über Natriumsulfat getrocknet, abfiltriert und zur Trockene eingeengt.
Ausbeute: 131,3 mg (50,5 % der Theorie)
DC-System IV: Rf = 0,49
MS-DCI: m/z 352 (M + H)

Beispiel 45

4-(N-tert.-Butoxycarbonyl)amino-2R,S-cyclohexylmethyl-3R,S-hydroxybuttersäure

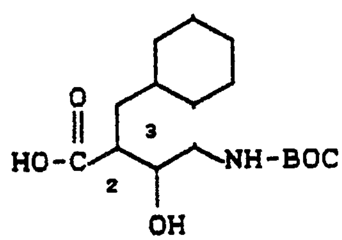

2R,S 3R,S

770 mg (2,5 mmol) 4-(N-tert.-Butoxycarbonyl)amino-2R,S-benzyl-3R,S-hydroxybuttersäure (Beispiel 39) werden in 20 ml MeOH gelöst. Nach Zugabe von 0,5 ml Eisessig und 1 g fünfprozentige Rh/C * wird der Ansatz 5 Stunden bei 100 bar Wasserstoffdruck bei Raumtemperatur hydriert. Der Katalysator wird abfiltriert und das Filtrat zur Trockene eingeengt. Der ölige Rückstand wird mit 10 ml Wasser versetzt und das Produkt mit 3 x 10 ml Essigester aus der sauren wäßrigen Phase extrahiert. Die vereinigte organische Phase wird über Natriumsulfat getrocknet, filtriert und eingeengt. Der Rückstand wird in Acetonitril/$H_2O$ aufgenommen und gefriergetrocknet.

* Rhodium auf Kohle-Katalysator

Ausbeute: 600 mg (76 % der Theorie)
DC-System IV: Rf = 0,60
(+)FAB-MS: m/z 338 (M + Na)

Die in den folgenden Tabellen aufgeführten Beispiele sind durch $^1$H-NMR-Werte und durch Massenspektroskopie charakterisiert.

Beispiele 46 bis 49

Die in der Tabelle 1 aufgeführten Verbindungen wurden aus den entsprechenden enantiomeren Vorstufen der Beispiele 34 - 37 durch Abspaltung der BOC-Schutzgruppe in Analogie zu Beispiel 40 hergestellte.

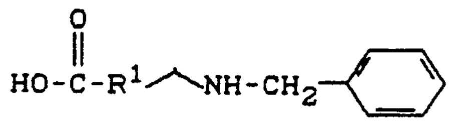

Tabelle 1

| Beispiel-Nr. | $R^1$ | Konfiguration | $[\alpha]_D$ (c=1 MeOH) Rohprodukte |
|---|---|---|---|
| 46 | 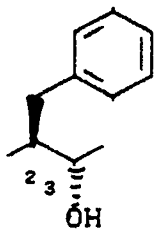 | 2S, 3R | - 13,88 |
| 47 | 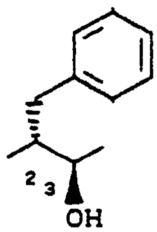 | 2R, 3S | + 9,97 |
| 48 | 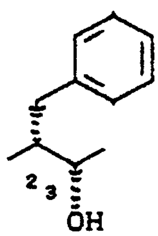 | 2R, 3R | + 13,95 |
| 49 | 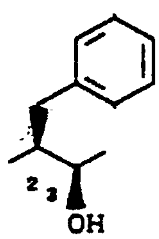 | 2S, 3S | - 12,75 |

Beispiele 50 bis 53

Die in der Tabelle 2 aufgeführten Verbindungen wurden aus den entsprechenden enantiomeren Vorstufen der Beispiele 46 - 49 durch Abspaltung der N-Benzyl-Schutzgruppe in Analogie zu Beispiele 41 hergestellt.

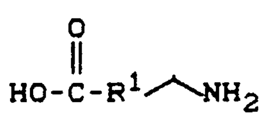

Tabelle 2

| Beispiel-Nr. | $R^1$ | Konfiguration | $[\alpha]_D$ (c = 1) (Rohprodukte) |
|---|---|---|---|
| 50 | 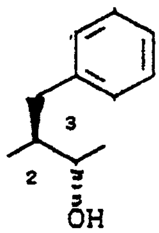 | 2S, 3R | - 19,92 (MeOH) |
| 51 | 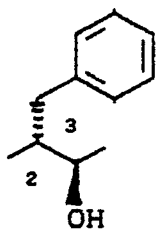 | 2R, 3S | + 15,05 |
| 52 | 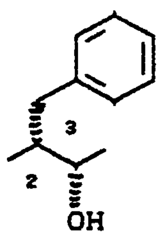 | 2R, 3R | + 36,06 |
| 53 | 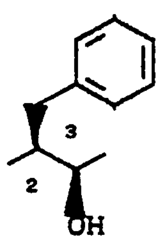 | 2S, 3S | - 37,41 |

Beispiele 54 bis 57

Die in der Tabelle 3 aufgeführten Verbindungen wurden aus den entsprechenden enantiomeren Vorstufen der Beispiele 50 - 53 durch Umsetzung mit Di-tert.butyldicarbonat in Analogie zu Beispiel 39 hergestellt.

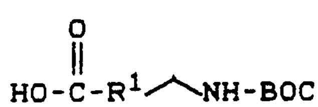

Tabelle 3

| Beispiel-Nr. | $R^1$ | Konfiguration | $[\alpha]_D$ (c = 1) (Rohprodukte) |
|---|---|---|---|
| 54 | 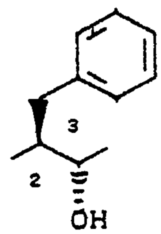 | 2S, 3R | - 7,47 |
| 55 | 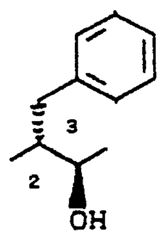 | 2R, 3S | + 14,26 |
| 56 | 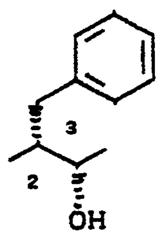 | 2R, 3R | + 10,75 |
| 57 | 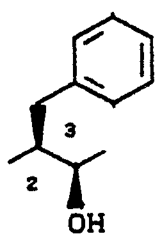 | 2S, 3S | - 11,62 |

Beispiele 58 bis 61

Die in der Tabelle 4 aufgeführten Verbindungen wurden aus den entsprechenden enantiomeren Vorstufen der Beispiele 54 - 57 durch katalytische Hochdruckhydrierung in Analogie zu Beispiel 45 oder durch Umsetzen der entsprechenden enantiomeren Vorstufen der Beispiele 62 bis 65 mit Di-tert.butyldicarbonat in Analogie zu Beispiel 54 - 57 hergestellt.

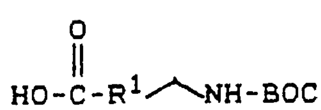

<u>Tabelle 4</u>

| Beispiel-Nr. | $R^1$ | Konfiguration |
|---|---|---|
| 58 | 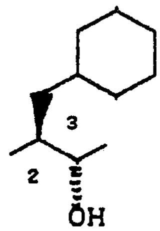 | 2S, 3R |
| 59 | 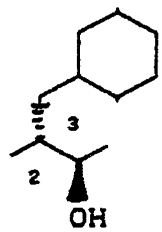 | 2R, 3S |

HO-

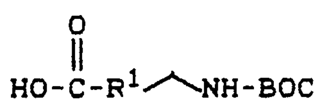

<u>Tabelle 4</u> (Fortsetzung)

| Beispiel-Nr. | $R^1$ | Konfiguration |
|---|---|---|
| 60 | 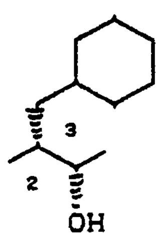 | 2R, 3R |
| 61 | 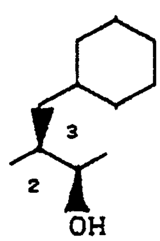 | 2S, 3S |

<u>Beispiele 62 bis 65</u>

Die in der Tabelle 5 aufgeführten Verbindungen wurden aus den entsprechenden enantiomerenreinen Vorstufen der Beispiele 50 - 53 durch katalytische Hochdruckhydrierung in Analogie zu Beispiel 45 hergestellt.

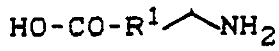

**Tabelle 4**

| Beispiel-Nr. | $R^1$ | Konfiguration |
|---|---|---|
| 62 | 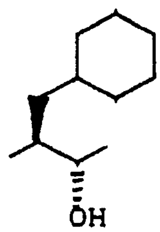 | 2S, 3R |
| 63 | 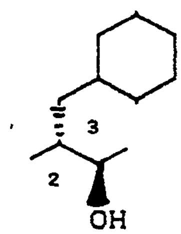 | 2R, 3S |
| 64 | 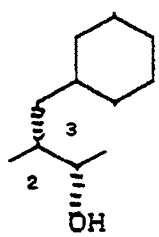 | 2R, 3R |
| 65 | 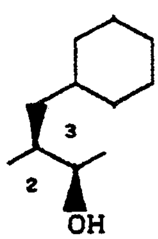 | 2S, 3S |

Beispiel 66

N-α-tert.-Butoxycarbonyl-Nim-tert.-Butoxycarbonyl-D-Histidyl-D-Phenylalaninbenzylester

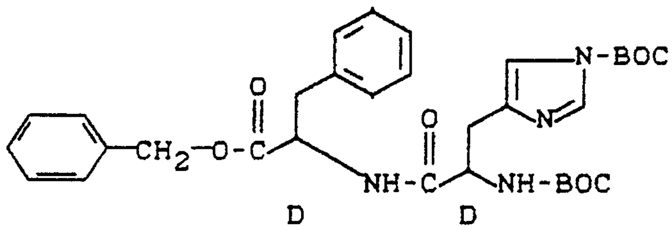

25 g (0,054 mol) D-Phenylalaninbenzylester-Tosylat (Senn Chemicals) und 23,3 g (0,065 g) N-α-tert.-Butoxycarbonyl-N-im-tert.-Butoxycarbonyl-D-Histidin (Bissendorf Biochemicals) werden in 200 ml $CH_2Cl_2$ und 100 ml DMF gelöst. Zu der Mischung gibt man unter Rühren 11,1 g (0,081 mol) 1-Hydroxybenzotriazol, 9,2 ml (0,081 mol) N-Ethylmorpholin und dann 14,6 g (0,070 mol) Dicyclohexylcarbodiimid.

Der Ansatz wird über Nacht bei Raumtemperatur gerührt, am Hochvakuum einrotiert und in 100 ml Essigester aufgenommen. Die Mischung wird kurz aufgerührt und 2 Stunden im Kühlschrank bei 5 °C aufbewahrt. Der ausgefallene Dicyclohexylharnstoff wird abgesaugt und das Filtrat mit gesattigter Natriumbicarbonatlösung, Salzsäure pH 3 und ges. Kochsalzlösung ausgeschüttelt, über Natriumsulfat getrocknet und zur Trockene eingeengt. Das Rohgemisch (45,5 g) wird an Kieselgel mit einem Gradienten aus $CH_2Cl_2$ und dann $CH_2Cl_2$/MeOH 99 : 1 chromatographiert.

Die sauberen produktenthaltenden Fraktionen werden vereinigt und eingeengt.
Ausbeute: 20,4 g (64 % der Theorie)
DC-System I: Rf = 0,79
HPLC-System I: Rt = 25,76 min
FAB-MS: m/z 593 (M + H); m/z 615 (M + Na)

Beispiel 67

D-Histidyl-D-Phenylalaninbenzylester-Dihydrochlorid

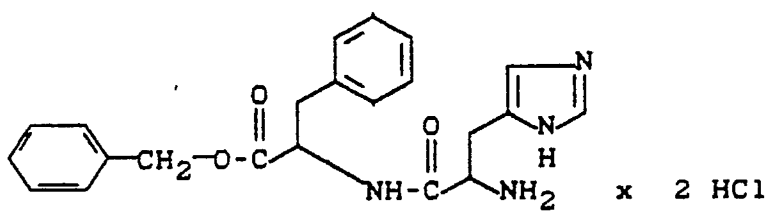

10,7 g (18 mmol) N-2-tert.Butoxycarbonyl-N-im-tert.-Butoxycarbonyl-D-Histidyl-D-phenylalanin-benzylester (Beispiel 66) werden über Nacht in 100 ml 4 N HCl (gasförmig) in Dioxan gerührt. Der Ansatz wird fast zur Trockene eingeengt, mit Ether koevaporiert, mit Ether verrührt, abgesaugt und über KOH getrocknet.
Ausbeute: 7,13 g (85 % der Theorie)
DC-System III: Rf = 0,21
(+)FAB-MS: m/z 393 (M + H)

Beispiel 68

D-Histidyl-D-Phenylalaninmethylester-Dihydrochlorid

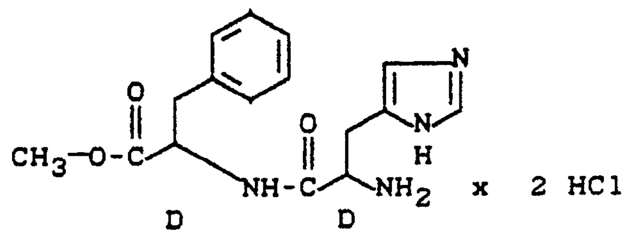

Die Titelverbindung ist analog Beispiel 66 und 67 durch Kopplung von D-Phenylalaninmethylester und Di-BOC-D-His und nachfolgender Abspaltung der BOC-Schutzgruppe erhältlich.
DC-System III: Rf = 0,27
(+)FAB-MS: m/z 317 (M + H)

Beispiel 69

D-Histidyl-D-Phenylalanin-tert.-butylester-Dihydrochlorid

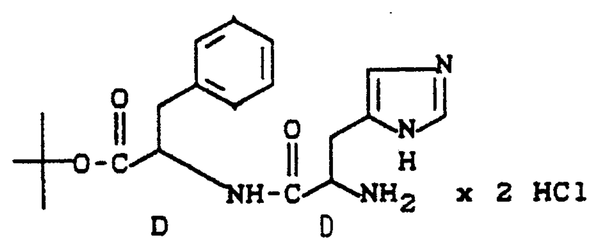

5,65 g (10 mmol) N-α-tert.-Butoxycarbonyl-N-im-tert.-Butoxycarbonyl-D-Histidyl-D-Phenylalanin-tert.-butylester (erhältlich aus Di-BOC-D-His und D-Phenyl-alaninetert.-butylester analog Beispiel 66) werden in 50 ml $CH_2Cl_2$ gelöst, im Eisbad gekühlt und mit 7,8 ml (101 mmol) Trifluoressigsäure versetzt. Es wird 45 min gerührt und nochmals die gleiche Menge an Trifluoressigsäure hinzugegeben. Nach weiteren 45 Minuten im Eisbad wird der Ansatz zur Trockene eingeengt und in $CH_2Cl_2$ aufgenommen. Die organische Phase wird mit ges. $NaHCO_3$ ausgeschüttelt, mit ges. Kochsalzlösung neutral gewaschen, über $Na_2SO_4$ getrocknet, mit 5 ml 4 N HCl in Dioxan versetzt und zur Trockene eingeengt.
Ausbeute: 2,36 g (54 % der Theorie)
DC-System III: Rf = 0,38
DC-System IV: Rf = 0,17
(+)FAB-MS: m/z 359 (M + H)

Beispiel 70

4-(N-Phthalyl)amino-2-R,S-benzyl-3R,S-hydroxybutyryl-D-hystidyl-D-phenylalanin-Benzylester

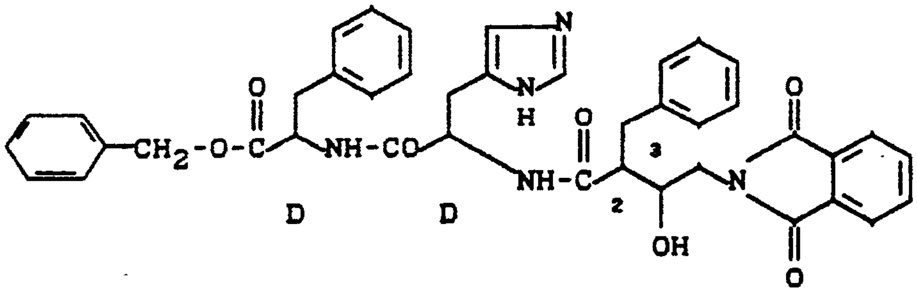

1,35 g (4 mmol) der Verbindung aus Beispiel 5 und 1,91 g (5 mmol) der Verbindung aus Beispiel 67 werden in 40 ml $CH_2Cl_2$ gelöst und nacheinander mit 2,77 ml (16 mmol) DIPEA, 703 mg (5,2 mmol) HOBT und 990 mg (4,8 mmol) DCC versetzt. Der Ansatz wird über Nacht gerührt und noch einmal mit 200 mg (1 mmol) DCC versetzt. Nach 2 Stunden gibt man 150 mg Glycin hinzu, engt am Rotationsverdampfer ein und versetzt mit Essigsäureethylester. Der ausgefallene Harnstoff wird abfiltriert und das Filtrat mit ges. $NaHCO_3$-Lösung, Puffer pH 7 (Merck, Art.-Nr. 9385) und ges. NaCl-Lösung gewaschen, über $Na_2SO_4$ getrocknet und eingeengt. Das Rohprodukt wird an Kieselgel mit einem Gradienten aus $CH_2Cl_2$/MeOH von 100 : 0 bis 95 : 5 chromatographiert.

Ausbeute: 2,14 g (75 % der Theorie)
DC-System I: Rf = 0,51 (Mitte des Fleckes)
(+)FAB-MS: m/z 714 (M + H)

Beispiel 71

4-(N-Phthalyl)amino-2R,S-benzyl-3R,S-hydroxybutyryl-D-hystidyl-D-phenylalanin

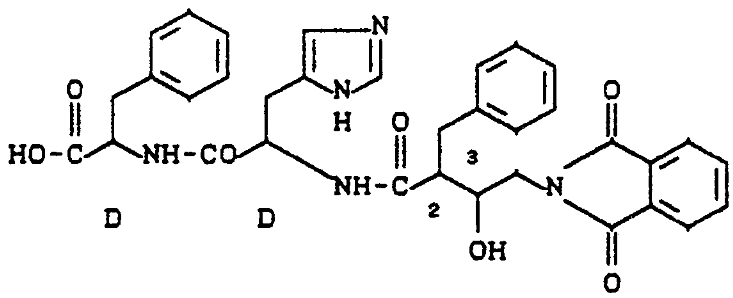

2,1 9 (2,9 mmol) der Verbindung aus Beispiel 70 werden in 40 ml Dioxan gelöst, mit 500 mg Pd/C (10 %) versetzt und nach Zugabe von 1 ml 1 N HCl 24 Stunden bei 3 bar hydriert. Der Katalysator wird abfiltriert, das Filtrat eingeengt und mehrmals mit Ether bis zur Trockene koevaporiert. Der Rückstand wird mit Diethylether verrührt, abgesaugt und getrocknet.

Ausbeute: 1,59 g (64 % der Theorie)
DC-System I: Rf = 0,21 - 0,30
DC-System III: Rf = 0,06
(+)FAB-MS: m/z 624 (M + H); m/z 630 (M + Li);
m/z 636 (M + 2Li-H)

Beispiel 72

4-(N-Phthalyl )amino2-R,S-benzyl-3R,S-hydroxybutyryl-D-hystidyl-D-phenylalanin(cyclopentyl)amid

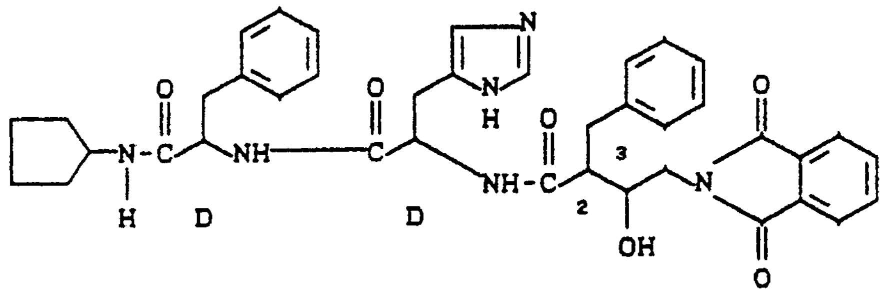

600 mg (0,96 mmol) der Verbindung aus Beispiel 71 werden in 10 ml $CH_2Cl_2$ gelöst und nacheinander mit 0,2 ml Cyclopentylamin, 150 mg HOBT und 280 mg DCC versetzt. Der Ansatz wird über Nacht gerührt, vom ausgefallenen Dicyclohexylharnstoff abfiltriert und analog Beispiel 70 ausgeschüttelt und das Rohprodukt an Kieselgel chromatographiert.
Ausbeute: 250 mg (38 % der Theorie)
DC-System I: Rf = 0,32 - 0,37
(+)FAB-MS: m/z 691 (M + H)

Beispiel 73

4-(N-tert.-Butoxycarbonyl-N-Benzyl)amino-2-R,S-benzyl-3-R,S-hydroxybutyryl-D-Histidin-methylester

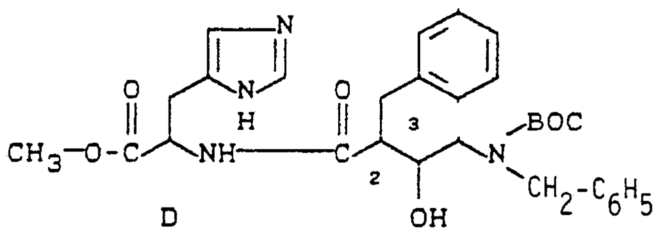

Die Titelverbindung wird durch DCC/HOBT-Kupplung (analog Beispiel 70 und 72) von 1,31 g (4,25 mmol) der Verbindung aus Beispiel 9 bzw. eines Gemisches der Verbindungen aus Beispiel 10 und 11 bzw. eines Gemisches der Beispiele 34 - 38 mit D-Histidinmethylester Dihydrochlorid, Aufarbeitung und Gradientenchromatographie an Kieselgel (analog Beispiel 82 und 84) erhalten.
Ausbeute: 780 mg (31,4 % der Theorie)
DC-System I: Rf = 0,35 - 0,44
(+)FAB-MS: m/z 551 (M + H)

Beispiel 74

4-(N-tert.-Butoxycarbonyl-N-Benzyl)amino-2-R,S-benzyl-3-R,S-hydroxybutyryl-D-Histidin

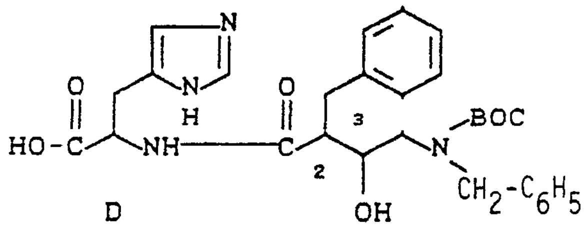

760 mg (1,4 mmol) der Verbindung aus Beispiel 73 werden in 20 ml Dioxan gelöst, mit 1,8 ml 1 N NaOH versetzt und über Nacht bei Raumtemperatur gerührt. Der Ansatz wird mit 10 ml $H_2O$ und 2 ml 1 N NaOH versetzt und 2 Stunden bei Raumtemperatur gerührt. Die Mischung wird neutral gestellt (1 N HCl), vom Dioxan abrotiert und lyophylisiert. Das Rohprodukt wird an Mitsubishi Diaion HP 20 mit einem Gradienten aus $H_2O$/MeOH 100 : 0 bis 30 : 70 entsalzt und eluiert. Die produktenthaltenden Fraktionen werden vereinigt, vom Methanol abrotiert und gefriergetrocknet.
Ausbeute: 590 mg (78 % der Theorie)
DC-System III: Rf = 0,06
(+)FAB-MS: m/z 537 (M + H); m/z 559 (M + Na);
m/z 581 (M + 2Na-H)

Beispiel 75

4-(N-tert.-Butoxycarbonyl-N-Benzyl)amino-2-R,S-benzyl-3-R,S-hydroxybutyryl-Histidyl-D-Phenylalanin-methylester

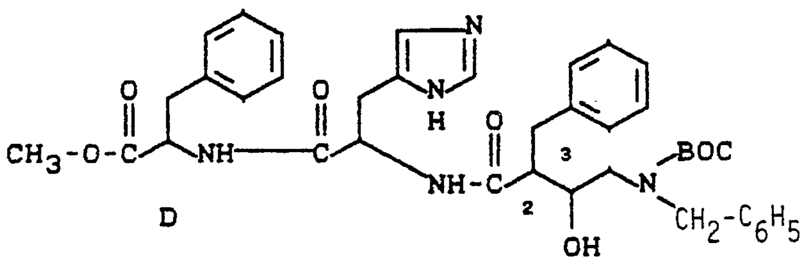

580 mg (1,1 mmol) der Verbindung aus Beispiel 74 und 403 mg (2 mmol) D-Phenylalaninmethylester Hydrochlorid werden in 10 ml $CH_2Cl_2$ gelöst und nacheinander mit 697 µl (4 mmol) DIPEA, 270 mg (2 mmol) HOBT und 412 mg (2 mmol) DCC versetzt. Der Ansatz wird über Nacht gerührt und nochmals mit 200 mg (1 mmol) D-Phenylalaninmethylester Hydrochlorid, 350 µl (2 mmol) DIPEA und 600 mg (3 mmol) DCC versetzt. Nach sechsstündigem Rühren bei Raumtemperatur gibt man 500 mg Glycin zu der Mischung und rührt über Nacht. Der Ansatz wird mit 50 ml $CH_2Cl_2$ aufgefüllt, vom Ungelösten abfiltriert und eingeengt. Der Rückstand wird in Essigester aufgenommen und mit gesättigter $NaHCO_3$, Puffer pH 7 und gesättigter NaCl-Lösung gewaschen. Die organische Phase wird über $Na_2SO_4$ getrocknet, eingeengt und nochmals mit $CH_2Cl_2$ koevaporiert.

Das Rohprodukt wird an Kieselgel mit einem Gradienten aus $CH_2Cl_2$/MeOH (analog Beispiel 70 und 72) chromatographiert.

Die Pauli-positiven (DC-Spritzreagenz auf Histidin) Fraktionen werden gepoolt und nach Einengen auf demselbem System rechromatographiert. Die produktenthaltenden Eluate werden vereinigt, eingeengt und am Hochvakuum getrocknet.
Ausbeute: 510 mg (67 % der Theorie)
DC-System I: Rf = 0,81 - 0,90
DC-System II: Rf = 0,27 - 0,37
(+)FAB-MS: m/z 698 (M + H)

Beispiel 76

4-(N-tert.-Butoxycarbonyl-N-Benzyl)amino-2-R,S-benzyl-3-R,S-hydroxybutyryl-D-Histidyl-D-Phenylalanin

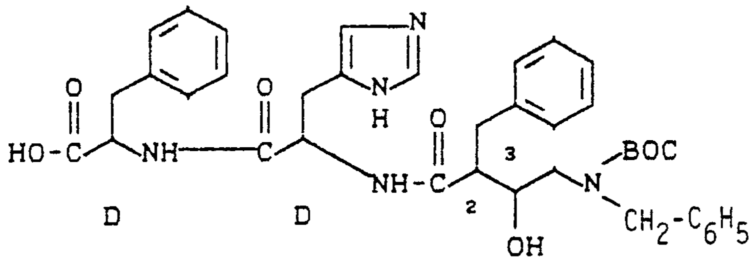

Die Titelverbindung wird aus 247 mg (0,354 mmol) der Verbindung aus Beispiel 75 durch basische Verseifung (analog Beispiel 74) und Entsalzen des Rohproduktes erhalten.
Ausbeute: 137 mg (57 % der Theorie)
DC-System III: Rf = 0,03
(+)FAB-MS: m/z 684 (M + H); m/z 706 (M + Na)

Beispiel 77

4-(N-tert.-Butoxycarbonyl-N-Benzyl)amino-2-R,S-benzyl-3-R,S-hydroxybutyryl-D-Histidyl-D-Phenylalanin (N-Morpholino)amid

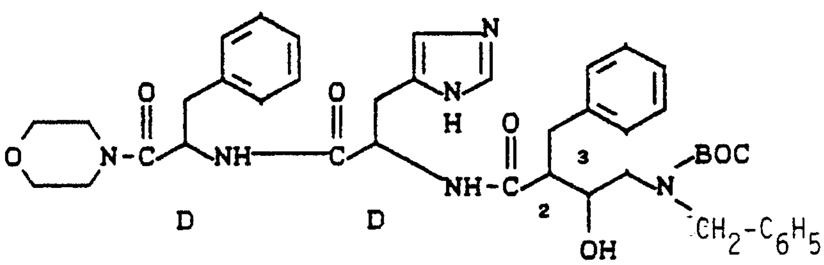

Die Titelverbindung wird ausgehend von 90 mg (146 µmol) der Verbindung aus Beispiel 76 durch Kopplung mit Morpholin (analog Beispiel 72) und Chromatographie des Rohproduktes erhalten.
Ausbeute: 77 mg (74 % der Theorie)
DC-System III: Rf = 0,26
(+)FAB-MS: m/z 753 (M + H); m/z 775 (M + Na)

Beispiel 78

(N-tert.-Butoxycarbonyl)amino-2R,S-benzyl-3-R,S-hydroxybutyryl-D-Histidyl-D-Phenylalanin-(2-methylpropyl)amid

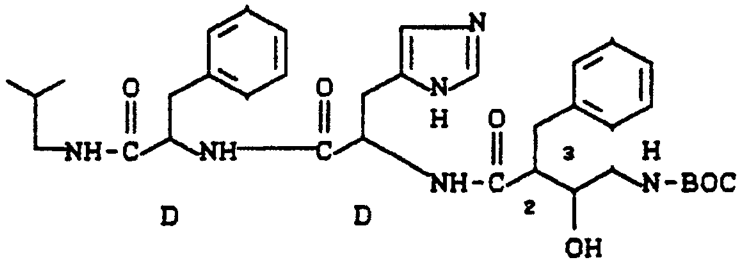

Die Titelverbindung wird ausgehend von der Verbindung aus Beispiel 39 bzw. eines Gemisches der Verbindungen aus den Beispielen 54 - 57 durch DCC/HOBT-Kopplung (analog Beispiel 70 und 72) bzw. n-PPA-Kupplung (analog Beispiel 91) mit D-Histidyl-D-Phenylalanin-(2-Methylpropyl)amid Dihydrochlorid (erhältlich durch katalytische Debenzylierung der Verbindung aus Beispiel 66, Kopplung des Produktes mit Isobutylamin und Umsetzen des Intermediates mit 4 N HCl/Dioxan) erhalten.

Alternativ kann die Verbindung aus Beispiel 39 bzw. das Gemisch der Verbindungen aus den Beispielen 54 - 57 zuerst mit D-Histinmethylester Dihydrochlorid umgesetzt werden (analog Beispiel 73). Der erhaltene Ester kann sodann verseift (analog Beispiel 74) und sodann mit D-Phenylalanin-(2-Methyl)propylamid Hydrochlorid (analog Beispiel 75) umgesetzt werden. Alternativ kann die Titelverbindung durch Umsetzen der Verbindung aus Beispiel 39 bzw. eines Gemisches der Verbindungen aus den Beispielen 54 - 57 in der bereits bei dem Beispiel 70 beschriebenen Weise mit der Verbindung aus dem Beispiel 67, katalytischer Debenzylierung des Intermediates (analog Beispiel 71) und nachfolgender Umsetzung der erhaltenen Säure mit Isobutylamin (analog Beispiel 72) erhalten werden. Die erhaltenen Rohprodukte werden wie bereits mehrfach beschrieben ausgeschüttelt und mit $CH_2Cl_2$/MeOH-Gradienten an Kieselgel chromatographiert.

Analytische Daten des Beispieles 78

DC-Sytem I: Rf = 0,28 - 0,37
(+)FAB-MS: m/z 649 (M + H)

Beispiele 79 - 86 und Beispiele 87 - 89

Die in Tabelle 6 aufgeführten Beispiele werden nach den in den Beispielen 66 - 78 aufgeführten Methoden hergestellt.

Die in Tabelle 7 aufgeführten Beispiel 87 - 89 werden ebenfalls nach den oben beschriebenen Methoden hergestellt. Als Retrostatin-Kopplungskomponenten wurden die Verbindung aus Beispiel 45 bzw. das Gemisch der Verbindungen aus den Beispielen 58 - 61 eingesetzt.

**Tabelle 6**

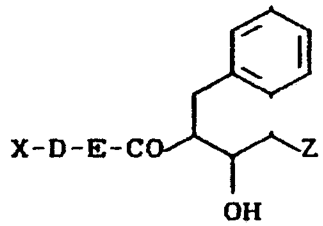

| Beisp. Nr. | X | D | E | Z | DC-System Rf-Werte | (+)FAB-MS |
|---|---|---|---|---|---|---|
| 79 | $CH_3O$ | - | D-His | 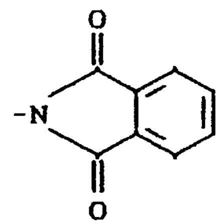 | III: 0,50 - 0,54<br>IV: 0,13 - 0,20 | m/z 491 (M + H) |
| 80 | $CH_3O$ | D-Phe | D-His | 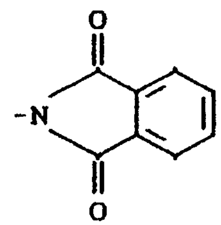 | I: 0,37 - 0,50 | m/z 638 (M + H) |
| 81 | $CH_3O$ | L-Phe | L-His | 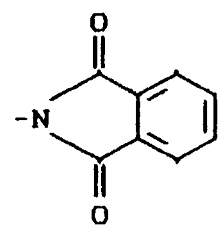 | IV: 0,33 - 0,50 | m/z 638 (M + H) |

**Tabelle 6**

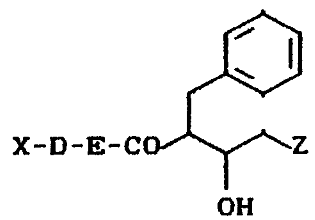

| Beisp. Nr. | X | D | E | Z | DC-System Rf-Werte | (+)FAB-MS |
|---|---|---|---|---|---|---|
| 82 | 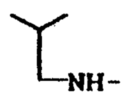 | D-Phe | D-His | 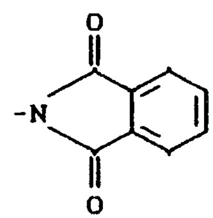 | I: 0,43 - 0,49 | m/z 679 (M + H) |
| 83 | 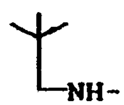 | D-Phe | D-His | 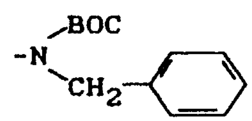 | III: 0,25 | m/z 753 (M + H)<br>m/z 775 (M + Na) |
| 84 | 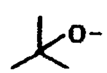 | D-Phe | D-His | NH-BOC | III: 0,28 - 0,40<br>IV: 0,38 - 0,50 | m/z 650 (M + H)<br>m/z 672 (M + Na) |
| 85 | 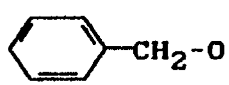 | D-Phe | D-His | NH-BOC | III: 0,34 - 0,37 | m/z 684 (M + H)<br>m/z 706 (M + Na) |
| 86 | OH | D-Phe | D-His | NH-BOC | III: 0,03 - 0,06 | m/z 759 (M + H) |

Tabelle 7

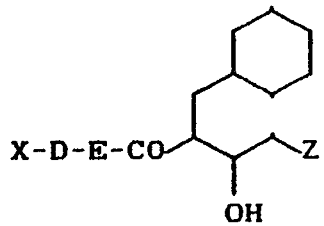

| Beisp. Nr. | X | D | E | Z | DC-System Rf-Werte | (+)FAB-MS |
|---|---|---|---|---|---|---|
| 87 | 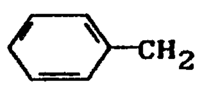 $CH_2$-O | D-Phe | D-His | NH-BOC | I: 0,27 - 0,38 | m/z 690 (M + H)<br>m/z 712 (M + Na) |
| 88 | HO- | D-Phe | D-His | NH-BOC | IV: 0,54 - 0,60 | m/z 600 (M + H)<br>m/z 622 (M + Na) |
| 89 | 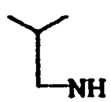 | D-Phe | D-His | NH-BOC | III: 0,52 - 0,64 | m/z 655 (M + H)<br>m/z 677 (M + Na) |

Beispiel 90

4-[N-(2-carboxy)benzoyl]amino-2-R,S-benzyl-3-R,S-hydroxybutyryl-D-Histidyl-D-Phenylalanin-(2-Methyl)-propylamid

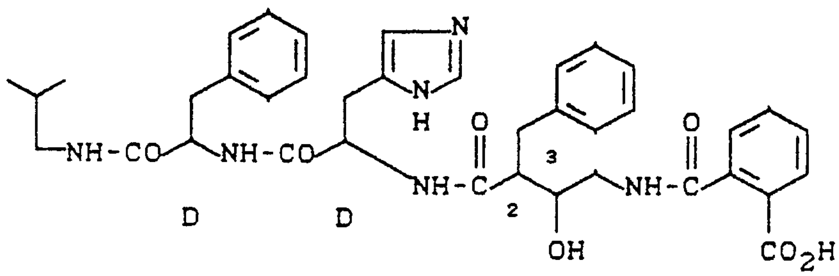

180 mg (250 µmol) der Verbindung aus Beispiel 82 werden in 500 µl Dioxan gelöst und mit 350 µl N NaOH versetzt. Der Ansatz wird über Nacht bei Raumtemperatur gerührt, vom Dioxan abrotiert und mit 1 N HCl auf pH 7 gestellt. Das Produkt wird in Diethylether extrahiert. Die organische Phase wird über $Na_2SO_4$ getrocknet und eingeengt.
Ausbeute: 130 mg (35 % der Theorie)
DC-System III: Rf = 0,03 - 0,08
(+)FAB-MS: m/z 697 (M + H); m/z 719 (M + Na)

Beispiel 91

4-[N-(2-carb)picolyl]amid]-benzoyl)amino-2-R,S-benzyl-3-R,S-hydroxybutyryl-D-Histidyl-D-Phenylalanin-(2-methyl)propylamid

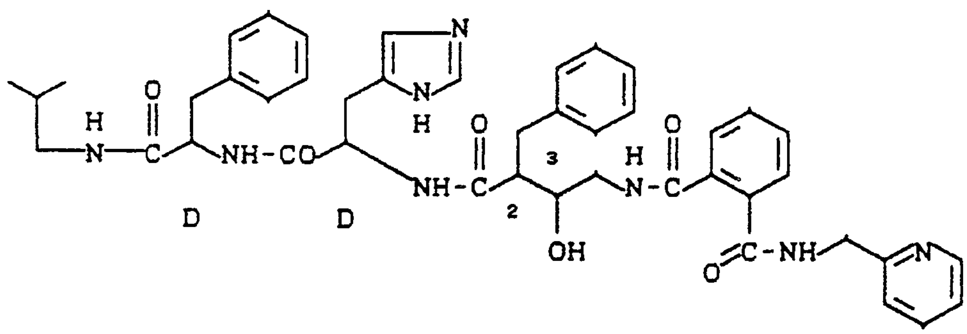

40 mg (57 µmol) der Verbindung aus Beispiel 90 und 7 µl (69 µmol) 2-Picolylamin werden in 2 ml $CH_2Cl_2$ gelöst. Der Ansatz wird unter Rühren mit 100 µl 9574 µmol) DIPEA und schließlich 55 µl (69 µmol) einer 50%igen Lösung von n-Propylphosphonsäureanhydrid in $CH_2Cl_2$ ("PPA", Hoechst AG) versetzt und über Nacht bei Raumtemperatur gerührt. Der Ansatz wird mit $CH_2Cl_2$ auf 10 ml aufgefüllt und mit ges. $NaHCO_3$-Lösung, Puffer pH 7 (Merck, Art.-Nr. 9439) und ges. NaCl-Lösung gewaschen, über $Na_2SO_4$ getrocknet und eingeengt.

Das Rohgemisch wird an Kieselgel 40 - 63 µm (Merck, Art.-Nr. 9385) mit einem Gradienten von $CH_2Cl_2$/MeOH 100 : 0 - 95 : 5 chromatographiert. Die produktenhaltenden Fraktionen werden vereinigt, eingeengt und am Hochvakuum getrocknet.
Ausbeute: 7 mg (16 % der Theorie)

DC-System III: Rf = 0,17 - 0,29
(+)FAB-MS: m/z 787 (M + H), m/z 809 (M + Na)

Beispiele 92 - 95

Die in Tabelle 8 aufgeführten Verbindungen wurden in Analogie zu den Beispielen 90 und 91 aus den Verbindungen der Beispiele 72 und 90 hergestellt.

Tabelle 8

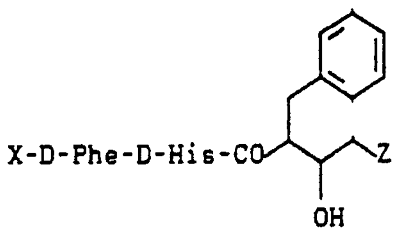

| Beisp. Nr. | X | Z | DC-System Rf-Werte | (+)FAB-MS |
|---|---|---|---|---|
| 92 | 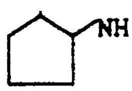 | 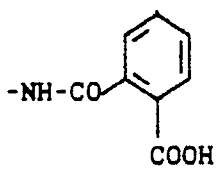 | III: 0,03-0,07 | m/z 709 (M+H)<br>m/z 731 (M+Na) |
| 93 | 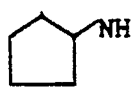 | 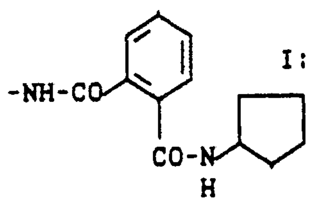 | I: 0,22 | m/z 776 (M+H)<br>m/z 798 (M+Na) |
| 94 | 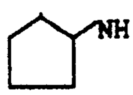 | 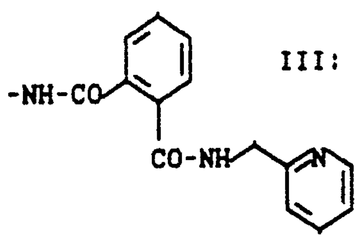 | III: 0,20-0,28 | m/z 799 (M+H) |
| 95 | 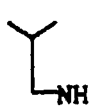 | 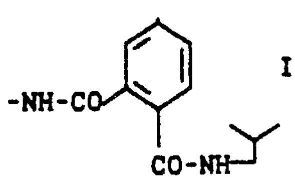 | III: 0,52-0,60 | m/z 752 (M+H)<br>m/z 774 (M+Na) |

Beispiele 96 - 102

Die in Tabelle 9 aufgeführten Verbindungen wurden aus den entsprechenden Vorstufen durch Abspaltung der N-tert.-Butoxycarbonylgruppen mit 4 N HCl in Dioxan in Analogie zu Beispiel 40 hergestellt.

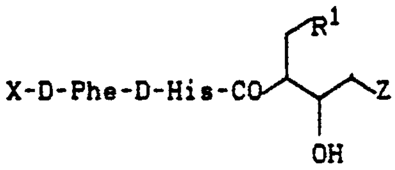 x 2 HCl

| Beisp. Nr. | X | $R^1$ | Z | DC-System Rf-Werte | (+)FAB-MS |
|---|---|---|---|---|---|
| 96 | $CH_3O$ | $C_6H_5$ | $-NH-C_6-H_5$ | III: 0,18-0,12 | m/z 598 (M |
| 97 | 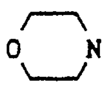 | $C_6H_5$ | $-NH-C_6H_5$ | III: 0,15 | m/z 653 (M+H)<br>m/z 675 (M+Na) |
| 98 | 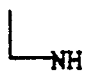 | $C_6H_5$ | $-HN-C_6H_5$ | III: 0,12 | m/z 653 (M+H)<br>m/z 675 (M+Na) |
| 99 | 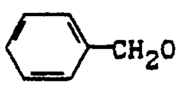 | $C_6H_5$ | $-NH_2$ | III: 0,02-0,10 | m/z 684 (M+H) |
| 100 | 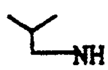 | $C_6H_5$ | $-NH_2$ | IV: 0,30-0,36 | m/z 549 (M+H) |
| 101 | 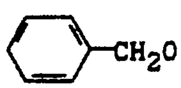 | $C_6H_{11}$ | $-NH_2$ | III: 0,14-0,40 | m/z 590 (M+H) |
| 102 | 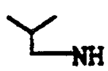 | $C_6H_{11}$ | $-NH_2$ | III: 0,16-0,20<br>IV: 0,18-0,30 | m/z 555 (M+H) |

Beispiel 103

4-[N-Benzyl-N-(3-Pyridyl)acetyl]amino-2-R,S-benzyl-3-R,S-hydroxybutyryl-D-Histidyl-D-Phenylalanin-Methylester

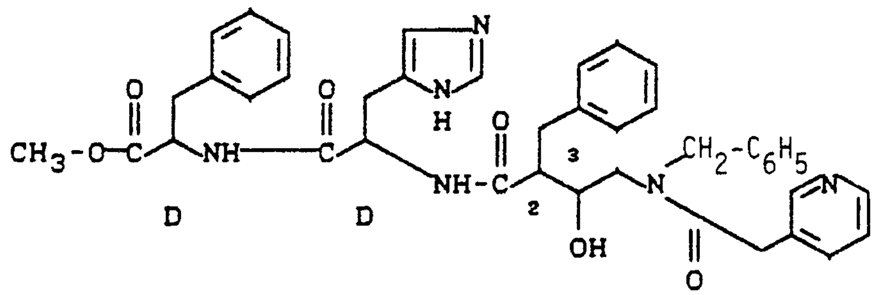

50 mg (84 µmol) der Verbindung aus Beispiel 90 und 17,4 g (100 µmol) 3-Pyridylessigsäure werden in 1 ml $CH_2Cl_2$ gelöst und nacheinander mit 146 µl (836 µmol) DIPEA und 80 µl (100 µmol) einer 50%igen Lösung von n-Propylphosphonsäureanhydrid in $CH_2Cl_2$ ("n-PPA", Hoechst AG) in Dichlormethan versetzt. Der Ansatz wird über Nacht gerührt, auf 10 ml aufgefüllt und mit ges. $NaHCO_3$-Lösung, Puffer pH 7 (Merck, Art.-Nr. 9439) und ges. NaCl-Lösung gewaschen, über $Na_2SO_4$ getrocknet und eingeengt.

Das Rohprodukt wird über 3,5 g Kieselgel 40 - 63 µm (Merck. Art.-Nr. 9385) mit einem Gradienten von $CH_2Cl_2$/MeOH 100 : 0 - 95 : 5 chromatographiert. Die produktenthaltenden, Pauli-positiven Fraktionen werden vereinigt und eingeengt.

Ausbeute: 18 mg (28,6 % der Theorie)
DC-System III: Rf = 0,32
HPLC-System II: Rt = 1,73 min, 2,12 min, 2,80 min und 3,53 min.
(+)FAB-MS: m/z 717 (M + H)

Beispiele 104 - 108

Die in Tabelle 10 aufgeführten Verbindungen wurden aus den in Tabelle 9 angegebenen Verstufen durch n-PPA-Kupplung mit den entsprechenden Säuren bzw. Aminosäurederivaten unter Standardaufarbeitung und Kieselgelchromatographie in Analogie zu Beispiel 103 hergestellt.

Alternativ kann auch eine DCC-HOBT-Kupplung der entsprchenden Edukte durchgeführt werden.

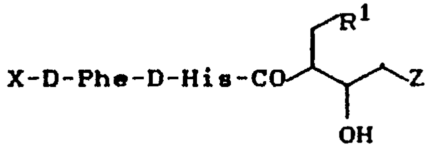

| Beisp. Nr. | X | $R^1$ | Z | DC-System Rf-Werte | (+)FAB-MS |
|---|---|---|---|---|---|
| 104 | 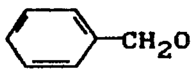 | $C_6H_5$ | 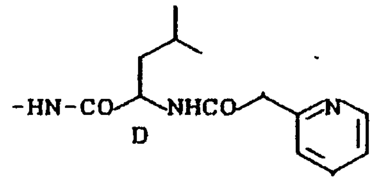 | III: 0,21-0,35 | m/z 816 (M+H) |
| 105 | 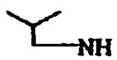 | $C_6H_5$ | 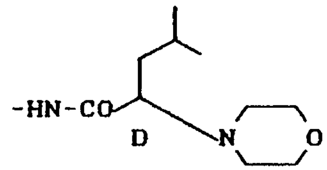 | IV: 0,44, 0,45, 0,49, 0,53 | m/z 732 (M+H) m/z 675 (M+H) |
| 106 | 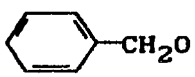 | $C_6H_{11}$ | 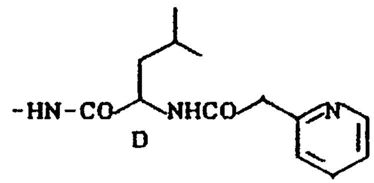 | III: 0,49, 0,54 0,57 0,63 | m/z 822 (M+H) |
| 107 | 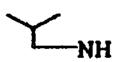 | $C_6H_{11}$ | 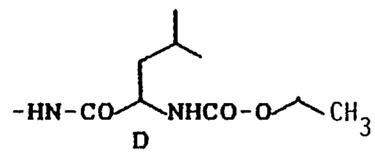 | III: 0,40-0,50 | m/z 740 (M+H) m/z 762 (M+N) |
| 108 | 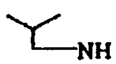 | $C_6H_{11}$ | 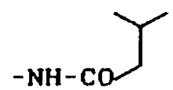 | III: 0,43-0,50 | m/z 639 (M+H) m/z 661 (M+Na) |

Beispiel 109

(4S)-3-[4-(N-tert.-Butoxycarbony]-N-benzyl)amino-(2R)-2-cyclohexylmethyl-(3R,S)-3-hydroxybutyryl]-4-benzyl-oxazolidin-2-on

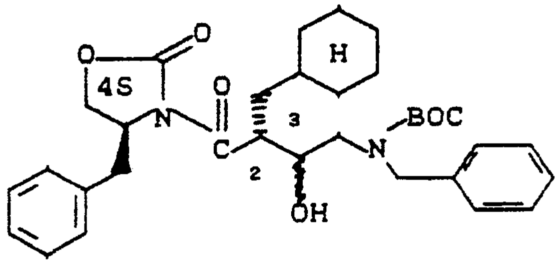

2R 3R,S

Die Titelverbindung wird analog Beispiel 22 ausgehend von der Verbindung aus Beispiel XVIII und dem Aldehyd aus Beispiel IV nach Chromatographie rein erhalten.

HPLC-System II: Rt = 91,73 min (4S,2R, 3R-Isomer)
Rt = 117,52 min (4S,2R, 3S-Isomer)
(+) FAB-MS: m/z 565 (M + H); m/z 587 ( M + Na)

Beispiel 110

4-(N-tert.-Butoxycarbonyl-N-benzyl)amino-(2R)-2-cyclohexylmethyl-(3R,S)-3-hydroxybuttersäure

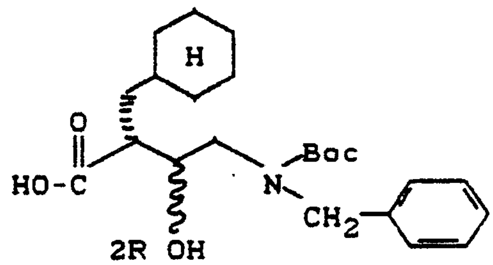

Die Titelverbindung wird durch Abspaltung des chiralen Hilfsreagenzes und nachfolgender Chromatographie (wie in Beispiel 34 beschrieben) aus der Verbindung des Beispiels 109 erhalten.

DC-System II: Rf = 0,42 (2R, 3R-Isomer)
0,38 (2R, 3S-Isomer)

HPLC-System III: Rt = 7,605 min (Ret-index 956),
2R, 3R-Isomer
Rt = 7,754 min (Ret-Index 979),
2R, 3S-Isomer
(+)FAB-MS: m/z 444 (M+K); m/z 482 (M+2K-H) m/z 520 (M+3K-2H).

Beispiele 111-114

Die Titelverbindungen (Tabelle 11) werden durch Chromatographie der entsprechenden Diastereomerengemische (Beispiel 13 für die Beispiele 111 und 114 bzw. Beispiel 110 für die Beispiele 112 und 113) an Kieselgel mit einem $CH_2Cl_2$/MeOH Gradienten (analog Beispiel 10 und Beispiel 11) erhalten. Zuerst eluieren die 2R,3R bzw. 2S, 3S-, dann die 2R,3S bzw 2S,3R-Isomeren. Alternativ kann die Herstellung der Beispiele 111-114 durch Abspaltung der optischen Hilfsreagenzien aus entprechenden enantiomerenreinen Vorstufen (analog Beispiel 34-37) erfolgen.

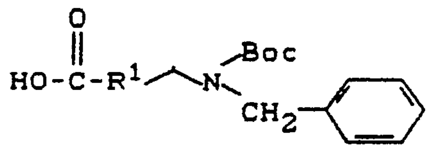

Tabelle 11

| Beispiel-Nr. | $R^1$ | Konfiguration | HPLC-System II; $R_t$ |
|---|---|---|---|
| 111 | 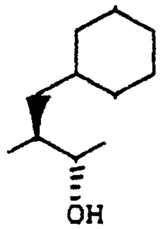 | 2S, 3R | 7,754 min |
| 112 | 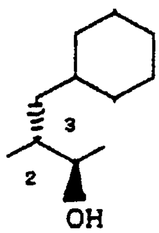 | 2R, 3S | 7,754 min |
| 113 | 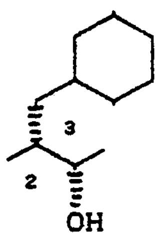 | 2R, 3R | 7,605 min |
| 114 | 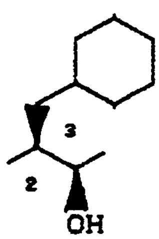 | 2S, 3S | 7,605 min |

Beispiele 115-118

Die in der Tabelle 12 aufgeführten Verbindungen wurden aus den entsprechenden enantiomeren Vorstufen der Beispiele 34-37 durch Abspaltung der BOC-Schutzgruppe in Analogie zu Beispiel 40 hergestellt.

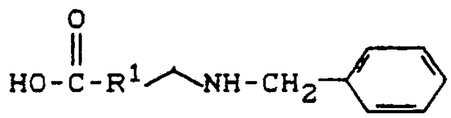 x HCl

Tabelle 12

| Beispiel-Nr. | $R^1$ | Konfiguration | MS-DCI |
|---|---|---|---|
| 115 | 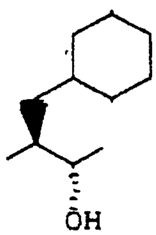 | 2S, 3R | m/z = 306 (M+H) |
| 116 | 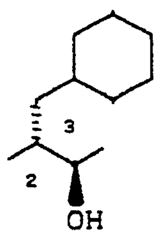 | 2R, 3S | |
| 117 | 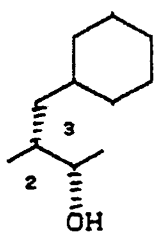 | 2R, 3R | |
| 118 | 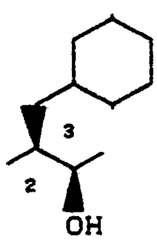 | 2S, 3S | m/z = 306 (M+H) |

Die in den folgenden Tabellen 13-24 aufgeführten Verbindungen werden aus den entsprechenden Vorstufen nach bereits beschriebenen bzw. bekannten Methoden der Peptidchemie erhalten.

| Tabelle | Beispiele | Vorstufe(n) |
|---|---|---|
| 13 | 119 - 123; 129 | Beispiel 7 |
| 14 | 124 - 128; 130 | Beispiel 8 |
| 15 | 131 - 135 | Beispiele 10-11 |
| 16 | 136 - 139 | Beispiele 34-37 |
| 17 | 140 - 143 | Beispiele 136-139 |
| 18 | 144 - 147 | Beispiele 140-143 |
| 19 | 148 - 151 | Beispiele 34-37 |
| 20 | 152 - 157 | Beispiel 111 + 114 |
| 21 | 158 - 161 | Beispiele 58-62 |
| 22 | 162 - 165 | Beispiele 158-161 |
| 23 | 166 - 169 | Beispiele 162-165 |
| 24 | 170 - 173 | Beispiele 111 + 114 |

Tabelle 13

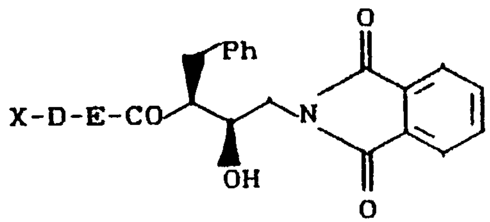

| Beisp. Nr. | X | D | E | DC-System Rf-Werte | HPLC-System Rt-Werte | (+)FAB-MS |
|---|---|---|---|---|---|---|
| 119 | 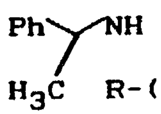 R-(+)- | - | - | I: 0,89 | II: 5,37 min | m/z 433 (M+H) |
| 120 | 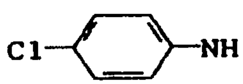 | - | - | | II: 6,32 min | |
| 121 | 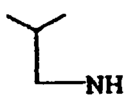 | D-Phe | D-His | | II: 4,34 min | m/z 679 (M+H) |
| 122 | " | L-Phe | L-His | | II: 4,65 min | m/z 701 (M+Na) |

**Tabelle 13** - Fortsetzung -

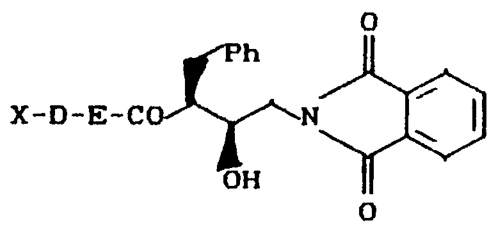

| Beisp. Nr. | X | D | E | DC-System Rf-Werte | HPLC-System Rt-Werte | (+)FAB-MS |
|---|---|---|---|---|---|---|
| 123 | 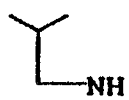 | D-Phe | D-Val- | | II: 8,19 min | m/z 641 (M+H) |
| 129 | $CH_3O$ | D-Phe | 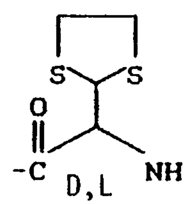 | | II: 7,78 min | m/z 662 (M+H)<br>m/z 684 (M+Na) |

Tabelle 14

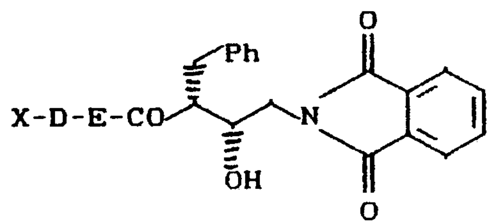

| Beisp. Nr. | X | D | E | DC-System Rf-Werte | HPLC-System Rt-Werte | (+)FAB-MS |
|---|---|---|---|---|---|---|
| 124 | 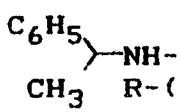 R-(+)- | - | - | I: 0,82 | II: 5,11 min | m/z 433 (M+H) |
| 125 | 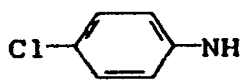 | - | - | | II: 6,32 min | |
| 126 | 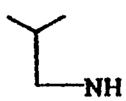 | D-Phe | D-His | | II: 4,68 min | m/z 679 (M+H) |
| 127 | " | L-Phe | L-His | | II: 4,36 min | m/z 701 (M+Na) |

**Tabelle 14** - Fortsetzung -

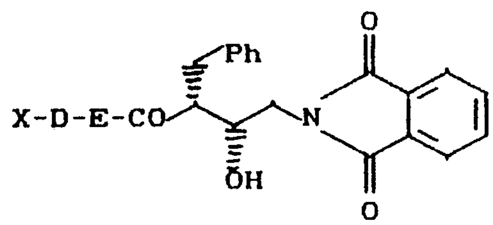

| Beisp. Nr. | X | D | E | DC-System Rf-Werte | HPLC-System Rt-Werte | (+)FAB-MS |
|---|---|---|---|---|---|---|
| 128 | 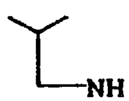 | D-Phe | D-Val | | II: 7,52 min | m/z 641 (M+H) |
| 130 | $CH_3O$ | D-Phe | 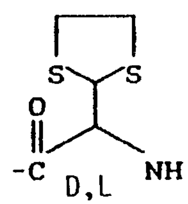 | | II: 6,95 min<br>8,76 min | m/z 662 (M+H)<br>m/z 684 (M+Na) |

Tabelle 15

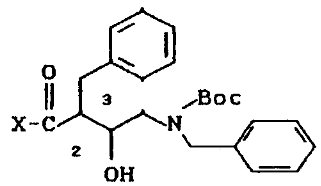

| Beisp. Nr. | X | Konfiguration | DC-System Rf-Werte | HPLC-System II Rt-Werte | (+)FAB-MS |
|---|---|---|---|---|---|
| 131 | 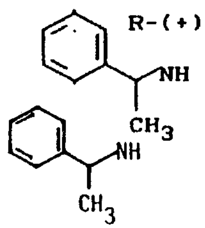 R-(+) | 2R,3R; 2S,3S | IX: 0,40<br>0,36 | 17,45 min<br>18,31 min | m/z = 503 (M+H) |
| 132 | | 2R,3S; 2S,3R | IX: 0,29<br>0,24 | 23,50 min<br>25,03 min | m/z = 503 (M+H) |
| 133 | 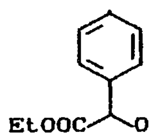 | 2R,3S; 2S,3S | VII: 0,38<br>0,33 | 63,1 min, breit | m/z = 562 (M+H) |
| 134 | 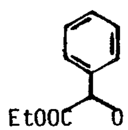 S-(+)- | 2R,3S; 2S,3R | VIII: 0,91 | 69,8 min, breit | |
| 135 | 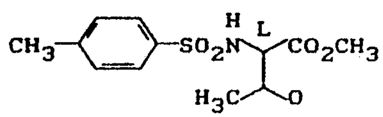 | 2R,3R; 2S,3S | | 37,87 min<br>38,17 min | m/z = 669 (M+H) |

**Tabelle 16**

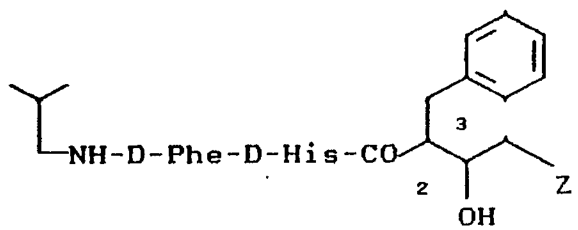

| Beispiel Nr. | Konfiguration | Z | DC-System Rf-Werte | HPLC-System II Rt-Werte | (+)FAB-MS |
|---|---|---|---|---|---|
| 136 | 2S,3R | NH-Boc | | 4,37 min | m/z 655 (M+Li) |
| 137 | 2R,3S | NH-Boc | | 4,25 min | m/z 655 (M+Li) |
| 138 | 2R,3R | NH-Boc | | 3,89 min | m/z 655 (M+Li) |
| 139 | 2S,3S | NH-Boc | | 3,69 min | m/z 655 (M+Li) |

Tabelle 17

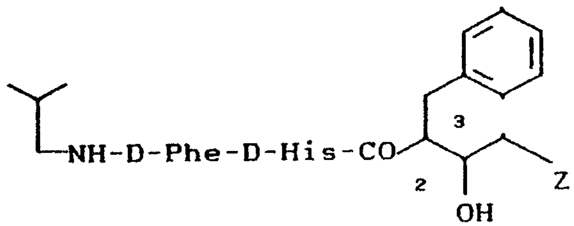

| Beispiel Nr. | Konfiguration | Z | DC-System Rf-Werte | HPLC-System II Rt-Werte | (+)FAB-MS |
|---|---|---|---|---|---|
| 140 | 2S,3R | $NH_2$ x HCl | III: 0,05 | | m/z 549 (M+H), m/z 571 (M+Na) |
| 141 | 2R,3S | $NH_2$ x HCl | III: 0,06 | | m/z 549 (M+H), m/z 571 (M+Na) |
| 142 | 2R,3R | $NH_2$ x HCl | III: 0,06 | | m/z 549 (M+H), m/z 571 (M+Na) |
| 143 | 2S,3S | $NH_2$ x HCl | III: 0,05 | | m/z 549 (M+H), m/z 571 (M+Na) |

**Tabelle 18**

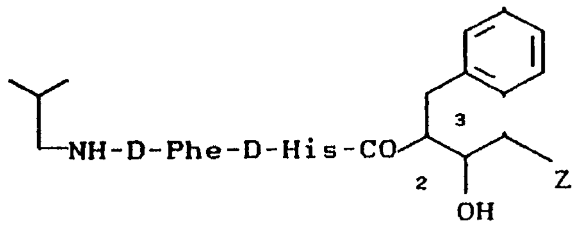

| Beispiel Nr. | Konfiguration | Z | DC-System Rf-Werte | HPLC-System II Rt-Werte | (+)FAB-MS |
|---|---|---|---|---|---|
| 144 | 2S,3R | NH-D-Leu-$NHCOOC_2H_5$ | I: 0,31 | 4,73 min | m/z 734 (M+H),<br>m/z 756 (M+Na) |
| 145 | 2R,3S | 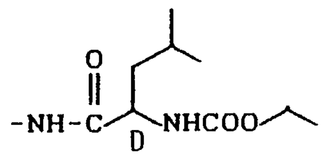 | I: 0,37 | 4,29 min | m/z 734 (M+H),<br>m/z 756 (M+Na) |
| 146 | 2R,3R | 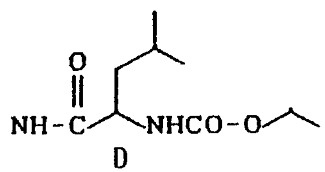 | I: 0,41 | 4,68 min | m/z 734 (M+H)<br>m/z 756 (M+Na) |
| 147 | 2S,3S | 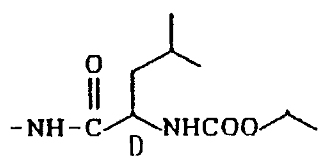 | I: 0,35 | 4,14 min | m/z 734 (M+H),<br>m/z 756 (M+Na) |

**Tabelle 19**

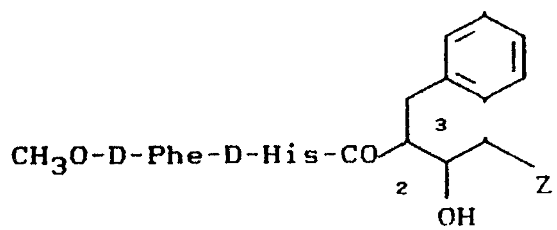

| Beispiel Nr. | Konfiguration | Z | DC-System Rf-Werte | HPLC-System II Rt-Werte | (+)FAB-MS |
|---|---|---|---|---|---|
| 148 | 2S,3R | 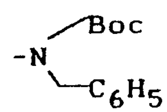 | | 14,64 min | m/z 698 (M+H), m/z 720 (M+Na) |
| 149 | 2R,3S | 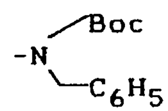 | | | m/z 698 (M+H), m/z 720 (M+Na) |
| 150 | 2R,3R | 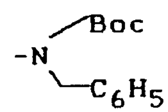 | | | m/z 698 (M+H), m/z 720 (M+Na) |
| 151 | 2S,3S | 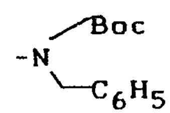 | | 11,25 min | m/z 698 (M+H), m/z 720 (M+Na) |

Tabelle 20

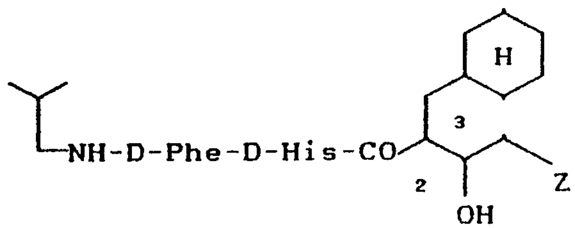

| Beispiel Nr. | Konfiguration | Z | DC-System Rf-Werte | HPLC-System II Rt-Werte | (+)FAB-MS |
|---|---|---|---|---|---|
| 152 | 2S,3S | 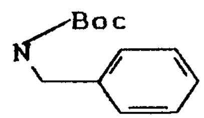 | I: 0,56 | 24,06 min | m/z 745 (M+H) |
| 153 | 2S,3R | 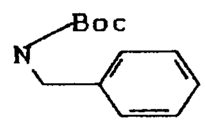 | I: 0,56 | 29,96 min | m/z 745 (M+H) |
| 154 | 2S,3S | 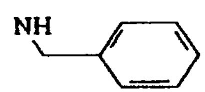 x HCl | IV: 0,16 | | m/z 645 (M+H) |
| 155 | 2S,3R | 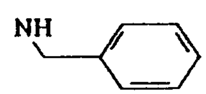 x HCl | IV: 0,16 | | m/z 645 (M+H) |

**Tabelle 20** - Fortsetzung -

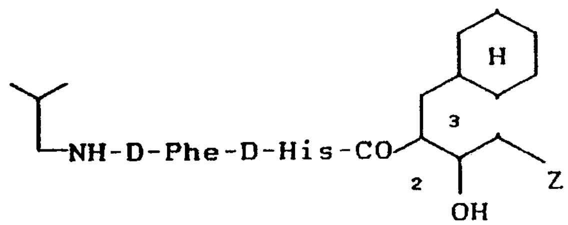

| Beispiel Nr. | Konfiguration | Z | DC-System Rf-Werte | HPLC-System II Rt-Werte | (+)FAB-MS |
|---|---|---|---|---|---|
| 156 | 2S,3S | 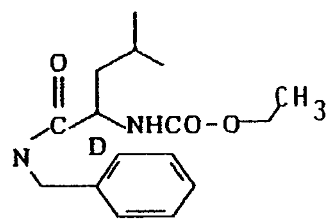 | I: 0,56 | 19,15 min | m/z 830 (M+H), m/z 836 (M+Li), m/z 852 (M+Na) |
| 157 | 2S,3R | 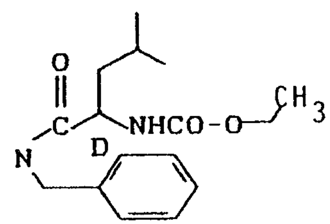 | III: 0,56 | 24,03 min | m/z 830 (M+H), m/z 836 (M+Li), m/z 852 (M+Na) |

<u>Tabelle 21</u>

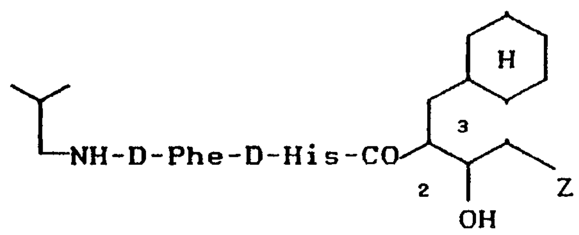

| Beispiel Nr. | Konfiguration | Z | DC-System Rf-Werte | HPLC-System II Rt-Werte | (+)FAB-MS |
|---|---|---|---|---|---|
| 158 | 2S,3R | -NH-Boc | III: 0,47 | 7,37 min | m/z 655 (M+H) |
| 159 | 2R,3S | -NH-Boc | | | |
| 160 | 2R,3R | -NH-Boc | | | |
| 161 | 2S,3R | -NH-Boc | III: 0,43 | 6,03 min | m/z 655 (M+H) |

Tabelle 22

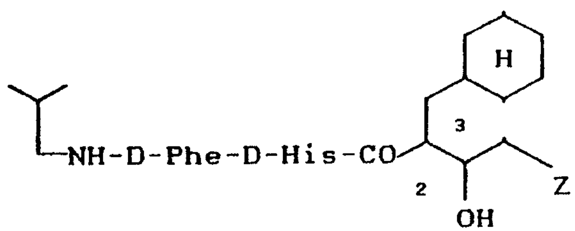

| Beispiel Nr. | Konfiguration | Z | DC-System Rf-Werte | HPLC-System II Rt-Werte | (+)FAB-MS |
|---|---|---|---|---|---|
| 162 | 2S,3R | $NH_2$ x HCl | | | m/z 555 (M+H)<br>m/z 561 (M+Li) |
| 163 | 2R,3S | $NH_2$ x HCl | | | m/z 555 (M+H)<br>m/z 561 (M+Li) |
| 164 | 2R,3R | $NH_2$ x HCl | | | m/z 555 (M+H)<br>m/z 561 (M+Li) |
| 165 | 2S,3S | $NH_2$ x HCl | | | m/z 555 (M+H),<br>m/z 561 (M+Li) |

Tabelle 23

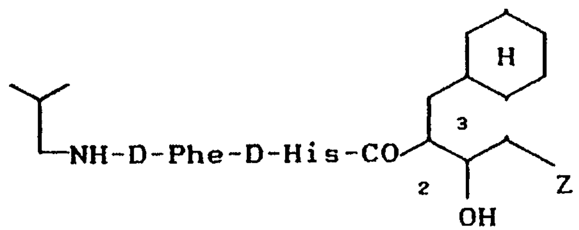

| Beispiel Nr. | Konfiguration | Z | DC-System Rf-Werte | HPLC-System II Rt-Werte | (+)FAB-MS |
|---|---|---|---|---|---|
| 166 | 2S,3R | 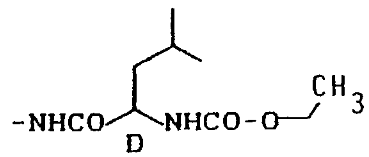 | | | m/z 740 (M+H) |
| 167 | 2R,3S | 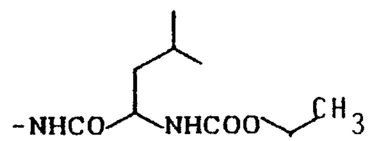 | | | m/z 740 (M+H) |
| 168 | 2R,3R | 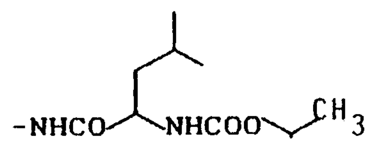 | | | m/z 740 (M+H) |
| 169 | 2S,3S | 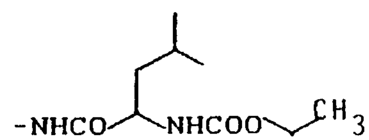 | III: 0,39 | 5,59 min | m/z 740 (M+H)<br>m/z 746 (M+Li) |

Tabelle 24

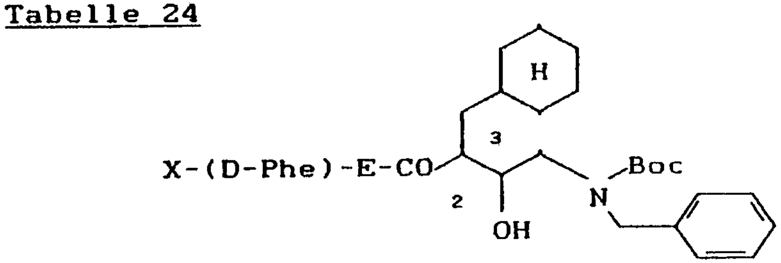

| Beispiel Nr. | X | E | Konfiguration | DC-System Rf-Werte | HPLC-System II Rt-Werte | (+)FAB-MS |
|---|---|---|---|---|---|---|
| 170 | 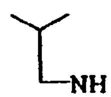 | D-Val | 2S,3S | I: 0,79 | Rt = 63,82 min | m/z 713 (M+Li) |
| 171 | 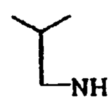 | D-Val | 2S,3R | | Rt = 82,71 min | m/z 713 (M+Li) |
| 172 | $CH_3O$- | 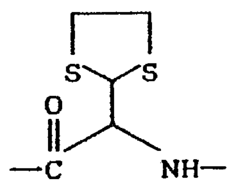 R,S | 2S,3S | | | |
| 173 | $CH_3O$- | 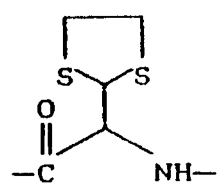 R,S | 2S,3R | | | |

Beispiel 174

(4S)-3-[4-(N-Benzyl)amino-2RS)-2-benzyl-(3S)-3-hydroxybutyryl]-4-(2-propyl)oxazolidin-2-on Hydrochlorid

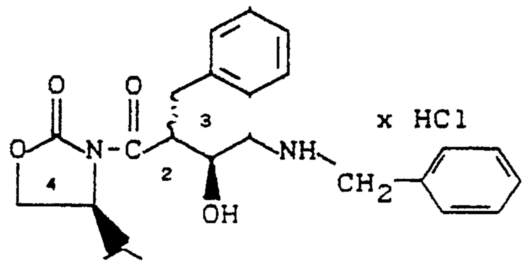

4S 2R, 3S

Die Titelverbindung wird durch Abspaltung der Boc-Schutzgruppe (analog Beispiel 40) aus 4,19 g (8,2 Mmol) eines Gemisches der Beispiel 19 und 20 erhalten.
Ausbeute: 3,38 g (92 % der Theorie)
DC-System IV: 0,46 und 0,41
(+)FAB-MS: m/z 411 (M+H)

Beispiel 175

(4R,5S)-3-[4-(N-Benzyl)amino-(2S)-2-benzyl-(3R,S)-3-hydroxy-butyryl]-4-methyl-5-phenyloxazolidin-2-on Hydrochlorid

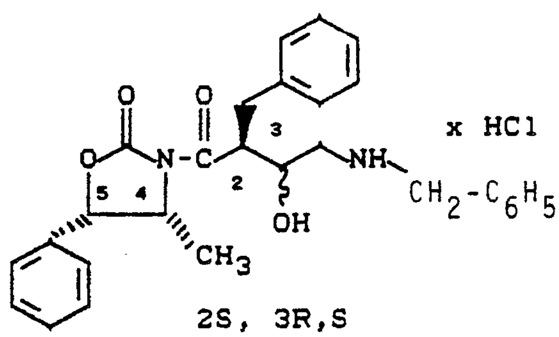

2S, 3R,S

Die Titelverbindung wird analog Beispiel 174 aus 6,96 g (12,4 Mmol) des Beispieles 26 erhalten.
Ausbeute: 4,68 g (76 % der Theorie)
DC-System III: Rf = 0,56 und 0,48
(+)FAB-MS: m/z 459 (M+H)

Beispiel 176

(4R)-3-[4-(N-Benzyl)amino-2S)-2-benzyl-(3R,S)-3-hydroxybutyryl]-4-benzyl-oxazolidin-2-on Hydrochlorid

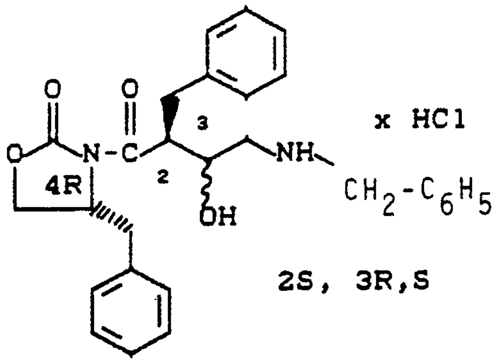

x HCl

2S, 3R,S

Die Titelverbindung wird analog Beispiel 174 aus 14,57 g (25,7 Mmol) des Beispieles 22 erhalten.
Ausbeute: 10 g (83,4 % der Theorie)
DC-System III: Rf = 0,58 und 0,52
(+)FAB-MS: m/z 465 (M+H)

Beispiel 177

(4S)-3-[4-Amino-(2R,S)-2-benzyl-(3S)-3-hydroxy-butyryl]-4-(2-propyl)oxazolidin-2-on-hydrochlorid (Beispiel 19)

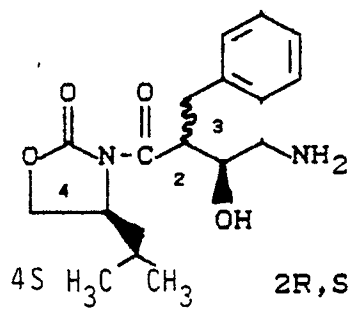

x HCl

2R,S 3

300 mg (0,67 Mmol) der Verbindung aus dem Beispiel 174 werden in ca. 120 ml Dioxan/Methanol/Wasser 60/40/20 gelöst und nach Zusatz von 150 mg Pd $(OH)_2$ bei 3,5 bar hydriert. Nach 6 Stunden wird vom Katalysator abfiltriert, vom Dioxan/Methanol abrotiert, mit Wasser verdünnt und lyophilisiert.
Ausbeute: 240 mg (100 % der Theorie)
DC-System V: Rf = 0,63
(+)FAB-MS m/z 321 (M+H); m/z 327 (M+Li); m/z 343 (M+Na)

Beispiel 178

(4R,5S)-3-[4-(N-Acetyl-N-benzyl)amino-(2S)-2-benzyl-(3R)-3-hydroxy-butyryl]-4-methyl-5-phenyl-oxazolidin-2-on

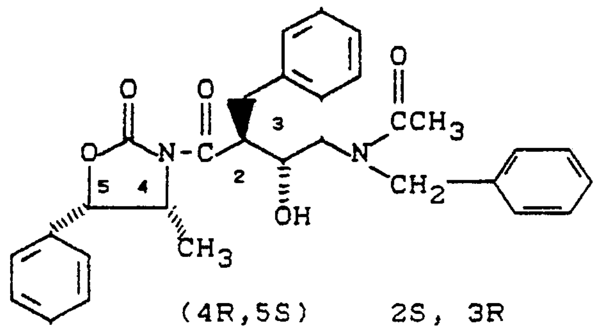

(4R,5S) 2S, 3R

Die Titelverbindung wird ausgehend von (4R,5S)-3-[4-(N-benzyl)amino-(2S)-2-benzyl-(3R)-3-hydroxy-butyryl]-4-methyl-5-phenyl-oxazolidin-2-on Hydrochlorid (erhältlich aus Beispiel 27 durch Abspaltung der Boc-Schutzgruppe analog Beispiel 175) durch PPA-Kopplung mit Essigsäure (analog Beispiel 91) und Kieselgelchromatographie erhalten.

DC-System II: Rf = 0,56

HPLC-System II: Rt = 10,75 min

(+)FAB-MS m/z 501 (M+H); m/z 507 (M+Li);

Beispiel 179

(4R,5S)-3-[4-(N-Benzyl)-N-({N-ethoxycarbonyl}-D-leucyl)amino-(2S)-2-benzyl-(3R,S)-3-hydroxy-butyryl]-4-methyl-5-phenyl-oxazolidin-2-on

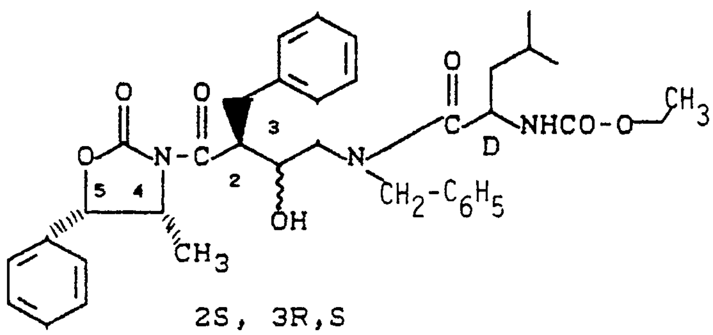

2S, 3R,S

Die Titelverbindung wird ausgehend von der Verbindung aus dem Beispiel 175 durch PPA-Kopplung (analog Beispiel 91) mit N-Ethoxycarbonyl-D-leucin und Chromatographie des Rohproduktes erhalten.

DC-System II: Rf = 0,68 und 0,57

HPLC-System II: Rt = 38,77 min und 46,17 min

(+)FAB-MS m/z 644 (M+H); m/z 650 (M+Li);

Beispiel 180

(4R)-3-[4-N-Benzyl-N-({N-ethoxycarbonyl}-D-leucyl)amino-(2S)-2-benzyl-(3R,S)-3-hydroxy-butyryl]-4-benzyl-oxazolidin-2-on

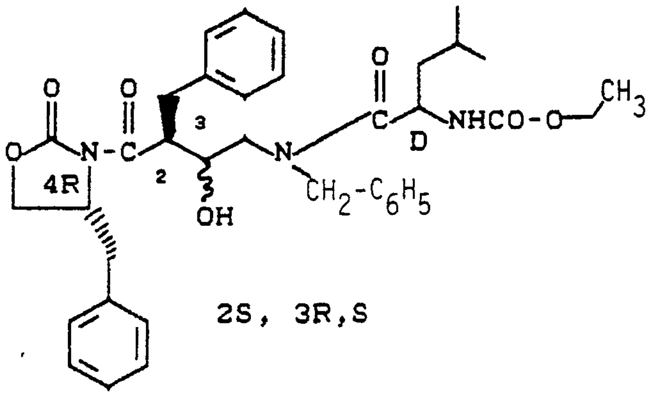

2S, 3R,S

Die Titelverbindung wird analog Beispiel 179 ausgehend von der Verbindung aus dem Beispiel 176 erhalten.

DC-System II: Rf = 0,66 und 0,61
HPLC-System II: Rt = 68,50 min und 85,68 min
(+)FAB-MS m/z 650 (M+H); m/z 672 (M+Na);

Beispiel 181

(2R,S)-2-Benzyl-(3R,S)-3-hydroxy-3-[N-tert.-butoxycarbonyl-(2'S)-pyrrolidyl-(2)]propionsäure-benzylester

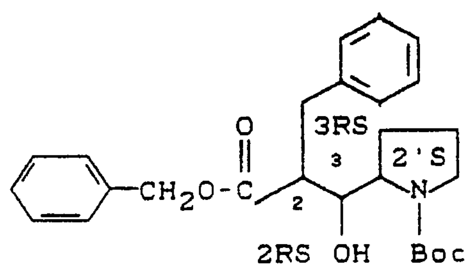

Die Titelverbindung wird analog Beispiel 4 aus 40,9 g (170,5 Mmol) des Beispieles 1 und 30,9 g (155 Mmol) des Beispieles XIX erhalten. Das Rohprodukt (74 g) wird analog chromatographiert.
Ausbeute: 18,8 g (27,6 % der Theorie)
DC-System XV: Rf = 0,10-0,20 (Stereoisomerengemisch)
(+)FAB-MS m/z 440 (M+H); m/z 462 (M+Na);

Beispiel 182

(2R,S)-2-Benzyl-(3R,S)-3-hydroxy-3-[N-tert.-butoxycarbonyl-(2'S)-pyrrolidyl-(2)]propionsäure

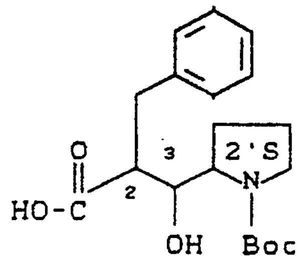

Die Titelverbindung wird analog Beispiel 9 aus 3,64 g (8,28 Mmol) der Verbindung aus Beispiel 181 erhalten.
Ausbeute: 1,65 g (57 % der Theorie)
DC-System I: Rf = 0,58
(+)FAB-MS m/z 388 (M+K); m/z 426 (M+2K-H)

Beispiel 183

(2R,S)-2-Benzyl-(3R,S)-3-hydroxy-3-[N-tert.-butoxycarbonyl-(2′S)-pyrrolidyl-(2)]propionyl-D-asparaginyl-D-phenylalaninbenzylester

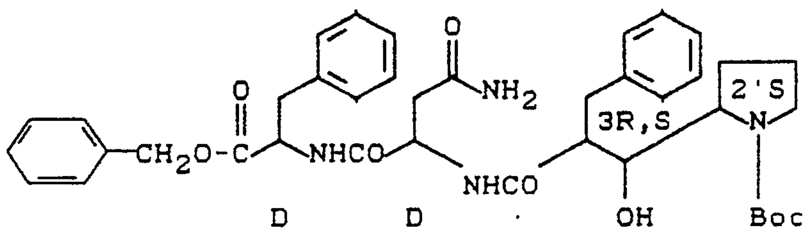

Die Titelverbindung wird analog Beispiel 70 aus der Verbindung des Beispieles 182 und D-Asparaginyl-D-phenylalanin Benzylester Hydrochlorid (erhältlich aus D-Phenylalaninbenzylester und Boc-D-Asparagin analog Beispiel 66 und 67) erhalten.
DC-System I: Rf = 0,48 - 0,58
(+)FAB-MS m/z 701 (M+H); m/z 723 (M+Na)

Beispiel 184

(2R,S)-2-Benzyl-(3R,S)-3-hydroxy-3-[N-tert.-butoxycarbonyl-(2′S)-pyrrolidyl-(2)]propionyl-D-asparaginyl-D-phenylalanin

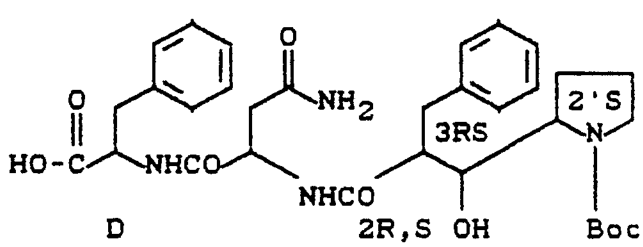

Die Titelverbindung wird analog Beispiel 71 aus der Verbindung des Beispieles 183 erhalten.

DC-System IV: Rf = 0,26
(+)FAB-MS m/z 611 (M+H); m/z 633 (M+Na)

Beispiel 185

(2R,S)-2-Benzyl-(3R,S)-3-hydroxy-[(2'S)-pyrrolidyl-(2)]propionyl-D-asparaginyl-D-phenylalanin Benzylester Hydrochlorid

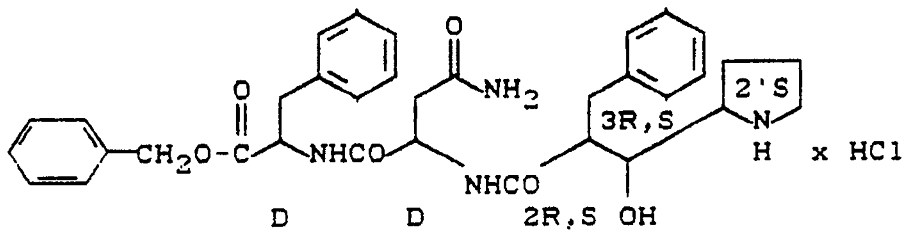

Die Titelverbindung wird aus der Verbindung des Beispieles 183 durch Abspaltung der Boc-Schutzgruppe (analog Beispiel 67) erhalten.
DC-System III: 0,17
(+)FAB-MS : m/z 601 (M+H)

Beispiel 186

(2R,S)-2-Benzyl-(3R,S)-3-hydroxy-[N-{tert.-Butoxycarbonyl-D-leucyl}-(2'S)-pyrrolidyl-(2)]propionyl-D-asparaginyl-D-phenylalanin Benzylester

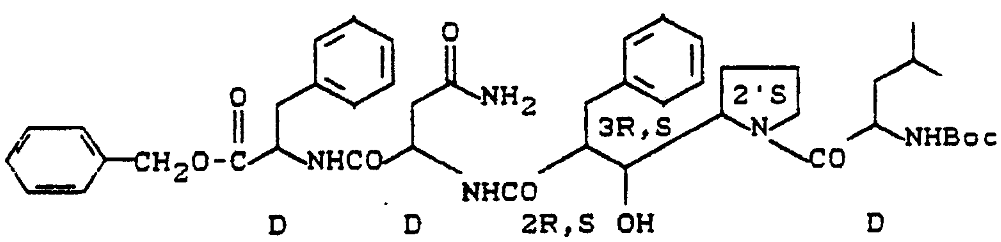

Die Titelverbindung wird analog Beispiel 107 aus der Verbindung des Beispiels 185 und Boc-D-Leu erhalten.
DC-System I: Rf = 0,61
(+)FAB-MS: m/z 814 (M+H), m/z 836 (M+Na)

Beispiel 187

(2R,S)-2-Benzyl-(3R,S)-3-hydroxy-[N-{D-leucyl}-(2'S)-pyrrolidyl-(2)]propionyl-D-asparaginyl-D-phenylalanin Benzylester Hydrochlorid

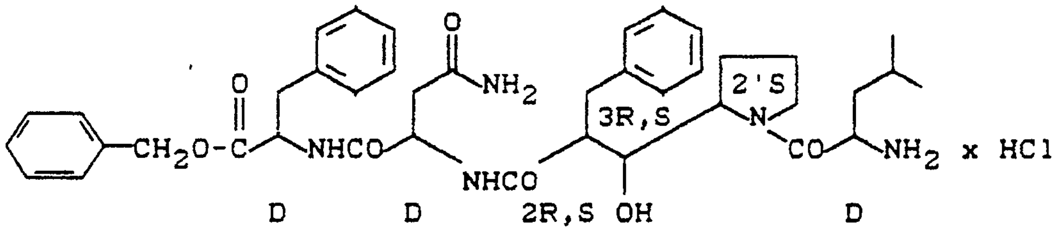

Die Titelverbindung wird analog Beispiel 185 aus der Verbindung des Beispieles 186 erhalten.

DC-System IV: Rf = 0,20

(+)FAB-MS: m/z 714 (M+H); m/z 736 (M+Na)

Beispiel 188

(2R,S)-2-Benzyl-(3R,S)-3-hydroxy-[N-{tert.-Butoxycarbonyl-D-valyl-D-leucyl}-(2′S)-pyrrolidyl-(2)]propionyl-D-asparaginyl-D-phenylalanin Benzylester

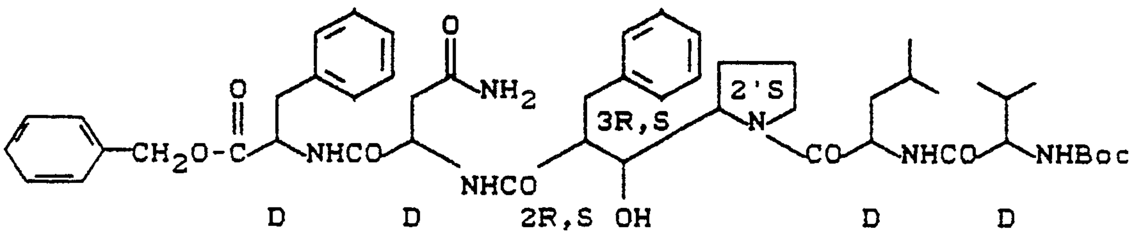

Die Titelverbindung wird analog Beispiel 186 aus der Verbindung des Beispieles 187 und Boc-D-Val erhalten.

DC-System IV: Rf = 0,59

(+)FAB-MS: m/z 913 (M+H)

HPLC-System II: Rf = 16,50 min

**Ansprüche**

1. Peptide der allgemeinen Formel

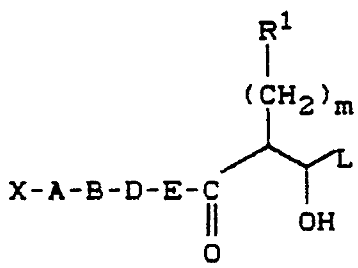

(I)

in welcher

X- für eine Gruppe der Formel

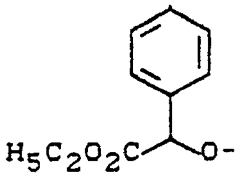 oder 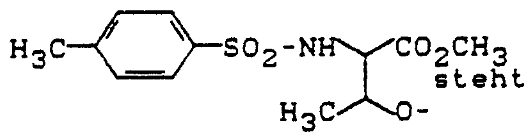 steht oder

für Hydroxy, Alkoxy mit bis zu 8 Kohlenstoffatomen, Benzyloxy oder für eine Gruppe der Formel $-NR^4R^5$ steht, worin

$R^4$ und $R^5$ gleich oder verschieden sind und jeweils Wasserstoff, geradkettiges oder verzweigtes Alkyl mit bis zu 8 Kohlenstoffatomen bedeuten, das gegebenenfalls durch Phenyl substituiert ist, oder Cycloalkyl mit 3 bis 8 Kohlenstoffatomen oder Aryl mit 6 bis 10 Kohlenstoffatomen bedeuten, das seinerseits durch Halogen substituiert sein kann

oder

$R^4$ und $R^5$ gemeinsam mit dem Stickstoffatom einen 5 bis 7-gliedrigen Heterocyclus mit bis zu 3 Heteroatomen aus der Reihe Stickstoff, Schwefel oder Sauerstoff bilden,

A, B, D und E gleich oder verschieden sind und jeweils

- für eine direkte Bindung stehen,
- für einen Rest der Formel

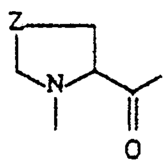

stehen,

worin

Z - Sauerstoff, Schwefel oder die Methylengruppe bedeutet

- für eine Gruppierung der Formel

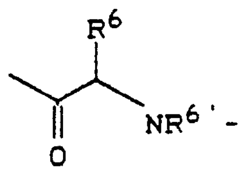 oder 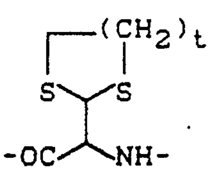

steht,

worin

t - die Zahl 1 oder 2 bedeutet,

$R^6$ - Wasserstoff oder Phenyl bedeutet oder

- geradkettiges oder verzweigtes Alkyl mit bis zu 8 Kohlenstoffatomen bedeutet, das gegebenenfalls durch Carboxy, Hydroxy, Halogen oder durch Alkoxy oder Alkoxycarbonyl mit jeweils bis zu 6 Kohlenstoffatomen substituiert ist, die ihrerseits durch Phenyl substituiert sein können,

oder durch eine Gruppe der Formel $-NR^7R^8$ oder $-CONR^7R^8$ substituiert ist

worin

$R^7$ und $R^8$ gleich oder verschieden sind und jeweils eine Aminoschutzgruppe, Wasserstoff, Acetoxy, Aryl mit 6 bis 10 Kohlenstoffatomen oder geradkettiges oder verzweigtes Alkyl mit bis zu 6 Kohlenstoffatomen bedeuten, das seinerseits durch Amino substituiert sein kann,

oder durch Aryl mit 6 bis 10 Kohlenstoffatomen substituiert ist, das gegebenenfalls bis zu 3-fach gleich oder verschieden durch Halogen, Nitro, Hydroxy oder Alkoxy mit bis zu 6 Kohlenstoffatomen substituiert ist,

oder durch Indolyl, Imidazolyl, Pyridyl, Triazolyl oder Pyrazolyl substituiert ist, die ihrerseits durch $R^8$ substituiert sein können, worin $R^8$ die oben angegebene Bedeutung hat,

$R^{6'}$ - Wasserstoff, Benzyl oder geradkettiges oder verzweigtes Alkyl mit bis zu 6 Kohlenstoffatomen bedeutet,

in ihrer D-Form, L-Form oder als D, L-Isomerengemisch,
m - für eine Zahl 0, 1 oder 2 steht,
$R^1$ - für geradkettiges oder verzweigtes Alkyl mit bis zu 8 Kohlenstoffatomen steht,
- für Cycloalkyl mit 3 bis 8 Kohlenstoffatomen steht,
- für Aryl mit 6 bis 10 Kohlenstoffatomen steht, das gegebenenfalls durch Nitro, Cyano, Halogen oder durch geradkettiges oder verzweigtes Alkyl mit bis zu 6 Kohlenstoffatomen substituiert sein kann,
L - für eine Gruppe der Formel $-CH_2-NR^2R^3$ oder

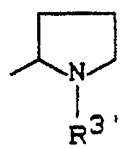

steht,
worin
$R^2$ und $R^3$ gleich oder verschieden sind und jeweils
- für Wasserstoff, Phenyl oder
- für geradkettiges oder verzweigtes Alkyl mit bis zu 8 Kohlenstoffatomen stehen, das gegebenenfalls durch Aryl mit 6 bis 10 Kohlenstoffatomen oder Pyridyl substituiert ist,
- für eine Gruppe der Formel $-COR^9$ stehen,
worin
$R^9$ - geradkettiges oder verzweigtes Alkyl oder Alkoxy mit bis zu 8 Kohlenstoffatomen oder Aryl mit 6 bis 10 Kohlenstoffatomen bedeutet, das seinerseits durch Carboxy, Alkoxycarbonyl mit bis zu 8 Kohlenstoffatomen oder durch eine Gruppe der Formel $-CONR^4R^5$ oder $-CO-NR^{10}R^{11}$ substituiert sein kann,
worin
$R^4$ und $R^5$ die oben angegebene Bedeutung haben und
$R^{10}$ und $R^{11}$ gleich oder verschieden sind und jeweils
- Wasserstoff oder geradkettiges oder verzweigtes Alkyl mit bis zu 8 Kohlenstoffatomen bedeuten, das seinerseits durch Phenyl oder Pyridyl substituiert sein kann,
oder
$R^2$ oder $R^3$ für eine Aminoschutzgruppe steht oder
$R^2$ und $R^3$ gemeinsam für Phthalimido oder Morpholino stehen oder für eine Gruppe der Formel

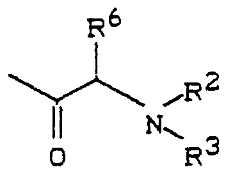

oder

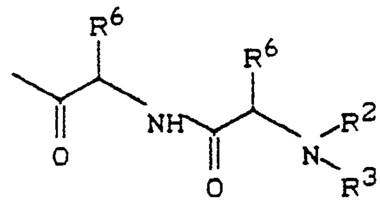

stehen
worin
$R^2$, $R^3$ und $R^6$ die oben angegebene Bedeutung haben
$R^{3'}$ die obenangegebene Bedeutung von $R^3$ hat und mit dieser gleich oder verschieden ist,
und deren physiologisch unbedenklichen Salze.
2. Peptide der Formel I nach Anspruch 1
worin
X - für eine Gruppe der Formel

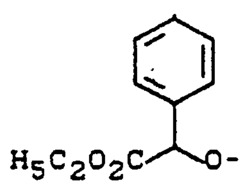

oder

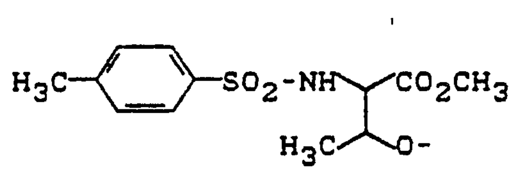

steht

für Hydroxy, Alkoxy mit bis zu 6 Kohlenstoffatomen, Benzyloxy oder für eine Gruppe der Formel $-NR^4R^5$ steht, worin

$R^4$ und $R^5$ gleich oder verschieden sind und jeweils Wasserstoff, geradkettiges oder verzweigtes Alkyl mit bis zu 6 Kohlenstoffatomen bedeuten, das gegebenenfalls durch Phenyl substituiert ist oder Cyclopropyl, Cyclopentyl, Cyclohexyl oder Phenyl bedeuten, das seinerseits durch Fluor oder Chlor substituiert ist, oder

$R^4$ und $R^5$ gemeinsam mit dem Stickstoffatom einen Morpholinring ausbilden,

A, B, D und E gleich oder verschieden sind und jeweils

- für eine direkte Bindung stehen oder
- für einen Rest der Formel

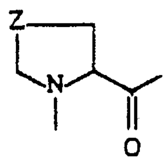

stehen,

worin

Z - Sauerstoff, Schwefel oder die Methylengruppe bedeutet,

oder

- für einen Rest der Formel

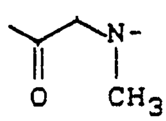 , 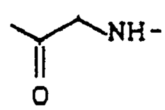 , 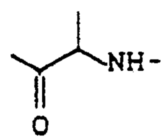 , 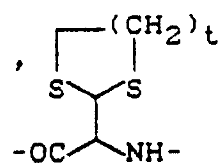 ,

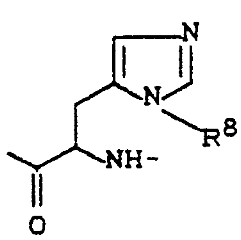 , 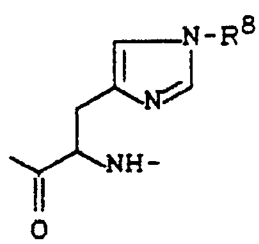 , 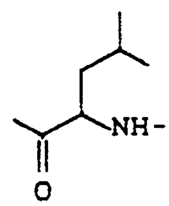 ,

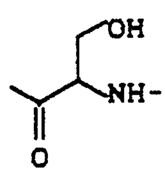 , 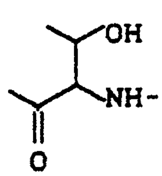 , 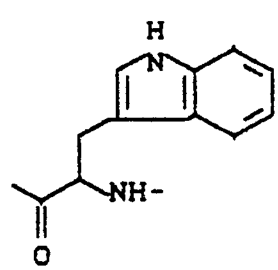 ,

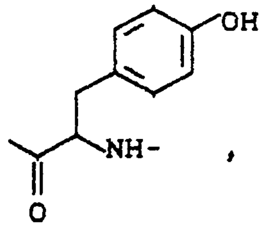 , 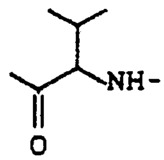 , 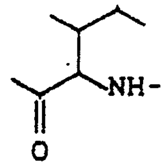 ,

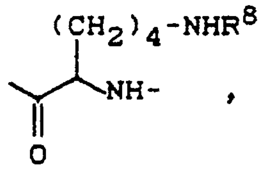 , 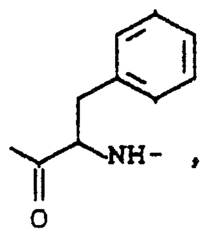 , 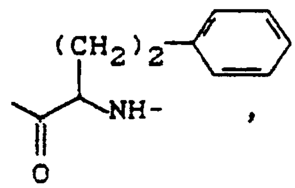 ,

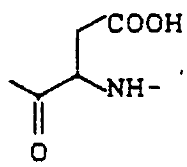 , 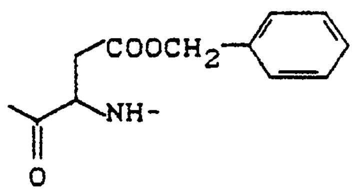 , 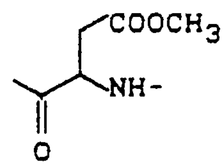

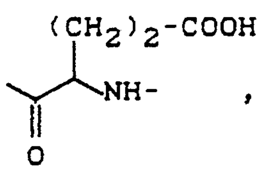 , 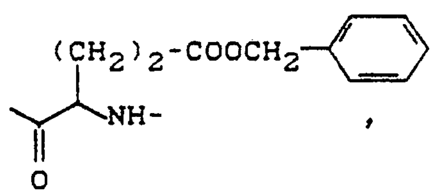 , 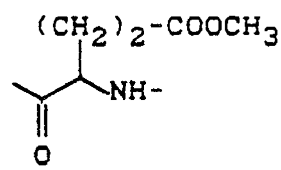

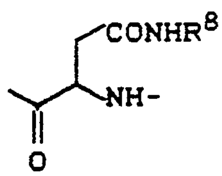 , 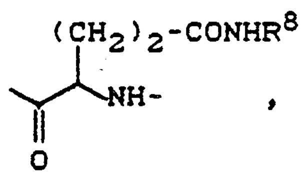 , 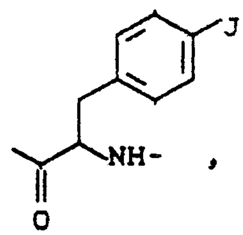 ,

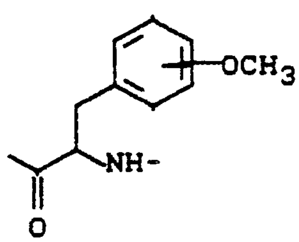 , 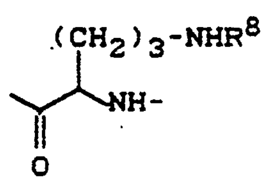 , 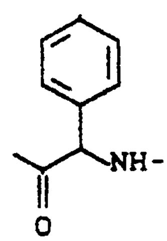 ,

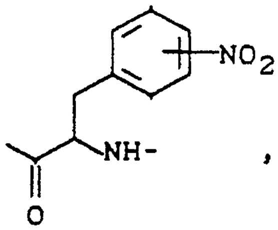 , 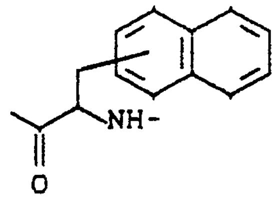 ,

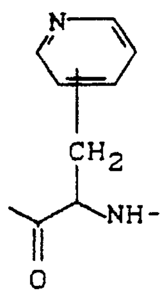 , 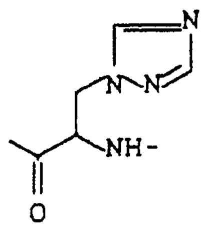 oder 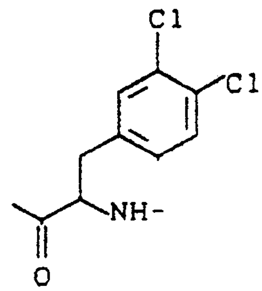

steht
in welcher
t - die Zahl 1 oder 2 bedeutet,
$R^8$ Wasserstoff, geradkettiges oder verzweigtes Alkyl mit bis zu 4 Kohlenstoffatomen oder eine Aminoschutzgruppe bedeutet,
in ihrer D-Form, L-Form oder als D, L-Isomerengemisch,
m - für eine Zahl 1 oder 2 steht,
$R^1$ - für geradkettiges oder verzweigtes Alkyl mit bis zu 6 Kohlenstoffatomen steht,
- für Cyclopropyl, Cyclopentyl oder Cyclohexyl steht,
- für Phenyl steht, das gegebenenfalls durch Nitro, Cyano, Fluor oder Chlor substituiert ist.
L - für eine Gruppe der Formel $-CH_2-NR_2R^3$ oder

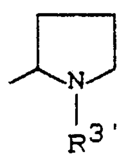

steht,
worin
$R^2$ und $R^3$ gleich oder verschieden sind und jeweils
- für Wasserstoff, Phenyl oder
- für geradkettiges oder verzweigtes Alkyl mit bis zu 6 Kohlenstoffatomen stehen, das gegebenenfalls durch Phenyl oder Pyridyl substituiert ist,
- für eine Gruppe der Formel $-COR^9$ stehen,
worin
$R^9$ - geradkettiges oder verzweigtes Alkyl oder Alkoxy mit bis zu 6 Kohlenstoffatom oder Phenyl bedeutet, das seinerseits durch Carboxy, Alkoxycarbonyl mit bis zu 6 Kohlenstoffatomen oder durch eine Gruppe der Formel $-CONR^4R^5$ oder $-CO-NR^{10}R^{11}$ substituiert sein kann,
worin
$R^4$ und $R^5$ die oben angegebene Bedeutung haben,
$R^{10}$ und $R^{11}$ gleich oder verschieden sind und jeweils
- Wasserstoff oder geradkettiges oder verzweigtes Alkyl mit bis zu 6 Kohlenstoffatomen bedeuten, das seinerseits durch Phenyl oder Pyridyl substituiert sein kann,
oder
$R^2$ oder $R^3$ - für eine Aminoschutzgruppe steht
$R^2$ und $R^3$ - gemeinsam für Phthalimido oder Morpholino stehen oder

$R^2$ oder $R^3$ - für die Gruppe der Formel

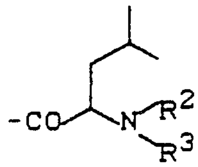

oder

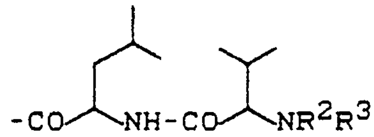

steht,
worin
$R^2$ und $R^3$ die oben angegebene Bedeutung haben,
und
$R^{3'}$ die oben angegebene Bedeutung von $R^3$ hat und mit dieser gleich oder verschieden ist und deren physiologisch unbedenklichen Salze.

3. Peptide der Formel I nach Anspruch 1
worin
x - für eine Gruppe der Formel

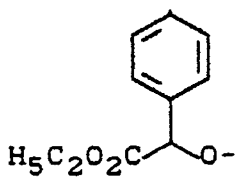

oder

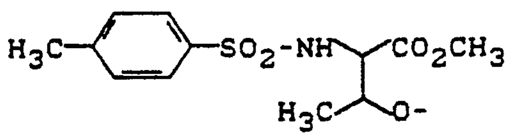

steht
für Hydroxy, Alkoxy mit bis zu 4 Kohlenstoffatomen, Benzyloxy oder für eine Gruppe der Formel $-NR^4R^5$ steht, worin
$R^4$ und $R^5$ gleich oder verschieden sind und jeweils Wasserstoff, geradkettiges oder verzweigtes Alkyl mit bis zu 4 Kohlenstoffatomen bedeuten, das gegebenenfalls durch Phenyl substituiert ist oder Cyclopentyl oder Phenyl, bedeuten, das seinerseits durch Chlor substituiert ist, oder
$R^4$ und $R^5$ gemeinsam mit dem Stickstoffatom einen Morpholinring bilden,
A, B, D und E gleich oder verschieden sind und jeweils
- für eine direkte Bindung stehen oder
- für Glycyl (Gly), Alanyl (Ala), Arginyl (Arg), Histidyl (His), Leucyl (Leu), Isoleucyl (Ile), Seryl (Ser), Threonyl (Thr), Tryptophyl (Trp), Tyrosyl (Tyr), Valyl (Val), Lysyl (Lys), Aspartyl (Asp), Asparaginyl (Asn), Glutamyl (Glu) oder Phenylalanyl (Phe) oder Prolyl (Pro) oder für eine Gruppe der Formel

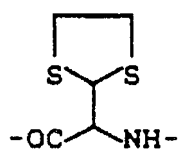

stehen,
gegebenenfalls mit einer Aminoschutzgruppe, in ihrer L-Form oder D-Form,
m - für die Zahl 1 steht,
$R^1$ - für geradkettiges oder verzweigtes Alkyl mit bis zu 4 Kohlenstoffatomen, Cyclohexyl oder Phenyl steht,
L - für eine Gruppe der Formel $-CH_2-NR^2R^3$ oder

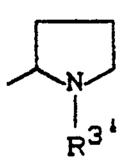

steht,
worin

$R^2$ und $R^3$ gleich oder verschieden sind und jeweils
- für Wasserstoff, Phenyl oder
- für geradkettiges oder verzweigtes Alkyl mit bis zu 4 Kohlenstoffatomen stehen, das gegebenenfalls durch Phenyl oder Pyridyl substituiert ist,
- für eine Gruppe der Formel $-COR^9$ stehen,

worin

$R^9$ - geradkettiges oder verzweigtes Alkyl oder Alkoxy mit bis zu 4 Kohlenstoffatomen oder Phenyl bedeutet, das seinerseits durch Carboxy, Alkoxycarbonyl mit bis zu 4 Kohlenstoffatomen oder durch eine Gruppe der Formel $-CONR^4R^5$ oder $-CONR^{10}R^{11}$ substituiert sein kann,

worin

$R^4$ und $R^5$ die oben angegebene Bedeutung haben,

$R^{10}$ und $R^{11}$ gleich oder verschieden sind und jeweils

- Wasserstoff oder geradkettiges oder verzweigtes Alkyl mit bis zu 4 Kohlenstoffatomen bedeuten, das seinerseits durch Phenyl oder Pyridyl substituiert sein kann,

oder

$R^2$ oder $R^3$ - für eine Aminoschutzgruppe steht

oder

$R^2$ und $R^3$ gemeinsam für Phthalimido oder Morpholino stehen oder

$R^2$ oder $R^3$ - für die Gruppe der Formel

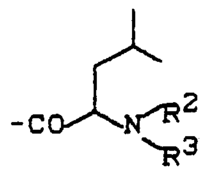

oder

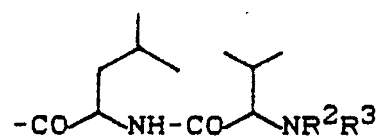

stehen,

worin

$R^2$ und $R^3$ die oben angegebene Bedeutung haben,

und

$R^{3'}$ die oben angegebene Bedeutung von $R^3$ hat und mit dieser gleich oder verschieden ist und deren physiologisch unbedenklichen Salze.

4. Peptide nach Anspruch 1 zur Bekämpfung von Krankheiten.

5. Verfahren zur Herstellung von Peptiden der allgemeinen Formel

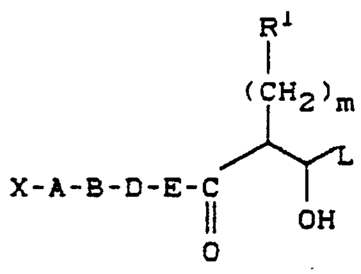

(I)

in welcher

x - für eine Gruppe der Formel

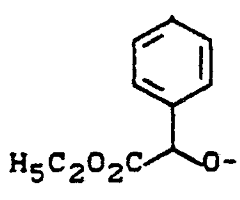

oder

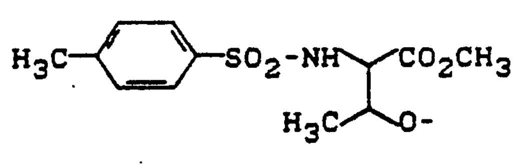

steht oder

für Hydroxy, Alkoxy mit bis zu 8 Kohlenstoffatomen, Benzyloxy oder für eine Gruppe der Formel $-NR^4R^5$ steht, worin

$R^4$ und $R^5$ gleich oder verschieden sind und jeweils Wasserstoff, geradkettiges oder verzweigtes Alkyl mit bis zu 8 Kohlenstoffatomen bedeuten, das gegebenenfalls durch Phenyl substituiert ist oder Cycloalkyl mit 3 bis 8 Kohlenstoffatomen oder Aryl mit 6 bis 10 Kohlenstoffatomen bedeuten, das seinerseits durch Halogen substituiert sein kann,
oder
$R^4$ und $R^5$ gemeinsam mit dem Stickstoffatom einen 5 bis 7-gliedrigen Heterocylus mit bis zu 3 Heteroatomen aus der Reihe Stickstoff, Schwefel oder Sauerstoff bilden,
A, B, D und E gleich oder verschieden sind und jeweils
- für eine direkte Bindung stehen oder
- für einen Rest der Formel

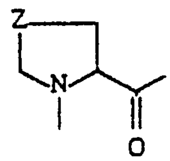

stehen
worin
Z - Sauerstoff, Schwefel oder die Methylengruppe bedeutet, oder
- für eine Gruppierung der Formel

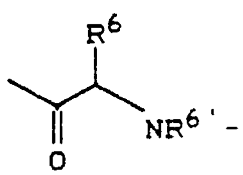

oder

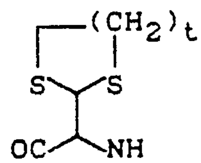

steht,
worin
t - die Zahl 1 oder 2 bedeutet,
$R^6$ - Wasserstoff oder Phenyl bedeutet oder
- geradkettiges oder verzweigtes Alkyl mit bis zu 8 Kohlenstoffatomen bedeutet, das gegebenenfalls durch Carboxy, Hydroxy, Halogen oder durch Alkoxy oder Alkoxycarbonyl mit jeweils bis zu 6 Kohlenstoffatomen substituiert ist, die ihrerseits durch Phenyl substituiert sein können,
oder durch eine Gruppe der Formel $-NR^7R^8$ oder $-CONR^7R^8$ substituiert ist
worin
$R^7$ und $R^8$ gleich oder verschieden sind und jeweils eine Aminoschutzgruppe, Wasserstoff, Acetoxy, Aryl mit 6 bis 10 Kohlenstoffatomen oder geradkettiges oder verzweigtes Alkyl mit bis zu 6 Kohlenstoffatomen bedeuten, das seinerseits durch Amino substituiert sein kann,
oder durch Aryl mit 6 bis 10 Kohlenstoffatomen substituiert ist, das gegebenenfalls bis zu 3-fach gleich oder verschieden durch Halogen, Nitro, Hydroxy oder Alkoxy mit bis zu 6 Kohlenstoffatomen substituiert ist,
oder durch Indolyl, Imidazolyl, Pyridyl, Triazolyl oder Pyrazolyl substituiert ist, die ihrerseits durch $R^8$ substituiert sein können, worin $R^8$ die oben angegebene Bedeutung hat,
$R^{6'}$ - Wasserstoff oder geradkettiges oder verzweigtes Alkyl mit bis zu 6 Kohlenstof fatomen bedeutet,
in ihrer D-Form, L-Form oder als D, L-Isomerengemisch
m - für eine Zahl 0, 1 oder 2 steht,
$R^1$ - für geradkettiges oder verzweigtes Alkyl mit bis zu 8 Kohlenstoffatomen steht,
- für Cycloalkyl mit 3 bis 8 Kohlenstoffatomen steht,
- für Aryl mit 6 bis 10 Kohlenstoffatomen steht, das gegebenenfalls durch Nitro, Cyano oder Halogen substituiert sein kann,
$R^2$ und $R^3$ gleich oder verschieden sind und jeweils
- Wasserstoff, Phenyl oder
- für geradkettiges oder verzweigtes Alkyl mit bis zu 8 Kohlenstoffatomen stehen, das gegebebenenfalls durch Aryl mit 6 bis 10 Kohlenstoffatomen oder Pyridyl substituiert ist,
- für eine Gruppe der Formel $-COR^9$ stehen, worin

$R^9$ - geradkettiges oder verzweigtes Alkyl oder Alkoxy mit bis zu 8 Kohlenstoffatomen oder Aryl mit 6 bis 10 Kohlenstoffatomen bedeutet, das seinerseits durch Carboxy, Alkoxycarbonyl mit bis zu 8 Kohlenstoffatomen oder durch eine Gruppe der Formel $-CONR^4R^5$ oder $-CO-NR^{10}R^{11}$ substituiert sein kann, worin
$R^4$ und $R^5$ die oben angegebene Bedeutung haben und
$R^{10}$ und $R^{11}$ gleich oder verschieden sind und jeweils
- Wasserstoff oder geradkettiges oder verzweigtes Alkyl mit bis zu 8 Kohlenstoffatomen bedeuten, das seinerseits durch Phenyl oder Pyridyl substituiert sein kann, oder
L für eine Gruppe der Formel $-CH_2-NR^2R^3$ oder

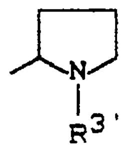

steht
worin
$R^2$ oder $R^3$ für eine Aminoschutzgruppe steht oder
$R^2$ und $R^3$ gemeinsam für Phthalimido oder Morpholino stehen oder für eine Gruppe der Formel

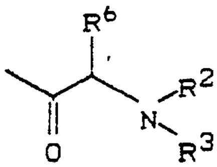

oder

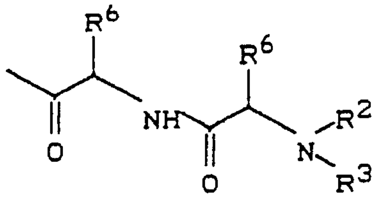

stehen
worin
$R^2$, $R^3$ und $R^6$ die oben angegebene Bedeutung haben und
$R^{3'}$ die oben angegebene Bedeutung von $R^3$ hat und mit dieser gleich oder verschieden ist
und deren physiologisch unbedenklichen Salze, dadurch gekennzeichnet, daß man
[A] entweder Verbindungen der allgemeinen Formel (II)

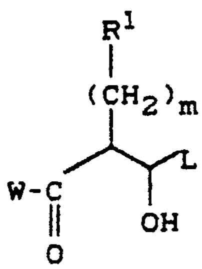

(II)

in welcher
$R^1$, L und m die oben angegebene Bedeutung haben,
und
W - für eine typische Carbonylschutzgruppe, wie beispielsweise Benzyloxy oder Alkoxy mit bis zu 6 Kohlenstoffatomen steht, die gegebenenfalls durch Alkoxycarbonyl mit bis zu 6 Kohlenstoffatomen substituiert sind
oder stereoselektiv Verbindungen der allgemeinen Formel (III)

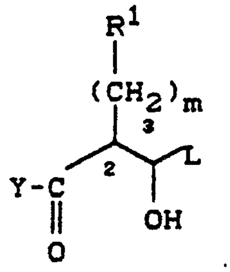

(III)

in welcher
$R^1$, L und m die oben angegebene Bedeutung haben,
und
Y - für eine N-gebundenen Oxazolidin-2-on-Ring steht, der gegebenenfalls durch Alkyl mit bis zu 4 Kohlenstoffatomen, Benzyl oder Phenyl substituiert ist,
zunächst durch Abspaltung der Schutzgruppen nach üblicher Methode in die entsprechenden Säuren überführt und in einem zweiten Schritt mit Verbindungen der allgemeinen Formel (IV)
$X'$ -A-B-D-E-$NH_2$ (IV)
in welcher
A, B, D und E die oben angegebene Bedeutung haben
und
$X'$ - für eine Carboxylschutzgruppe steht
in inerten Lösungsmitteln, gegebenenfalls in Anwesenheit eines Hilfsstoffes umsetzt und die Schutzgruppe $X'$ nach üblicher Methode abspaltet und die Reste X und L zur Erfassung des oben aufgeführten Bedeutungumfangs nach bekannter Methode, beispielsweise durch Aminierung, Veresterung oder Hydrolyse einführt
oder indem man
[B] Verbindungen der allgemeinen Formeln (II) und (III) durch Umsetzung von einem entsprechenden Bruchstück, bestehend aus einer oder mehreren Aminosäuregruppierungen, mit einer freien, gegebenenfalls in aktivierter Form vorliegenden Carboxylgruppe mit einem komplementierenden Bruchstück, bestehend aus einer oder mehreren Aminosäuregruppierungen, mit einer Aminogruppe, gegebenenfalls in aktivierter Form, herstellt, und diesen Vorgang gegebenenfalls so oft mit entsprechenden Bruchstükken wiederholt, bis man die gewünschten Peptide der allgemeinen Formel (I) hergestellt hat, anschließend gegebenenfalls Schutzgruppen abspaltet, gegen andere Schutzgruppen austauscht und gegebenenfalls die Reste L und X nach den unter Verfahren [A] aufgeführten Methoden derivatisiert, wobei zusätzliche reaktive Gruppen, wie z.B. Amino-oder Hydroxygruppen, in den Seitenketten der Bruchstükke gegebenenfalls durch übliche Schutzgruppen geschützt werden können.

6. Arzneimittel enthaltend mindestens ein Peptid nach Anspruch 1.

7. Verfahren zur Herstellung eines Arzneimittels nach Anspruch 6 dadurch gekennzeichnet, daß man Peptide nach Anspruch 1 gegebenenfalls mit Hilfe von üblichen Hilfs- und Trägerstoffen in eine geeignete Applikationsform bringt.

8. Verwendung von Peptiden nach Anspruch 1 zur Herstellung von Arzneimitteln.

9. Verwendung von Peptiden nach Anspruch 1 zur Herstellung von renininhibitorischen Arzneimitteln.

10. Verwendung von Peptiden nach Anspruch 1 zur Bekämpfung von Krankheiten.

11. Verbindungen der allgemeinen Formel (II)

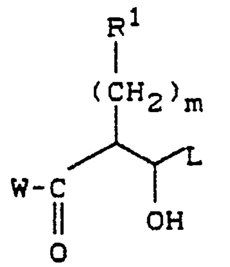

(II)

in welcher

$R^1$, L und m die im Anspruch 1 angegebene Bedeutung haben und W für eine typische Carboxylschutzgruppe wie Benzyloxy oder Alkoxy mit bis zu 6 Kohlenstoffatomen steht, die gegebenenfalls durch Alkoxycarbonyl mit bis zu 6 Kohlenstoffatomen substituiert sind.

12. Verbindungen der allgemeinen Formel (III)

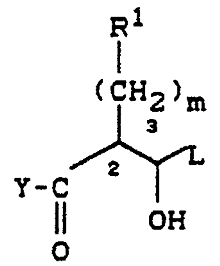

(III)

in welcher
$R^1$, L und m die im Anspruch 1 angegebene Bedeutung haben,
und
Y für eine N-gebundenen Oxazolidin-2-on-Ring steht, der gegebenenfalls durch Alkyl mit bis zu 4 C-Atomen, Benzyl oder Phenyl substituiert ist.

13. Verfahren zur Herstellung von Verbindungen der allgemeinen Formel (II) gemäß Anspruch 11, dadurch gekennzeichnet, daß man Verbindungen der allgemeinen Formel (V)

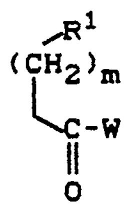

(V)

in welcher
$R^1$, W und m im Anspruch 11 angegebene Bedeutung haben
mit Verbindungen der allgemeinen Formel (VI)

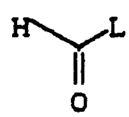

(VI)

in welcher
L die im Anspruch 11 angegebene Bedeutung hat,
in einem inerten Lösungsmittel gegebenenfalls bei Anwesenheit einer Base kondensiert.

14. Verfahren zur Herstellung von Verbindungen der allgemeinen Formel (III) gemäß Anspruch 12, dadurch gekennzeichnet, daß man Verbindungen der allgemeinen Formel (VII)

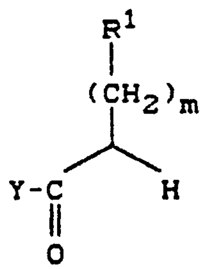

(VII)

in welcher
Y, $R^1$ und m die im Anspruch 12 angegebene Bedeutung haben,
mit Verbindungen der allgemeinen Formel (VI)

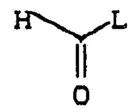

(VI)

in welcher
L die im Anspruch 12 angegebene Bedeutung hat,
in einem inerten Lösungsmittel gegebenenfalls in Anwesenheit einer Base kondensiert.